# FASCICULE DE BREVET EUROPEEN

(11) **EP 2 142 493 B1**
(45) Date de publication et mention de la délivrance du brevet: **27.03.2019**
(21) Numéro de dépôt: 08735856.0
(22) Date de dépôt: 04.04.2008
(51) Int. Cl.: C07C 43/21, C07C 69/01, C07C 69/24, C07C 229/42, C07C 305/24, C07D 215/14, C07D 317/54, C07F 9/12, A61K 31/09, A61K 31/095, A61K 31/222, A61K 31/216, A61K 31/66, A61K 31/47, C07C 43/23, C07C 229/12, C07C 271/22, C07C 271/44, C07C 205/37, C07C 309/65, C07H 15/203

(54) **ISO CA-4 ET ANALOGUES : PUISSANTS CYTOTOXIQUES, INHIBITEURS DE LA POLYMERISATION DE LA TUBULINE**
ISO-CA-4 UND DESSEN ANALOGA ALS WIRKSAME ZYTOTOXISCHE MITTEL, DIE DIE TUBULINPOLYMERISATION INHIBIEREN
ISO CA-4 AND ANALOGUES THEREOF AS POTENT CYTOTOXIC AGENTS INHIBITING TUBULINE POLYMERISATION

(30) Priorité: 04.04.2007 FR 0754280
(43) Date de publication de la demande: 13.01.2010
(73) Titulaire: Centre National de la Recherche Scientifique (C.N.R.S.), 75016 Paris (FR); Université Paris-Sud, 91400 Orsay (FR)
(72) Inventeur: ALAMI, Mouâd, 77600 Bussy Saint Georges (FR); BRION, Jean-Daniel, 95320 Saint Leu la Foret (FR); PROVOT, Olivier, 78500 Sartrouville (FR); PEYRAT, Jean-François, 91140 Villebon sur Yvette (FR); MESSAOUDI, Samir, 91380 Chilly Mazarin (FR); HAMZE, Abdallah, 91300 Massy (FR); GIRAUD, Anne, 94170 Le Perreux sur Marne (FR); BIGNON, Jérôme, 91530 Le Val Saint Germain (FR); BAKALA, Joanna, 75012 Paris (FR); LIU, Jian-Miao, 75013 Paris (FR)
(74) Mandataire: Lavoix
(86) Numéro de dépôt international: PCT/EP2008/054118
(87) Numéro de publication internationale: WO 2008/122620

(56) Documents cités:
- EP-A- 0 278 915
- WO-A-99/34788
- WO-A-2006/026747
- US-A- 5 430 062
- Y. AL-ATTAR ET AL: "143. Synthese einiger auxochromhaltiger alpha, alpha-Diaryläthylene und Stilbene" HELV.CHIM.ACTA, vol. 46, 1963, pages 1286-1294, XP002459021
- PETTIT G R ET AL: "Isolation and structure of combretastatin" CANADIAN JOURNAL OF CHEMISTRY, NATIONAL RESEARCH COUNCIL. OTTAWA, CA, vol. 60, 1982, pages 1374-1376, XP009092133 ISSN: 0008-4042 cité dans la demande
- DATABASE BEILSTEIN [Online] BEILSTEIN INSTITUTE FOR ORGANIC CHEMISTRY, FRANKFURT-MAIN, DE; XP002459031 Database accession no. BRN:9201980 & GOOSSEN,L.J. ET AL: SYN.LETT., vol. 10, 2002, pages 1721-1723,
- POPOV ET AL: "Analysis of complexes of inhibitors with Cryptosporidium hominis DHFR leads to a new trimethoprim derivative" BIOORGANIC & MEDICINAL CHEMISTRY LETTERS, OXFORD, GB, vol. 16, no. 16, 15 août 2006 (2006-08-15), pages 4366-4370, XP005544081 ISSN: 0960-894X
- BOTELLA L ET AL: "Synthesis of methylated resveratrol and analogues by Heck reactions in organic and aqueous solvents" TETRAHEDRON, ELSEVIER SCIENCE PUBLISHERS, AMSTERDAM, NL, vol. 60, no. 26, 21 juin 2004 (2004-06-21), pages 5563-5570, XP004515021 ISSN: 0040-4020

## Description

L'invention concerne de nouveaux composés inhibiteurs de la polymérisation de la tubuline utiles pour le traitement du cancer, leurs procédés de préparation ainsi que leurs utilisations.

Le cancer est la cause majeure de décès dans le monde après les maladies cardiovasculaires. Sur un total mondial de 58 millions de décès enregistrés en 2005, 7,6 millions (soit 13%) étaient dus au cancer. De très nombreux efforts ont été déployés ces dernières années en matière de prévention, confort apporté aux patients et traitements ciblés. Les progrès de l'oncologie médicale sont dus en grande partie à la compréhension des différents mécanismes d'action mis en cause lors de cancers, mais également au développement de nombreux médicaments cytotoxiques associés ou non en polythérapie. On peut citer par exemple le cisplatine, les anthracyclines, le méthotrexate, le 5FU, les taxoïdes, l'irinotécan...).

Si la chirurgie et la radiothérapie sont des traitements particulièrement efficaces lorsqu'un cancer est limité à une seule région de l'organisme, la chimiothérapie devient indispensable lorsque les cellules cancéreuses se sont dispersées. Les médicaments cytotoxiques peuvent donc être administrés avant une intervention chirurgicale ou une radiothérapie pour réduire la taille de la tumeur. Ils sont très souvent utilisés après ces interventions afin d'éliminer les métastases et l'ensemble des cellules cancéreuses qui auraient résisté à ces traitements.

Si de très nombreux traitements à base de cytotoxiques ont fait progresser la recherche médicale (association de cytotoxiques pour éviter les phénomènes de résistance, réduction des effets indésirables améliorant le confort des patients), les chimiothérapies antitumorales ont besoin de nouvelles molécules efficaces pour pallier les phénomènes de résistance aux traitements usuels de plus en plus fréquemment rencontrés. Par ailleurs les médicaments actuels utilisés dans les cancers du sein (27,4% des cas de cancers chez la femme), poumon (13% des cas et en augmentation vertigineuse chez la femme), prostate (15,5%), colon et rectum (13%) permettent de diminuer le degré de gravité des tumeurs sans pour autant mener à des guérisons totales.

Parmi les principaux médicaments anticancéreux utilisés en thérapeutique humaine, les agents interagissant avec la tubuline occupent une place importante. Il est possible de distinguer deux familles d'agents :
(a) les taxanes qui agissent en inhibant la division des cellules cancéreuses provoquant ainsi leur mort. Ils favorisent la polymérisation de la tubuline, la stabilisation de microtubules non fonctionnels et en inhibent la dépolymérisation. Il s'agit du paclitaxel (Taxol®) et du docétaxel (Taxotère®). Ce dernier est l'un des agents de chimiothérapie les plus utilisés au monde pour le traitement du cancer de sein, du cancer du poumon non à petites cellules et du cancer de la prostate métastatique hormono-résistant ; et
(b) les alcaloïdes de la pervenche, dont la liaison à la tubuline entraîne une inhibition de la polymérisation en microtubules, empêchant ainsi la constitution du fuseau mitotique. Il s'agit de la vincristine, de la vindésine, de la vinblastine et de la vinorelbine, qui constituent sur le plan mondial près de 10% du marché des produits antitumoraux cytotoxiques.

Bien qu'ils soient efficaces, l'utilisation des taxanes et des alcaloïdes des Vinca est limitée par le développement de phénomènes de résistance et l'induction d'effets indésirables qui nécessitent donc une surveillance systématique. A titre d'exemple, citons que la vincristine possède une toxicité nerveuse sensitomotrice alors que la toxicité hématologique est souvent le facteur limitant dans le cas d'un traitement avec la vinblastine, la vindésine ou la vinorelbine.

Devant l'urgence de cette situation, le développement de nouveaux inhibiteurs est devenu un enjeu majeur ces dernières années. Les critères recherchés pour les nouveaux composés anti-tumoraux sont:
1. l'efficacité de l'activité antitumorale sur diverses souches dans des modèles *in vitro* mais également dans des modèles animaux *in vivo,*
2. la levée de la multirésistance aux médicaments,
3. la conception de molécules originales hydrosolubles et si possible possédant une structure chimique simple,
4. la diminution de la toxicité systémique,
5. l'identification du mécanisme d'action.

En 1982, Pettit et coll. (Can. J. Chem. 1982, 60, 1374-1376) ont isolé de l'écorce du *Combretum caffrum*, saule d'Afrique du Sud de la famille des Combretaceae, la combrétastatine A-4 représentée ci-dessous.

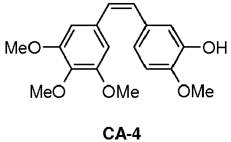

Cette molécule naturelle de structure extrêmement simple, est caractérisée par un motif stilbène de configuration *Z* substitué sur les deux noyaux aromatiques par des groupements méthoxy et un hydroxy. L'intérêt porté à cette molécule par la communauté scientifique est lié tout particulièrement à ses activités antitumorales (cytotoxique et inhibiteur de la polymérisation de la tubuline).

Les premières évaluations biologiques de la combrétastatine A-4 (CA-4) ont montré:
- une activité cytotoxique très puissante sur de nombreuses lignées cellulaires avec une CI₅₀ de l'ordre du nanomolaire (ex : CI₅₀ = 0,9 nM sur cellules HCT 15). L'activité cytotoxique de la CA-4 a été également étudiée sur des cellules endothéliales de la veine ombilicale humaine (HUVEC) et semble faire intervenir un mécanisme par apoptose plutôt que par nécrose cellulaire ;
- une activité antimitotique (agent poison du fuseau). Elle se lie à la tubuline sur le site de fixation de la Colchicine ce qui a pour conséquence d'inhiber sa polymérisation en microtubules empêchant ainsi la formation du fuseau mitotique ; et
- une activité anti-angiogénique *in vitro* par inhibition de la prolifération de cellules endothéliales.

Cependant, *in vivo* l'activité antitumorale de la CA-4 décroît, voire disparaît totalement (par exemple on n'observe aucune activité antitumorale sur l'adénocarcinome du colon 26 de souris). Cette baisse ou absence d'activité peut-être expliquée d'une part par la faible solubilité dans l'eau due au caractère lipophile de la CA-4, ce qui entraîne *in vivo* une mauvaise pharmacocinétique, et d'autre part par la facilité de l'isomérisation de la double liaison de configuration *Z* en *E.* A cet égard, il a été montré que l'isomère *E* de la CA-4 possède une activité cytotoxique sur des cellules leucémiques P-388 de souris environ 60 fois plus faible que l'isomère *Z* naturel.

En raison de la structure chimique très simple de la CA-4 (par comparaison à celles des alcaloïdes des Vinca) et de ses activités biologiques, de nombreux travaux ont été réalisés sur ce composé et on dénombre à ce jour près de 500 publications et plus de 70 demandes de brevets.

Des composés analogues de la CA-4 ont été synthétisés et évalués. Les molécules CA-4-P, OXI4503 et AVE-8062A représentées ci-dessous sont actuellement en développement dans différents laboratoires.

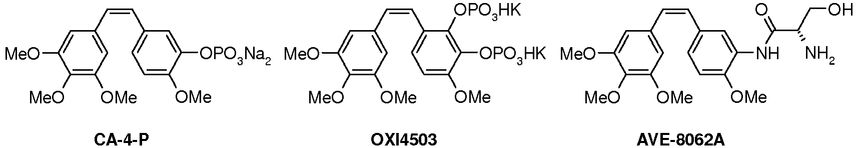

Elles possèdent cependant une double-liaison de géométrie *Z* pouvant conduire à l'isomère *E* biologiquement peu actif.

La demande internationale WO 2006/026747 décrit des dérivés diphényléthylène répondant à la formule suivante :

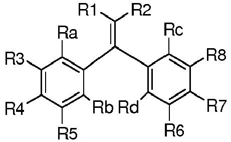

Ces composés, qui existent sous forme d'isomères E ou Z ou de leur mélange, sont décrits comme étant des inhibiteurs de la tubuline. La plupart des exemples cités sont des composés pour lesquels la double liaison est subtituée, et en particulier monosubstituée par un groupement CN (c'est-à-dire que R1, R2 = H, CN). Cependant, aucun test biologique n'a été réalisé. Il est donc difficile de pouvoir évaluer le réel potentiel anticancéreux de ces composés.

Toutefois, un article de la même équipe (Zhang et al. Cancer Res. 2006, 66(2), 951-959) décrit l'activité antitumorale du composé CC-5079 possédant un substituant CN sur la double liaison, ce qui laisserait à penser que la substitution de la double liaison par ce groupement CN est importante pour l'activité thérapeutique de ces composés.

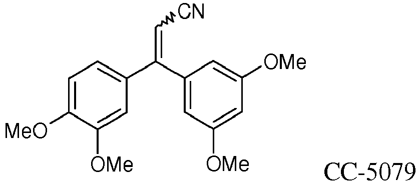

La demanderesse a ainsi découvert de manière surprenante une nouvelle famille de composés dérivés de la CA-4 présentant une forte cytotoxicité (CI₅₀ dans la gamme nanomolaire) sur une grande variété de lignées cellulaires cancéreuses humaines, avec une inhibition de la polymérisation de la tubuline à des concentrations de l'ordre du micromolaire. De plus, ces nouveaux composés induisent l'apoptose, provoquent l'arrêt du cycle cellulaire dans la phase G2/M et possèdent des activités anti-vasculaires.

De façon particulièrement intéressante, ces composés, appelés iso-CA-4, ne présentent pas de stéréochimie au niveau de la double liaison éthylènique, et ne peuvent pas, par conséquent, s'isomériser *in vivo* pour conduire à l'isomère E moins actif.

Plus précisément, l'invention a pour objet les composés de formule (I) suivante : 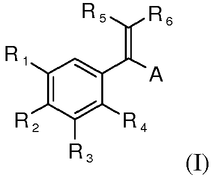 dans laquelle :
- R₁, R₂ et R₃ représententchacun un groupe méthoxy,
- R₄ représente un atome d'hydrogène,
- R₅ et R₆ sont identiques et représentent chacun un atome d'hydrogène ou de fluor, et
- A est un cycle choisi dans le groupe comprenant les groupes aryles et hétéroaryles, lesdits groupes pouvant être :
   ▪ soit accolés à un hétérocycle comportant de 5 à 7 chaînons, comportant éventuellement une ou plusieurs insaturations et éventuellement substitué par un ou plusieurs groupements alkyles en C₁ à C₄,
   ▪ soit substitués par un ou plusieurs groupes choisis parmi les halogènes, les groupes -B(OH)₂, alkyles en C₁ à C₄, alcényles en C₂ à C₄, alcynyles en C₂ à C₄, aryle, hétéroaryle, -COOH, -NO₂, méthylènedioxy, -NR₇R₈, -NHCOR₇, -CONR₇R₈, -NHCOOR₉, -OSi(alkyle en C₁-C₄)₃, -NHSO₂R₉, alkoxy en C₁ à C₄ éventuellement substitué par un ou plusieurs atomes de fluor, -OCONR₇R₈, -OSO₂CF₃, -OSO₂R₉, -SO₂R₉, -OSO₃H, -OPO(OR₁₀)₂, -ONR₇R₈, -OR₁₁, -SO₂NR₁₂R₁₃, -SO₂NHCOR₁₄, -OCOR₁₅, -OCOOR₁₆, -SR₁₇, et -OCO(CH₂)ₙC₆H₄N[(CH₂)ₘCl]₂ avec n = 1 à 4 et m = 1 à 3,
dans lesquels :
◆ R₇ et R₈ représentent, indépendamment l'un de l'autre, un atome d'hydrogène ou un groupe alkyle en C₁ à C₄, aryle ou hétéroaryle, et avantageusement représentent un atome d'hydrogène ou un groupe alkyle en C₁ à C₄,
◆ R₉ représente un groupe alkyle en C₁ à C₄, aryle ou hétéroaryle, et avantageusement représente un groupe alkyle en C₁ à C₄,
◆ R₁₀ représente un atome d'hydrogène ou un groupe alkyle en C₁ à C₄ ou un groupe benzyle,
◆ R₁₁ représente un atome d'hydrogène, un groupe O-protecteur, un sucre choisi parmi le glucose, le mannose, l'arabinose ou le galactose, un aminosucre ou un acide aminé, les groupements OH et NH₂ libres des sucres, aminosucres et acides aminés pouvant être éventuellement substitués par un groupement O-protecteur et N-protecteur, respectivement,
◆ R₁₂ et R₁₃ représentent, indépendamment l'un de l'autre, un atome d'hydrogène, un groupe alkyle en C₁ à C₄, un groupe aryle ou hétéroaryle,
◆ -COR₁₄ représente le reste d'une molécule d'acide aminé liée au groupement -SO₂NH- ou R₁₄ représente un groupe alkyle en C₁ à C₄,
◆ R₁₅ représente un atome d'hydrogène, un groupe alkyle en C₁ à C₄, aryle, ou hétéroaryle, ou un groupe -(CH₂)ₘCO₂H ou -(CH₂)ₘNR₇R₈ avec m = 1 à 3,
◆ R₁₆ représente un groupe alkyle en C₁ à C₄, aryle, ou hétéroaryle, ou un groupe -(CH₂)ₘCO₂H ou -(CH₂)ₘNR₇R₈ avec m = 1 à 3, et
◆ R₁₇ représente un atome d'hydrogène ou un groupe alkyle en C₁ à C₄ ou aryle, ainsi que ses sels pharmaceutiquement acceptables, à l'exclusion des composés de formules suivantes :

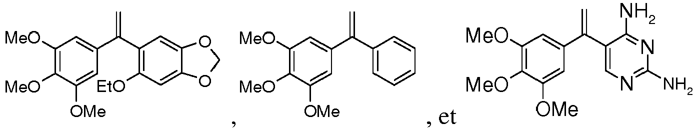

Par le terme "halogène", on entend au sens de la présente invention les atomes de fluor, chlore, brome et iode.

Par le terme "groupe alkyle en C₁ à C₄", on entend au sens de la présente invention tout groupe hydrocarboné comportant de 1 à 4 atomes de carbone, linéaire ou ramifié, en particulier les groupes méthyle, éthyle, *n*-propyle, isopropyle, *n-*butyle, *iso*-butyle, *sec*-butyle, et *tert*-butyle.

Par le terme "groupe alcényle en C₂ à C₄", on entend au sens de la présente invention tout groupe hydrocarboné comportant de 2 à 4 atomes de carbone, linéaire ou ramifié, et comportant au moins une double liaison, tel qu'un groupe vinyle (éthényle).

Par le terme "groupe alcynyle en C₂ à C₄", on entend au sens de la présente invention tout groupe hydrocarboné comportant de 2 à 4 atomes de carbone, linéaire ou ramifié, et comportant au moins une triple liaison, tel qu'un groupe éthynyle ou propynyle.

Par le terme "groupe alkoxy en C₁ à C₄", on entend au sens de la présente invention tout groupe O-alkyle comportant de 1 à 4 atomes de carbone, linéaire ou ramifié, en particulier les groupes méthoxy, éthoxy, propoxy, *n*-butoxy, *iso*-butoxy et *tert*-butoxy.

Par le terme "groupe aryle", on entend au sens de la présente invention un ou plusieurs cycles aromatiques ayant de 5 à 10 atomes de carbone, pouvant être accolés. En particulier, les groupes aryles peuvent être des groupes monocycliques ou bicycliques, comme par exemple le groupe phényle ou naphthyle. Avantageusement, le groupe aryle est un phényle.

Par le terme "groupe hétéroaryle", on entend au sens de la présente invention tout groupe aromatique comprenant de 5 à 10 atomes cycliques, qui sont des atomes de carbone et un ou plusieurs hétéroatomes, tels que par exemple des atomes de soufre, d'azote ou d'oxygène. L'hétéroaryle selon la présente invention peut être constitué par un ou deux cycles accolés. Des exemples de groupes hétéroaryles sont les groupes quinolyle, isoquinolyle, imidazolyle, indolyle, pyridyle, triazinyle, thiazoyle, pyridazinyle et thiophényle.

Par le terme "hétérocycle", on entend au sens de la présente invention tout cycle hydrocarboné, saturé ou non, mais non aromatique, de 5 à 7 chaînons, contenant un ou plusieurs hétéroatomes, tels que par exemple des atomes de soufre, d'azote ou d'oxygène.

Dans la cadre de la présente invention, le groupement constitué par un hétérocycle accolé à un groupe aryle peut être avantageusement un chromanyle, un chroményle ou un 1,3-benzodioxolyle.

Par "sucre", on entend notamment, au sens de la présente invention, l'érythrose, le thréose, le ribose, l'arabinose, le xylose, le lyxose, l'allose, l'altrose, le glucose, le mannose, le gulose, l'idose, le galactose, le talose, l'érythrulose, le ribulose, le xylulose, le psicose, le fructose, le sorbose ou encore le tagatose, sous forme D ou L. Avantageusement, il s'agit du glucose, du mannose, de l'arabinose ou du galactose

On entend par "aminosucre", au sens de la présente invention, un sucre dans lequel un groupe amino remplace un groupe hydroxyle, comme par exemple la glucosamine et la galactosamine.

On entend par "acide aminé", au sens de la présente invention, tous les résidus des acides α-aminés naturels (par exemple Alanine (Ala), Arginine (Arg), Asparagine (Asn), Acide aspartique (Asp), Cystéine (Cys), Glutamine (Gln), Acide glutamique (Glu), Glycine (Gly), Histidine (His), Isoleucine (Ile), Leucine (Leu), Lysine (Lys), Méthionine (Met), Phénylalanine (Phe), Proline (Pro), Sérine (Ser), Thréonine (Thr), Tryptophane (Trp), Tyrosine (Tyr) et Valine (Val)) sous la forme D ou L, ainsi que les acides aminés non naturels (par exemple (1-naphthyl)alanine, (2-naphthyl)alanine, homophénylalanine, (4-chlorophényl)alanine, (4-fluorophényl)alanine, (3-pyridyl)alanine, phénylglycine, acide diaminopimélique, acide 2,6-diaminoheptane-1,7-dioïque, acide 2-aminobutyrique, acide 2-aminotétralin-2-carboxylique, erythro-β-méthylphénylalanine, threo-β-méthylphénylalanine, (2-méthoxyphényl)alanine, acide 1-amino-5-hydroxyindan-2-carboxylique, acide 2-aminohéptane-1,7-dioïque, (2,6-diméthyl-4-hydroxyphényl)alanine, erythro-β-méthyltyrosine ou threo-β-méthyltyrosine).

Par le terme "groupe O-protecteur", on entend au sens de la présente invention tout substituant qui protège le groupe hydroxyle ou carboxyle, c'est à dire un atome d'oxygène réactif, contre les réactions indésirables tels que les groupes O-protecteurs décrits dans Greene, "Protective Groups In Organic Synthesis", (John Wiley & Sons, New York (1981)) et Harrison et al. "Compendium of Synthetic Organic Methods", Vols. 1 à 8 (J. Wiley & sons, 1971 à 1996). Les groupes O-protecteurs comprennent les éthers de méthyle ou d'alkyle substitués ou non, par exemple, méthoxyméthyle, benzyloxyméthyle, 2-méthoxyéthoxyméthyle, 2-(triméthylsilyle) éthoxyméthyle, *t*-butyle, benzyle et triphénylméthyle, les éthers de benzyle (substitués ou non), les tétrahydropyranyle éthers, les éthers d'allyle, les éthyle éthers substitués, par exemple, 2,2,2-trichloroéthyle, les silyle éthers ou les éthers d'alkylsilyle, par exemple, triméthylsilyle, *t*-butyldiméthylsilyle et *t-*butyldiphénylsilyle, les éthers d'hétérocycle et les esters préparés par réaction du groupe hydroxyle avec un acide carboxylique par exemple, les esters de *tert-butyle,* de benzyle ou de méthyle, les carbonates en particulier le carbonate de benzyle ou d'halogénoalkyle, l'acétate, le propionate, le benzoate et similaires. Avantageusement, il s'agit d'un *tert*-butyle, d'un acétyle ou d'un benzyle.

Par le terme "groupe N-protecteur", on entend au sens de la présente invention tout substituant qui protège le groupe NH₂ contre les réactions indésirables tels que les groupes N-protecteur décrits dans Greene, "Protective Groups In Organic synthesis", (John Wiley & Sons, New York (1981)) et Harrison et al. « Compendium of Synthetic Organic Methods", Vols. 1 à 8 (J. Wiley & sons, 1971 à 1996). Les groupes N-protecteur comprennent les carbamates, amides, dérivés N-alkylés, dérivés amino acétale, dérivés N-benzylé, dérivés imine, dérivés énamine et dérivés N-hétéroatome. En particulier, le groupe N-protecteur comprend le formyle, l'acétyle, le benzoyle, le pivaloyle, le phénylsulfonyle, le benzyle (Bn), le t-butyloxycarbonyle (Boc), le benzyloxycarbonyle (Cbz), le 9-fluorénylméthoxycarbonyle (Fmoc), le p-méthoxybenzyloxycarbonyle, le p-nitrobenzyl-oxycarbonyle, le trichloroéthoxycarbonyl (TROC), l'allyloxycarbonyl (Alloc), le 9-Fluorénylméthyloxycarbonyl (Fmoc), le trifluoro-acétyle, les carbamates de benzyle (substitués ou non) et similaires. Avantageusement, il s'agit du groupe Fmoc.

On entend par "liaison ester ou amide", un groupement -C(O)O- ou -C(O)NH-. Dans le cas particulier de la présente invention, le carbonyle de la liaison ester ou amide sera préférentiellement lié au résidu de la molécule à activité antitumorale tandis que l'oxygène ou le groupe NH de cette même liaison sera lié au groupe aryle ou hétéroaryle défini dans A.

Dans la présente invention, on entend désigner par "pharmaceutiquement acceptable" ce qui est utile dans la préparation d'une composition pharmaceutique, qui est généralement sûr, non toxique et ni biologiquement ni autrement non souhaitable et qui est acceptable pour une utilisation vétérinaire de même que pharmaceutique humaine.

On entend désigner par "sels pharmaceutiquement acceptables" d'un composé des sels qui sont pharmaceutiquement acceptables, comme définis ici, et qui possèdent l'activité pharmacologique souhaitée du composé parent. De tels sels comprennent :
(1) les hydrates et les solvates,
(2) les sels d'addition d'acide formés avec des acides inorganiques tels que l'acide chlorhydrique, l'acide bromhydrique, l'acide sulfurique, l'acide nitrique, l'acide phosphorique et similaires ; ou formés avec des acides organiques tels que l'acide acétique, l'acide benzènesulfonique, l'acide benzoïque, l'acide camphosulfonique, l'acide citrique, l'acide éthanesulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires. Avantageusement, il s'agit de l'acide chlorhydrique ; ou
(3) les sels formés lorsqu'un proton acide présent dans le composé parent soit est remplacé par un ion métallique, par exemple un ion de métal alcalin, un ion de métal alcalino-terreux ; soit se coordonne avec une base organique ou inorganique. Les bases organiques acceptables comprennent la diéthanolamine, l'éthanolamine, N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium. Avantageusement, le proton acide est déplacé par un ion Na⁺, notamment en utilisant de l'hydroxyde de sodium.

Les sels d'addition d'acide sont formés en particulier avec une fonction amine ou avec une pyridine. Les sels d'addition de base sont formés en particulier avec une fonction acide carboxylique (-COOH), phosphate (-OP(O)(OH)₂) ou encore sulfate (-OSO₃H).

On décrit également ici les prodrogues des composés de formule (I). Par "prodrogue", on entend désigner, au sens de la présente invention, un composé qui est administré sous une forme inactive (ou moins active) et qui est métabolisé *in vivo,* notamment par action d'enzymes ou de l'acide gastrique, en une forme active (ou plus active). L'utilisation d'une prodrogue permet d'améliorer en particulier les paramètres physico-chimiques d'une molécule tels que la solubilité ainsi que la pharmaco-cinétique (vectorisation, biodisponibilité, etc.), afin de favoriser son assimilation par un organisme après administration. En particulier, une prodrogue d'une molécule portant un groupement amino (NH₂) pourra résulter notamment de l'acylation ou de la phosphorylation de ce groupement amino. Lorsqu'une molécule porte un groupement hydroxy (OH), la prodrogue pourra résulter en particulier de l'acylation ou de la phosphorylation de ce groupement hydroxy.

De façon également avantageuse, R₅ et R₆ représentent chacun un atome d'hydrogène.

Avantageusement, R₁, R₂ et R₃ représentent chacun un groupe méthoxy, et R₅ et R₆ représentent chacun un atome d'hydrogène.

De façon également avantageuse, R₅ et R₆ représentent chacun un atome de fluor.

Avantageusement, R₁, R₂ et R₃ représentent chacun un groupe méthoxy, et R₅ et R₆ représentent chacun un atome de fluor.

De manière encore avantageuse, la molécule à activité antitumorale sera une molécule à activité anti-vasculaire, cytotoxique, anti-angiogénique, anti-apoptotique ou inhibitrice de kinase. En particulier, elle pourra être choisie parmi la 6-mercaptopurine, la fludarabine, la cladribine, la pentostatine, la cytarabine, le 5-fluorouracile, la gemcitabine, le méthotrexate, le raltitrexed, l'irinotécan, le topotécan, l'étoposide, la daunorubicine, la doxorubicine, l'épirubicine, l'idarubicine, la pirarubicine, la mitoxantrone, la chlorméthine, la cyclophosphamide, l'ifosfamide, le melphalan, le chlorambucil, le busulfan, la carmustine, la fotémustine, la streptozocine, le carboplatine, le cisplatine, l'oxaliplatine, la procarbazine, la dacarbazine, la bléomycine, la vinblastine, la vincristine, la vindésine, la vinorelbine, le paclitaxel, le docétaxel, la L-asparaginase, la flutamide, la nilutamide, la bicalutamide, l'acétate de cyprotérone, la triptoréline, la leuproréline, la goséréline, la buséréline, le formestane, l'aminoglutéthimide, l'anastrazole, le létrozole, le tamoxifène, l'octréotide, le lanréotide, l'acide (Z)-3-[2,4-diméthyl-5-(2-oxo-1,2-dihydro-indol-3-ylidèneméthyl)-1H-pyrrol-3-yl]-propionique (SU 6668), l'acide 4-((9-chloro-7-(2,6-difluorophényl)-5H-pyrimidol(5,4-d)(2)benzazépin-2-yl)amino) benzoïque (MLN-8054), l'acide 5,6-diméthylxanthénone-4-acétique (DMXAA) ou encore l'acide 3-(4-(1,2-diphénylbut-1-ényl)phényl)acrylique (GW 5638). Avantageusement, il s'agit de DMXAA.

Avantageusement, la molécule à activité antitumorale comportera une fonction acide carboxylique COOH, telle que SU 6668, MLN-8054, DMXAA ou GW 5638, permettant ainsi de la coupler au groupement aryle ou hétéroaryle de A, substitué par au moins un groupement OH ou NH₂, par une réaction d'estérification ou d'amidification. Il pourra cependant être utilisé une molécule à activité antitumorale sur laquelle une fonction acide aura été greffée pour permettre le couplage avec le groupement aryle ou hétéroaryle de A.

La liaison ester ou amide ainsi formée présente l'avantage de pouvoir être facilement hydrolysée *in vivo.* Ainsi, après administration du composé de l'invention, la molécule à activité antitumorale ainsi qu'une nouvelle molécule de l'invention pourront être libérées, permettant une double action thérapeutique.

Dans un mode de réalisation particulier, A est choisi dans le groupe comprenant les groupes phényle, naphtyle, quinolinyle et indolyle, et de préférence phényle, lesdits groupes pouvant être :
▪ soit accolés à un hétérocycle comportant de 5 à 7 chaînons, comportant éventuellement une ou plusieurs insaturations et éventuellement substitué par un ou plusieurs groupements alkyles en C₁ à C₄,
▪ soit substitués par un ou plusieurs groupes choisis parmi les halogènes, les groupes -B(OH)₂, alkyles en C₁ à C₄, alcényles en C₂ à C₄, alcynyles en C₂ à C₄, aryle, hétéroaryle, -COOH, -NO₂, méthylènedioxy, -NR₇R₈, -NHCOR₇, -CONR₇R₈, -NHCOOR₉, -OSi(alkyle en C₁-C₄)₃, -NHSO₂R₉, alkoxy en C₁ à C₄ éventuellement substitué par un ou plusieurs atomes de fluor, -OCONR₇R₈, -OSO₂CF₃, -OSO₂R₉, -SO₂R₉, -OSO₃H, -OPO(OR₁₀)₂, -ONR₇R₈, -OR₁₁, -SO₂NR₁₂R₁₃, -SO₂NHCOR₁₄, -OCOR₁₅, -OCOOR₁₆, -SR₁₇, -OCO(CH₂)ₙC₆H₄N[(CH₂)ₘCl]₂ avec n = 1 à 4 et m = 1 à 3, et un résidu d'une molécule à activité anti-tumorale lié par l'intermédiaire d'une liaison ester ou amide,
R₇, R₈, R₉, R₁₀, R₁₁, R₁₂, R₁₃, R₁₄, R₁₅, R₁₆ et R₁₇ étant tels que définis ci-dessus.

Avantageusement, A est un cycle choisi dans le groupe comprenant les groupes aryles et hétéroaryles, ledit cycle pouvant être substitué par un ou plusieurs groupes choisis parmi -Me, -OH, -OMe, -OCF₃, -NH₂, -NO₂, -COOH, -B(OH)₂, - OSi*t*BuMe₂, -OCOMe, -OCO*t*Bu, méthylénedioxy, -OCONEt₂, -OCO(CH₂)₂COOH, -OCOCH₂NMe₂, -OSO₃H, -OSO₂CF₃, -OP(O)(OH)₂, -OP(O)(OEt)₂, -OPO(OCH₂Ph)₂, -Br, -F, -OCO(CH₂)₃C₆H₄N[(CH₂)₂Cl]₂, -OCO(C₅H₄N), 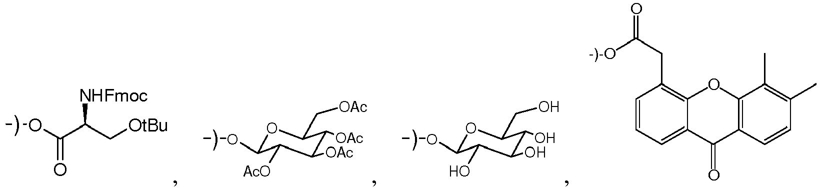 et

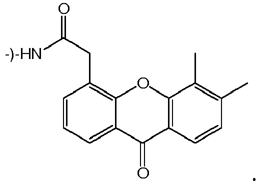

A représente de façon encore plus avantageuse un groupe phényle, naphtyle, quinolinyle ou indolyle, et de préférence phényle, ledit groupe pouvant être substitué par un ou plusieurs groupes choisis parmi -Me, -OH, -OMe, -OCF₃, -NH₂, -NO₂, - COOH, -B(OH)₂, -OSi*t*BuMe₂, -OCOMe, -OCO*t*Bu, méthylénedioxy, -OCONEt₂, - OCO(CH₂)₂COOH, -OCOCH₂NMe₂, -OSO₃H, -OSO₂CF₃, -OP(O)(OH)₂, - OP(O)(OEt)₂, -OPO(OCH₂Ph)₂, -Br, -F, -OCO(CH₂)₃C₆H₄N[(CH₂)₂Cl]₂, - OCO(C₅H₄N),

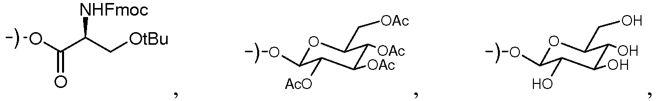

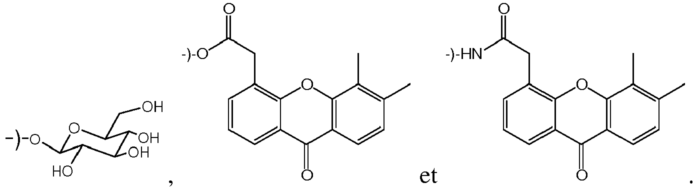

A représente de façon également avantageuse un groupe aryle ou hétéroaryle, avantageusement un groupe phényle ou naphtyle, et de préférence phényle, lesdits groupes pouvant être substitués par un ou plusieurs groupes choisis dans le groupe comprenant éthyle, propyle, butyle, -NH₂, -NHMe, -NMe₂, -NMeEt, -NHEt, -NEt₂, -NHCOMe, -NHCOEt, -CONH₂, -CONMe₂, -CONEt₂, -OSiMe₃, -OSiEt₃, -NHCO₂Me, -NHCO₂Et, -OCH₂F, -OCHF₂, -OEt, -OCOEt, -SO₂Me, -SO₂Et, -OPO(OMe)₂, -OPO(OEt)₂, -ONH₂, -ONMe₂, -ONEt₂, -SO₂NH₂, -SO₂NMe₂, -SO₂NEt₂, -SO₂NMeEt, -SO₂NHMe, -SO₂NHEt, -SO₂NHCOMe, -SO₂NHCOEt.

Avantageusement, les composés de l'invention répondent à la formule (II) suivante : 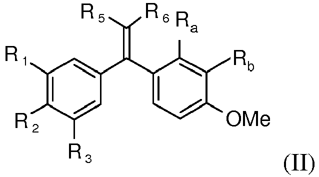 dans laquelle :
- R₁, R₂, R₃, R₅ et R₆ sont tels que définis ci-dessus,
- Rₐ représente un atome d'hydrogène ou d'halogène, ou un groupe -B(OH)₂, alkyles en C₁ à C₄, alcényles en C₂ à C₄, alcynyles en C₂ à C₄, aryle, hétéroaryle, -COOH, -NO₂, méthylènedioxy, -NR₇R₈, -NHCOR₇, -CONR₇R₈, -NHCOOR₉, -OSi(alkyle en C₁-C₄)₃, -NHSO₂R₉, alkoxy en C₁ à C₄ éventuellement substitué par un ou plusieurs atomes de fluor, -OCONR₇R₈, -OSO₂CF₃, -OSO₂R₉, -SO₂R₉, -OSO₃H, -OPO(OR₁₀)₂, -ONR₇R₈, -OR₁₁, -SO₂NR₁₂R₁₃, -SO₂NHCOR₁₄, -OCOR₁₅, -OCOOR₁₆, -SR₁₇ ou -OCO(CH₂)ₙC₆H₄N[(CH₂)ₘCl]₂ avec n = 1 à 4 et m = 1 à 3, et
- R_{b} représente un atome d'halogène, et de préférence un atome de fluor, un groupe -OR₁₁, -OCOR₁₅, -OCOOR₁₅, -OCONR₇R₈, -OSO₂R₉, -OSO₂CF₃, -OSO₃H, -OPO(OR₁₀)₂, -NH₂, -NHCOR₇, -NHCOOR₉, -NHSO₂R₉ ou un résidu d'une molécule à activité antitumorale lié par l'intermédiaire d'une liaison ester ou amide,
R₇, R₈, R₉, R₁₀, R₁₁, R₁₂, R₁₃, R₁₄, R₁₅, R₁₆ et R₁₇ étant tels que définis ci-dessus.

Avantageusement, Rₐ représente un atome d'hydrogène ou un groupe -NR₇R₈, -NHCOR₇, -CONR₇R₈, -NHCOOR₉, -NHSO₂R₉, -OCONR₇R₈, -OSO₂CF₃, -OSO₂R₉, -OSO₃H, -OPO(OR₁₀)₂, -ONR₇R₈, -OR₁₁, -SO₂NR₁₂R₁₃, -SO₂NHCOR₁₄, -OCOR₁₅ ou -OCOOR₁₆, avec R₇, R₈, R₉, R₁₀, R₁₁, R₁₂, R₁₃, R₁₄, R₁₅ et R₁₆ tels que définis ci-dessus.

De manière encore plus avantageuse, Rₐ représente un atome d'hydrogène ou un groupe -NR₇R₈, -NHCOR₇, -CONR₇R₈, -NHCOOR₉, -OCONR₇R₈, -OPO(OR₁₀)₂, -OCOR₁₅ ou -OCOOR₁₆, avec R₇, R₈, R₉, R₁₀, R₁₅ et R₁₆ tels que définis ci-dessus.

En core plus avantageusement, Rₐ représente un atome d'hydrogène.

En particulier, les composés de l'invention pourront être choisis parmi :

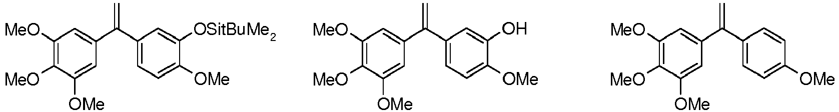

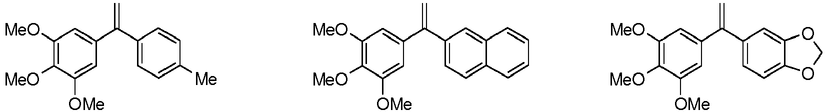

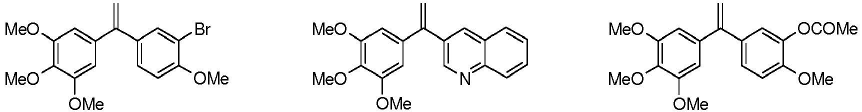

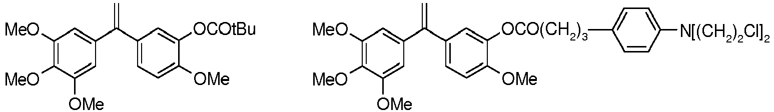

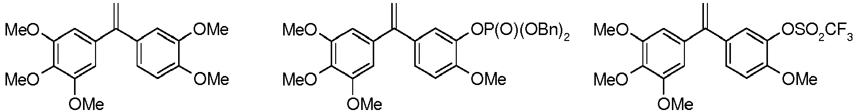

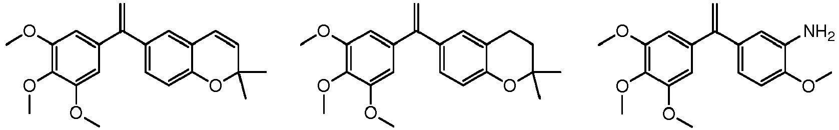

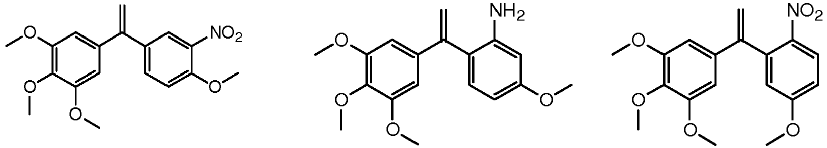

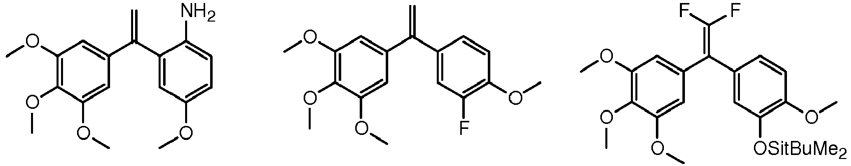

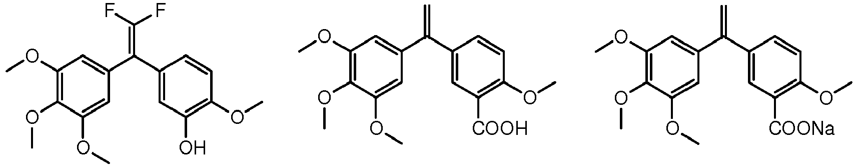

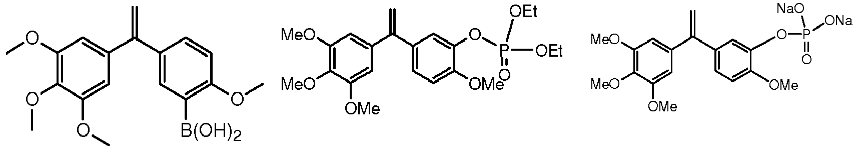

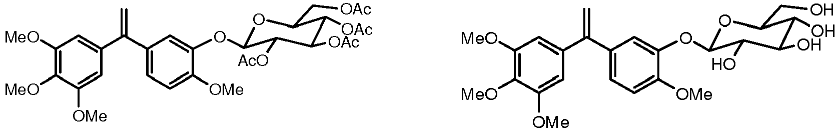

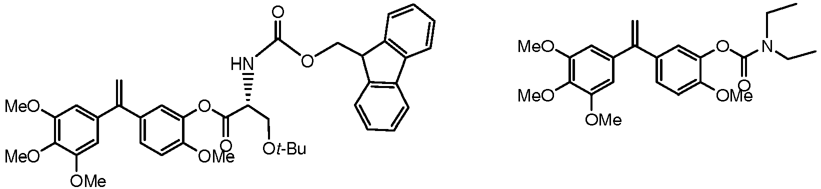

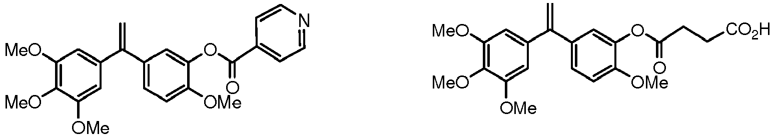

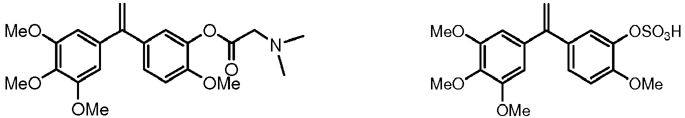

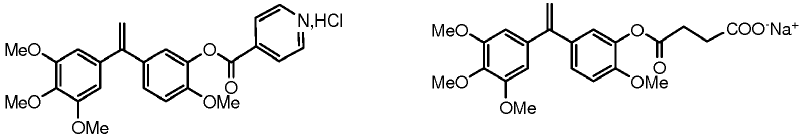

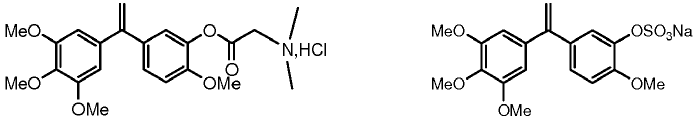

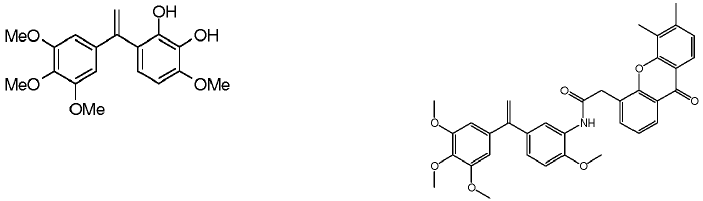

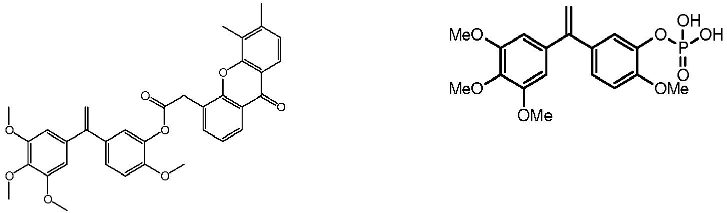

L'absence de stéréochimie de la double liaison des composés de formule (I), résout de manière définitive le problème d'isomérisation susceptible d'intervenir *in vivo,* entraînant des chutes (ou absence) d'activité cytotoxique comme c'est par exemple le cas de la CA-4.

L'invention a également pour objet les procédés de synthèse des composés de formule (I).

Les composés de formule (I) peuvent être synthétisés selon des procédés connus de l'homme du métier, à partir de produits disponibles dans le commerce ou préparés selon des méthodes connues de l'homme du métier, et en particulier selon tout procédé comprenant les étapes successives suivantes :
- faire réagir un composé de formule (II) suivante : 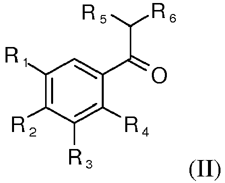 dans laquelle R₁, R₂, R₃, R₄, R₅ et R₆ sont tels que définis précédemment,
   avec un composé organométallique de formule A-M dans laquelle A est tel que défini précédemment et M représente un métal alcalin ou un métal alcalino-terreux substitué par un halogène, pour former le composé de formule (III) suivante : 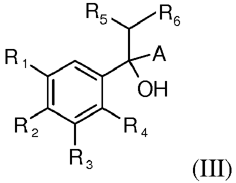
- faire réagir le composé formule (III) ainsi obtenu avec un acide pour former le composé de formule (I) correspondant ;
suivies d'éventuelles étapes classiques supplémentaires de modification des substituants de A.

Par « métal alcalin », on entend notamment le sodium (Na), le lithium (Li) ou le potassium (K).

Par « métal alcalino-terreux », on entend notamment le calcium (Ca) ou le magnésium (Mg).

Avantageusement, M représente l'atome de lithium ou le groupe MgX dans lequel X représente un halogène, de préférence le brome ou le chlore, et avantageusement, le brome.

De manière également avantageuse, l'acide utilisé dans la dernière étape est de l'acide *para*-toluènesulfonique (APTS).

Les composés de formule (I) peuvent également être préparés à partir du composé de formule (IV) suivante : 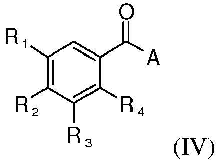 pour laquelle R₁, R₂, R₃, R₄ et A sont tels que définis précédemment,
par une réaction de Wittig, en présence de bromure ou de chlorure de méthyl triphénylphosphonium (dans le cas où R₅ = R₆ = H) ou de difluorométhyl phosphonate d'éthyle (dans le cas où R₅ = R₆ = F), et en présence d'une base,
cette réaction pouvant être éventuellement suivie d'étapes classiques supplémentaires de modification des substituants de A.

De manière avantageuse, la base utilisée pour la réaction de Wittig sera le lithium hexamethyldisilazide (LiHMDS).

Le composé de formule (IV) peut être obtenu notamment par oxydation de l'alcool correspondant de formule (V) suivante : 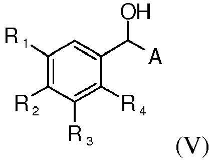 pour laquelle R₁, R₂, R₃, R₄ et A sont tels que définis précédemment,
en utilisant par exemple l'oxyde de manganèse ou le chlorochromate de pyridinium (PCC).

L'alcool (V) peut lui-même être obtenu à partir de l'aldéhyde de formule (VI) suivante : 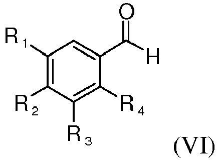 pour laquelle R₁, R₂, R₃ et R₄ sont tels que définis précédemment,
par réaction avec un composé organométallique de formule A-M dans laquelle A et M sont tels que définis précédemment.

Les étapes de synthèse sont ainsi compatibles avec les exigences industrielles. Par ailleurs, les analogues (sucres, phosphates, acides boroniques...) ainsi préparés sont solubles dans l'eau et assimilables par voie orale.

L'invention a également pour objet les composés de formules (I) ou les composés choisis parmi 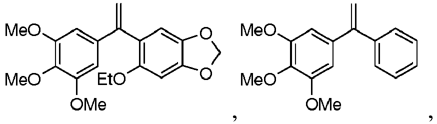 et 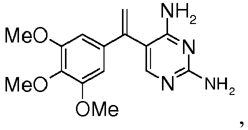 ainsi que leurs sels pharmaceutiquement acceptables et leurs prodrogues, en tant que médicaments, avantageusement en tant que médicaments inhibiteurs de la polymérisation de la tubuline, et encore avantageusement, en tant que médicaments destinés à traiter ou prévenir les maladies prolifératives, telles que le cancer, le psoriasis ou la fibrose, et en particulier le cancer.

En particulier, les composés de l'invention pourront être utiles dans le traitement d'un cancer, tel que ceux susceptibles d'être traités par CA-4 ou par le taxotère.

L'invention concerne également l'utilisation d'un composé de formule (I) ou un composé choisi parmi : 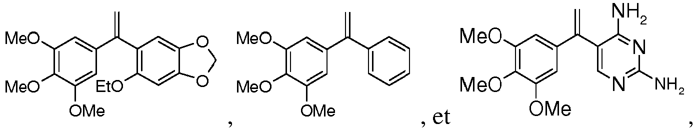 ou un de leurs sels pharmaceutiquement acceptables pour la fabrication d'un médicament inhibiteur de la polymérisation de la tubuline, et avantageusement destiné à traiter ou prévenir les maladies prolifératives, telles que le cancer, le psoriasis ou la fibrose, et en particulier le cancer.

L'invention a également pour objet une composition pharmaceutique comprenant au moins un composé de formule (I) ou un composé choisi parmi 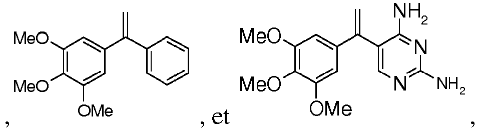 ou un de leurs sels pharmaceutiquement acceptables en association avec un ou plusieurs excipients pharmaceutiquement acceptables.

L'invention a également pour objet une composition pharmaceutique comprenant au moins un composé de formule (I) ou un composé choisi parmi 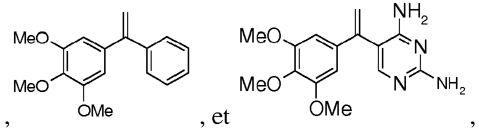 ou un de leurs sels pharmaceutiquement acceptables en association avec au moins un autre principe actif, notamment un composé anti-cancéreux, cytotoxique ou non, en association avec un ou plusieurs excipients pharmaceutiquement acceptables.

A titre d'exemples de principes actifs pouvant être associés au composé de formule (I) dans une composition selon l'invention, on cite de façon non limitative la 6-mercaptopurine, la fludarabine, la cladribine, la pentostatine, la cytarabine, le 5-fluorouracile, la gemcitabine, le méthotrexate, le raltitrexed, l'irinotécan, le topotécan, l'étoposide, la daunorubicine, la doxorubicine, l'épirubicine, l'idarubicine, la pirarubicine, la mitoxantrone, la chlorméthine, la cyclophosphamide, l'ifosfamide, le melphalan, le chlorambucil, le busulfan, la carmustine, la fotémustine, la streptozocine, le carboplatine, le cisplatine, l'oxaliplatine, la procarbazine, la dacarbazine, la bléomycine, la vinblastine, la vincristine, la vindésine, la vinorelbine, le paclitaxel, le docétaxel, la L-asparaginase, la flutamide, la nilutamide, la bicalutamide, l'acétate de cyprotérone, la triptoréline, la leuproréline, la goséréline, la buséréline, le formestane, l'aminoglutéthimide, l'anastrazole, le létrozole, le tamoxifène, l'octréotide, le lanréotide, l'acide (Z)-3-[2,4-diméthyl-5-(2-oxo-1,2-dihydro-indol-3-ylidèneméthyl)-1H-pyrrol-3-yl]-propionique, l'acide 4-((9-chloro-7-(2,6-difluorophényl)-5H-pyrimidol(5,4-d)(2)benzazépin-2-yl)amino)benzoïque, l'acide 5,6-diméthylxanthénone-4-acétique ou encore l'acide 3-(4-(1,2-diphénylbut-1-ényl)phényl)acrylique.

Les composés selon l'invention peuvent être administrés par voie orale, sublinguale, parentérale, sous-cutanée, intramusculaire, intraveineuse, transdermique, locale ou rectale.

Les composés selon l'invention peuvent être utilisés dans le traitement et la prévention des maladies prolifératives comme les cancers, le psoriasis et la fibrose.

Ils peuvent être utilisés à des doses comprises entre 0,01 mg et 1000 mg par jour, donnés en une seule dose une fois par jour ou de préférence administrés en plusieurs doses tout au long de la journée, par exemple deux fois par jour en doses égales. La dose administrée par jour est avantageusement comprise entre 5 mg et 500 mg, encore plus avantageusement entre 10 mg et 200 mg. Il peut être nécessaire d'utiliser des doses sortant de ces gammes ce dont l'homme du métier peut se rendre compte lui-même.

Les composés selon l'invention peuvent être utilisés pour diminuer ou inhiber la polymérisation de la tubuline, notamment *in vitro* ou *in vivo.*

La présente invention a également pour objet une composition pharmaceutique comprenant :
(i) au moins un composé de formule (I) ou un composé choisi parmi 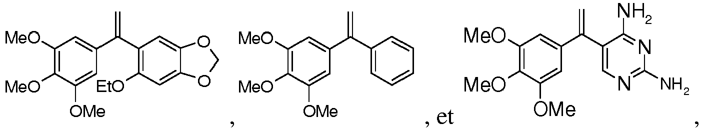
(ii) au moins un autre principe actif, notamment utile pour le traitement de maladies prolifératives telles que le cancer, le psiorasis ou la fibrose, et avantageusement un agent anti-cancéreux tel qu'un agent anti-vasculaire, cytotoxique ou anti-angiogénique,
   en tant que produits de combinaison pour une utilisation simultanée, séparée ou étalée dans le temps.

A titre de principe actif, on peut citer notamment, de façon non limitative, la 6-mercaptopurine, la fludarabine, la cladribine, la pentostatine, la cytarabine, le 5-fluorouracile, la gemcitabine, le méthotrexate, le raltitrexed, l'irinotécan, le topotécan, l'étoposide, la daunorubicine, la doxorubicine, l'épirubicine, l'idarubicine, la pirarubicine, la mitoxantrone, la chlorméthine, la cyclophosphamide, l'ifosfamide, le melphalan, le chlorambucil, le busulfan, la carmustine, la fotémustine, la streptozocine, le carboplatine, le cisplatine, l'oxaliplatine, la procarbazine, la dacarbazine, la bléomycine, la vinblastine, la vincristine, la vindésine, la vinorelbine, le paclitaxel, le docétaxel, la L-asparaginase, la flutamide, la nilutamide, la bicalutamide, l'acétate de cyprotérone, la triptoréline, la leuproréline, la goséréline, la buséréline, le formestane, l'aminoglutéthimide, l'anastrazole, le létrozole, le tamoxifène, l'octréotide, le lanréotide, l'acide (Z)-3-[2,4-diméthyl-5-(2-oxo-1,2-dihydro-indol-3-ylidèneméthyl)-1H-pyrrol-3-yl]-propionique, l'acide 4-((9-chloro-7-(2,6-difluorophényl)-5H-pyrimidol(5,4-d)(2)benzazépin-2-yl)amino)benzoïque, l'acide 5,6-diméthylxanthénone-4-acétique ou encore l'acide 3-(4-(1,2-diphénylbut-1-ényl)phényl)acrylique.

La composition pharmaceutique telle que décrite peut être utile en particulier pour le traitement des maladies prolifératives, telles que le cancer, le psoriasis ou la fibrose, et en particulier le cancer.

La présente invention concerne également l'utilisation d'une composition pharmaceutique comprenant :
(iii) au moins un composé de formule (I) ou un composé choisi parmi 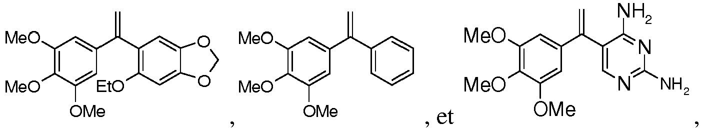
(iv) au moins un autre principe actif, notamment notamment utile pour le traitement de maladies prolifératives telles que le cancer, le psiorasis ou la fibrose, et avantageusement un agent anti-cancéreux tel qu'un agent anti-vasculaire, cytotoxique ou anti-angiogénique,
en tant que produits de combinaison pour une utilisation simultanée, séparée ou étalée dans le temps, pour la fabrication d'un médicament destiné au traitement de maladies prolifératives, telles que le cancer, le psoriasis ou la fibrose, et en particuleir le cancer.

L'invention va maintenant être illustrée par les exemples 1 à 5 et les figures 1 à 5 qui suivent.
La Figure 1 illustre l'effet du composé (I-1) sur la répartition des cellules étudiées selon les différentes phases du cycle cellulaire après 24 heures de traitement.
La Figure 2 illustre l'induction de l'apoptose dans des cellules leucémiques (K562, HCT116, MDA-MB 231) traitées pendant 24 heures avec le composé (I-1). L'apoptose est mise en évidence par la mesure de l'activité enzymatique des caspases 3 et 7 et les résultats sont exprimés en % par rapport aux cellules traitées pendant 24 heures avec du DMSO à 0,1 %.
La Figure 3 illustre l'activité cytotoxique du composé (I-1) sur les cellules endothéliales humaines EAhy926, mesurée immédiatement à la fin du traitement par le composé (I-1).
La Figure 4 illustre l'activité cytotoxique du composé (I-1) sur les cellules endothéliales humaines EAhy926 mesurée après 72 heures (CI₅₀ = 2 nM).
La Figure 5 illustre l'activité anti-vasculaire du composé (I-1), en comparaison avec du NaCl 0,9% et du DMXAA, chez la souris *Nude* et représente des photos obtenues par imagerie par résonance magnétique de tumeurs avant et après injection de Dotarem.

### Exemple 1 : synthèse

### 1.1. Composé de formule (I-5)

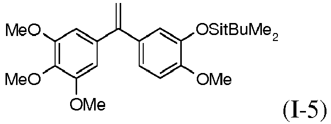

A -78°C, on additionne 1 mmol de *t*BuLi (2 eq) à une solution contenant 0,5 mmol de *t*butyl[(5-iodo-2-méthoxybenzyl)]diméthylsilane dissous dans 15 mL d'hexane distillé. Après 45 minutes d'agitation à cette température on ajoute 0,5 mmol de 3,4,5-triméthoxyacétophénone dilué dans 5 mL de toluène distillé. Ce mélange est agité 12 heures en laissant progressivement remonter la température puis est lentement hydrolysé par une solution de NH₄Cl saturée jusqu'à pH = 7-8. Après extraction à l'éther diéthylique (3 x 20 mL), les phases organiques réunies sont séchées sur Na₂SO₄ et concentrées à l'évaporateur rotatif. Le brut réactionnel est repris dans 10 mL de CH₂Cl₂ auxquels on additionne quelques grains d'acide *para-*toluènesulfonique (APTS) hydraté puis est agité 3 heures à température ambiante. La solution est lavée par une solution saturée de NaCl, extraite au CH₂Cl₂. Après séchage sur Na₂SO₄ et concentration à l'évaporateur rotatif, on recueille une huile qui est purifiée sur gel de silice. Rendement 55%.
¹H RMN: δ ppm, CD₃COCD₃, 300 MHz: 0,15 (s, 6H), 0,98 (s, 9H), 3,75 (s, 3H), 3,78 (s, 3H), 3,95 (s, 3H), 5,33 (d, 1H, *J* = 1,2 Hz), 5,34 (d, 1H, *J* = 1,2 Hz), 6,60 (s, 2H), 6,83 (t, 1H, *J* = 1,2 Hz), 6,96 (m, 2H). Analyse élémentaire: (MM = 430,22) Calculé C: 66,94, H: 7,96; Trouvé C: 66,85, H: 7,92.

### 1.2. Composé de formule (I-1)

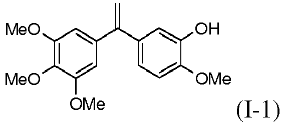

Le composé silylé (I-5) (0,17 mmol) est dissous dans 10 mL de méthanol auquel on ajoute 0,25 mmol de K₂CO₃. La solution est agitée à température ambiante pendant 12 heures puis est lavée par une solution saturée de NaCl. La phase aqueuse est extraite par de l'acétate d'éthyle (3 x 10 mL). Les phases organiques réunies sont séchées sur Na₂SO₄ et concentrées à l'évaporateur rotatif pour donner un résidu purifié sur gel de silice. Rendement 94%.
¹H RMN: δ ppm, CDCl₃, 300 MHz: 3,81 (s, 6H), 3,87 (s, 3H), 3,91 (s, 3H), 5,30 (d, 1H, *J* = 1,5 Hz), 5,37 (d, 1H, *J* = 1,5 Hz), 5,60 (sl, 1H), 6,55 (s, 2H), 6,82 (m, 2H), 6,97 (d, 1H, *J* = 2,1 Hz). Spectrométrie de masse (ESI) [M+Na]⁺ = 339. Analyse élémentaire: (MM = 316,13) Calculé C: 68,34, H: 6,37; Trouvé C: 68,25, H: 6,33.

### 1.3. Synthèse des composés (I-2), (I-3) et (I-4) par action d'organomagnésiens commerciaux

### 1.3.1. Composé de formule (I-2)

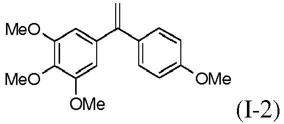

A 0°C et sous atmosphère d'argon, on additionne lentement au goutte à goutte une solution commerciale de bromure de (4-méthoxyphényl) magnésium (2,2 mmol) à une solution contenant 1 mmol de 3,4,5-triméthoxyacétophénone diluée dans 5 mL de tétrahydrofurane (THF) distillé. La solution est agitée 12 heures à température ambiante puis est hydrolysée par ajout d'une solution saturée de NH₄Cl jusqu'à pH = 7-8. Après extraction au dichlorométhane (3 x 20 mL), les phases organiques réunies sont séchées sur Na₂SO₄ et concentrées à l'évaporateur rotatif. Le brut réactionnel est ensuite traité comme pour (I-1) à l'acide *para*-toluènesulfonique pour conduire après purification sur gel de silice au dérivé attendu. Rendement 64%. ¹H RMN: δ ppm, CD₃COCD₃, 300 MHz: 3,75 (s, 3H), 3,78 (s, 6H), 3,82 (s, 3H), 5,34 (m, 2H), 6,60 (s, 2H), 6,92 (d, 2H, *J* = 8,7 Hz), 7,29 (d, 2H, *J* = 8,7 Hz). Analyse élémentaire: (MM = 300,14) Calculé C: 71,98, H: 6,71; Trouvé C: 71,85, H: 6,66.

### 1.3.2. Composé de formule (I-3)

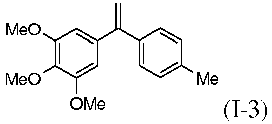

Il a été préparé selon le mode opératoire décrit pour le composé de formule (I-2) à partir de bromure d'*ortho*-tolylmagnésium et de 3,4,5-triméthoxyacétophénone.
Rendement 54%.
¹H RMN: δ ppm, CD₃COCD₃, 300 MHz: 2,33 (s, 3H), 3,75 (s, 3H), 3,76 (s, 6H), 5,38 (d, 1H, *J* = 1,2 Hz), 5,40 (d, 1H, *J* = 1,2 Hz), 6,59 (s, 2H), 7,22-7,25 (m, 4H). Analyse élémentaire: (MM = 284,14) Calculé C: 76,03, H: 7,09; Trouvé C: 75,74, H: 6,99.

### 1.3.3. Composé de formule (I-4)

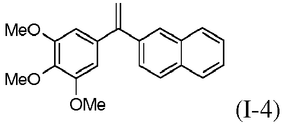

Il a été préparé à partir de bromure de 2-naphtylmagnésium et de 3,4,5-triméthoxyacétophénone selon le mode opératoire décrit pour le composé de formule (I-2). Rendement 81%.
¹H RMN: δ ppm, CD₃COCD₃, 300 MHz: 3,77 (s, 9H), 5,54-5,64 (m, 2H), 6,67 (s, 2H), 7,50-7,55 (m, 3H), 7,87-7,91 (m, 4H). Analyse élémentaire: (MM = 320,14) Calculé C: 78,73, H: 6,29; Trouvé C: 78,64, H: 6,20.

### 1.4. Composé de formule (I-10)

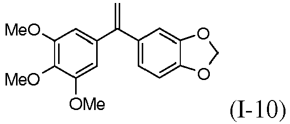

A -78°C, on additionne 1 mmol de *t*BuLi (2 eq) à une solution contenant 0,5 mmol de 5-bromo-benzo[1,3]dioxole dissous dans 15 mL d'hexane distillé. Après 45 minutes d'agitation à cette température on ajoute 0,5 mmol de 3,4,5-triméthoxyacétophénone diluée dans 5 mL de toluène distillé. Ce mélange est agité 12 heures en laissant progressivement remonter la température puis est lentement hydrolysé par une solution de NH₄Cl saturée jusqu'à pH = 7-8. Après extraction à l'éther diéthylique (3 x 20 mL), les phases organiques réunies sont séchées sur Na₂SO₄ et concentrées à l'évaporateur rotatif. Le brut réactionnel est repris dans 10 mL de CH₂Cl₂ auxquels on additionne quelques grains d'APTS hydraté puis est agité 3 heures à température ambiante. La solution est lavée par une solution saturée de NaCl, extraite au CH₂Cl₂. Après séchage sur Na₂SO₄ et concentration à l'évaporateur rotatif, on recueille une huile qui est purifiée sur gel de silice. Rendement 19%.
¹H RMN: δ ppm, CDCl₃, 300 MHz: 3,72 (s, 6H), 3,78 (s, 3H), 5,21 (d, 1H, *J* = 1,5 Hz), 5,25 (d, 1H, *J* = 1,5 Hz), 5,86 (s, 2H), 6,46 (s, 2H), 6,67 (d, 1H, *J* = 8,7 Hz), 6,72-6,76 (m, 2H). Spectrométrie de masse (ESI) [M+Na]⁺ = 337. Analyse élémentaire: (MM = 314,12) Calculé C: 68,78, H: 5,77; Trouvé C: 68,68, H: 5,72.

### 1.5. Composé de formule (I-13)

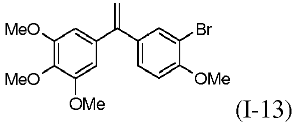

A -100°C, on additionne 1 mmol de *n*BuLi (1 eq) à une solution contenant 1 mmol de 2-bromo-4-iodo-1-méthoxy-benzène dissous dans 15 mL d'hexane distillé. Après 45 minutes d'agitation à cette température on ajoute 0,5 mmol de 3,4,5-triméthoxyacétophenone diluée dans 5 mL de toluène distillé. Ce mélange est agité 12 heures en laissant progressivement remonter la température puis est lentement hydrolysé par une solution de NH₄Cl saturée jusqu'à pH = 7-8. Après extraction à l'éther diéthylique (3 x 20 mL), les phases organiques réunies sont séchées sur Na₂SO₄ et concentrées à l'évaporateur rotatif. Le brut réactionnel est repris dans 10 mL de CH₂Cl₂ auxquels on additionne quelques grains d'APTS hydraté puis est agité 3 heures à température ambiante. La solution est lavée par une solution saturée de NaCl, extraite au CH₂Cl₂. Après séchage sur Na₂SO₄ et concentration à l'évaporateur rotatif, on recueille une huile qui est purifiée sur gel de silice. Rendement 53%.
¹H RMN: δ ppm, CDCl₃, 300 MHz: 3,65 (s, 3H), 3,78 (s, 6H), 3,85 (s, 3H), 5,30 (s, 1H), 5,70 (s, 1H), 6,50 (s, 2H), 6,80 (d, 1H, *J* = 8,7 Hz), 7,36-7,46 (m, 2H). Spectrométrie de masse (ESI) [M+Na]⁺ = 403. Analyse élémentaire: (MM = 378,05) Calculé C: 57,01, H: 5,05; Trouvé C: 56,78, H: 4,90.

### 1.6. Composé de formule (I-14)

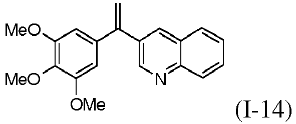

A -100°C, on additionne 1 mmol de *t*BuLi (1 eq) à une solution contenant 1 mmol de 3-bromoquinoléine dissoute dans 15 mL d'éther diéthylique distillé. Après 2 heures d'agitation à cette température on ajoute 1 mmol de 3,4,5-triméthoxyacétophenone diluée dans 5 mL d'éther diéthylique. Ce mélange est agité 2 heures en laissant progressivement remonter la température puis est lentement hydrolysé par une solution de NH₄Cl saturée jusqu'à pH = 7-8. Après extraction à l'éther diéthylique (3 x 20 mL), les phases organiques réunies sont séchées sur Na₂SO₄ et concentrées à l'évaporateur rotatif. Le brut réactionnel est repris dans 10 mL de CH₂Cl₂ auxquels on additionne 4 mmol de diméthylaminopyridine (DMAP) et 2,4 mmol de chlorure de mésyle. L'ensemble est agité 1 heure à température ambiante et la solution est lavée par une solution saturée de NaCl puis extraite au CH₂Cl₂. Après concentration le brut réactionnel à nouveau repris dans 15 mL de CH₂Cl₂ auxquels on additionne 27 mmol de 1,8-diazabicyclo[5.4.0]undec-7-ène (DBU). L'ensemble est porté à reflux pendant 3 heures. Après lavage par une solution de NaCl, extraction au CH₂Cl₂, et séchage sur Na₂SO₄ on recueille un solide blanc qui est purifié sur gel de silice. Rendement global 36%.
¹H RMN: δ ppm, CDCl₃, 300 MHz: 3,77 (s, 6H), 3,80 (s, 3H), 5,52 (s, 2H), 6,51 (s, 2H), 7,41-7,50 (t, 1H, *J* = 6,9 Hz), 7,60-7,64 (t, 1H, *J* = 6,9 Hz), 7,72 (d, 1H, *J* = 6,9 Hz), 7,96-8,08 (m, 2H), 8,88 (d, 1H, *J* = 2,1 Hz). Analyse élémentaire: (MM = 321,14) Calculé C: 74,75, H: 5,96; Trouvé C: 74,61, H: 5,90.

### 1.7. Composé de formule (I-6)

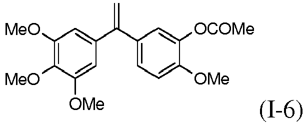

A une solution du composé (I-1) (0,316 mmol) dissous dans 1 mL de CH₂Cl₂ sont ajoutés 54 µL de pyridine et 0,016 mmol de DMAP. Le mélange est refroidi à 0°C, et 42 µL d'anhydride acétique (0,442 mmol) sont additionnés lentement. Après 1 heure d'agitation à 0°C, le mélange réactionnel est hydrolysé (H₂O, 3 mL) puis extrait à l'acétate d'éthyle (3 x 3 mL). Les phases organiques sont rassemblées, séchées sur sulfate de sodium, et concentrées pour donner un résidu qui est purifié sur gel de silice. Rendement 65%.
¹H RMN: δ ppm, CDCl₃, 300 MHz: 2,28 (s, 3H), 3,74 (s, 6H), 3,78 (s, 3H), 3,84 (s, 3H), 5,26 (d, 1H, *J* = 1,5 Hz), 5,31 (d, 1H, *J* = 1,5 Hz), 6,48 (s, 2H), 6,86 (d, 1H, *J* = 8,7 Hz), 6,97 (d, 1H, *J* = 2,1 Hz), 7,16 (dd, 1H, *J* = 8,4 Hz, *J* = 2,1 Hz). Spectrométrie de masse (ESI) [M+Na]⁺ = 381. Analyse élémentaire: (MM = 358,14) Calculé C: 67,03, H: 6,19; Trouvé C: 66,88, H: 6,06.

### 1.8. Composé de formule (I-7)

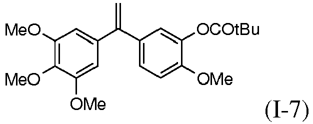

Le composé (I-7) a été préparé de façon similaire à (I-6) à partir de l'anhydride pivaloïque. Rendement 74%. ¹H RMN: δ ppm, CDCl₃, 300 MHz: 1,28 (s, 9H), 3,74 (s, 6H), 3,80 (s, 3H), 3,84 (s, 3H), 5,26 (d, 1H, *J* = 1,5 Hz), 5,30 (d, 1H, *J* = 1,5 Hz), 6,48 (s, 2H), 6 ,82 (d, 1H, *J* = 12,6 Hz), 6,95 (d, 1H, *J* = 3,3 Hz), 7,10 (dd, 1H, *J* = 12,6 Hz, *J* = 3,3 Hz). Spectrométrie de masse (ESI) [M+Na]⁺ = 423. Analyse élémentaire: (MM = 400,19) Calculé C: 68,98, H: 7,05; Trouvé C: 68,69, H: 6,96.

### 1.9. Composé de formule (I-8)

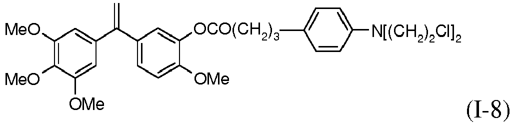

A une solution du composé (I-1) (0,316 mmol) dilué dans 5 mL de CH₂Cl₂ sont ajoutés 0,376 mmol de 1-éthyl-3-(3'-diméthylaminopropyl)carbodiimide (EDCI), 0,347 mmol de DMAP et 0,347 mmol de chlorambucil. Après 1 heure d'agitation à température ambiante, le mélange réactionnel est hydrolysé à l'aide d'une solution aqueuse saturée de NaHCO₃ et extrait à l'acétate d'éthyle (3 x 3 mL). Les phases organiques sont rassemblées, séchées sur sulfate de sodium et concentrées pour donner un résidu qui est purifié sur gel de silice. Rendement 70%.
¹H RMN: δ ppm, CDCl₃, 300 MHz: 1,98-2,10 (m, 2H), 2,59 (t, 2H, *J* = 7,5 Hz), 2,67 (t, 2H, *J* = 7,2 Hz), 3,6-3,75 (m, 8H), 3,82 (s, 6H), 3,86 (s, 3H), 3,88 (s, 3H), 5,35 (d, 1H, *J* = 1,0 Hz), 5,40 (d, 1H, *J* = 1,0 Hz), 6,56 (s, 2H), 6,68 (d, 2H, *J* = 8,7 Hz), 6,94 (d, 1H, *J* = 8,7 Hz), 7,03 (d, 1H, *J* = 2,4 Hz), 7,12 (d, 2H, *J* = 8,7 Hz), 7,25 (dd, 1H, *J* = 8,7 Hz, *J* = 2,1 Hz). Spectrométrie de masse (ESI) [M+Na]⁺ = 624. Analyse élémentaire: (MM = 601,20) Calculé C: 63,79, H: 6,19; Trouvé C: 63,68, H: 6,16.

### 1.10. Composé de formule (I-9)

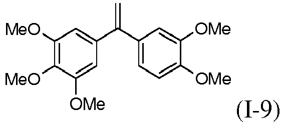

A une solution du composé (I-1) (0,158 mmol) dilué dans 5 mL d'acétone sont ajoutés 0,632 mmol de K₂CO₃ et 0,632 mmol de sulfate de diméthyle. Après 12 heures d'agitation à température ambiante, le mélange réactionnel est hydrolysé à l'aide d'une solution aqueuse et extrait à l'acétate d'éthyle (3 x 3 mL). Les phases organiques sont rassemblées, séchées sur sulfate de sodium, et concentrées pour donner un résidu qui est purifié sur gel de silice. Rendement 80%.
¹H RMN: δ ppm, CDCl₃, 300 MHz: 3,80 (s, 6H), 3,84 (s, 3H), 3,87 (s, 3H), 3,89 (s, 3H), 5,33 (d, 1H, *J* = 1,5 Hz), 5,36 (d, 1H, *J* = 1,5 Hz), 6,56 (s, 2H), 6,83 (d, 1H, J = 8,4 Hz), 6,88-6,92 (m, 2H). Spectrométrie de masse (ESI) [M+Na]⁺ = 353. Analyse élémentaire: (MM = 330,15) Calculé C: 69,07, H: 6,71; Trouvé C: 68,85, H: 6,56.

### 1.11. Composé de formule (I-12)

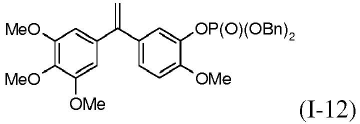

Le composé (I-1) (0,136 mmol) est dilué dans un mélange composé de 153 µL de tétrachlorure de carbone et 1,3 mL d'acétonitrile sec à -25 °C. Après 10 minutes d'agitation, sont ajoutés successivement de la diisopropyléthylamine (0,663 mmol), de la diméthylaminopyridine (0,0136 mmol) et du phosphite de dibenzyle (0,458 mmol) au milieu réactionnel. Après 1 heure 30 min d'agitation à -25°C, le mélange réactionnel est hydrolysé par une solution aqueuse saturée de KH₂PO₄ et extrait avec de l'acétate d'éthyle (3 x 3 mL). Les phases organiques sont rassemblées, séchées sur sulfate de sodium, et concentrées pour donner un résidu qui est purifié sur gel de silice. Rendement 40%.
¹H RMN: δ ppm, CDCl₃, 300 MHz: 3,81 (s, 6H), 3,82 (s, 3H), 3,88 (s, 3H), 5,17 (d, 4H, *J* = 7,8 Hz,), 5,32 (s, 1H), 5,33 (d, 1H, *J* = 0,6 Hz), 6,55 (s, 2H), 6,89 (d, 1H, *J* = 8,4 Hz), 7,14 (m, 1H), 7,23 (t, 1H, *J* = 7,3 Hz), 7,23-7,4 (m, 10 H). Spectrométrie de masse (ESI) [M+Na]⁺ = 599. Analyse élémentaire: (MM = 576,19) Calculé C: 66,66, H: 5,77; Trouvé C: 66,58, H: 5,72.

### 1.12. Composé de formule (I-11)

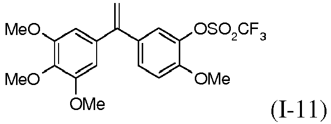

A une solution du composé (I-1) (0,78 mmol) dans 5 mL de CH₂Cl₂ sont ajoutées successivement à -10 °C : 3,11 mmol de pyridine et 1,17 mmol d'anhydride triflique dilué dans 5 mL de CH₂Cl₂. L'ensemble est agité 1 heure à température ambiante avant d'être hydrolysé par une solution 1M d'acide chlorhydrique (5 mL). La phase organique est séparée et la phase aqueuse est extraite au CH₂Cl₂ (2 x 10 mL). Les phases organiques sont rassemblées, séchées sur sulfate de sodium, et concentrées pour donner un résidu qui est purifié sur gel de silice. Rendement 90%.
¹H RMN: δ ppm, CDCl₃, 300 MHz: 3,84 (s, 6H), 3,90 (s, 3H), 3,96 (s, 3H), 5,42 (m, 2H), 6,54 (s, 2H), 7,02 (d, 1H, *J* = 8,4 Hz), 7,24 (d, 1H, *J* = 2,2 Hz), 7,36 (dd, 1H, *J* = 8,4 Hz, *J* = 2,2 Hz). Analyse élémentaire: (MM = 448,08) Calculé C: 50,89, H: 4,27; Trouvé C: 50,78, H: 4,21.

### 1.13. Composé de formule (I-15)

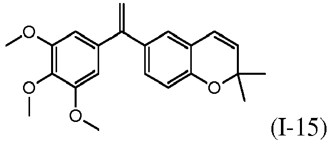

Ce composé a été préparé selon le mode opératoire décrit pour le composé de formule (I-2) à partir du dérivé lithié préparé à partir du 6-iodo-2,2-diméthyl-2H-chromène.
Rendement 37%.
¹H RMN: δ, ppm, CDCl₃, 300 MHz: 1,43 (s, 6H), 3,82 (s, 6H), 3,88 (s, 3H), 5,29 (d, 1H, *J* = 1,2 Hz), 5,36 (d, 1H, *J* = 1,2 Hz), 5,62 (d, 1H, *J* = 10.0 Hz), 6,30 (d, 1H, *J* = 10,0 Hz), 6,65 (s, 2H), 6,73 (d, 1H, *J* = 8,4 Hz), 6,88 (d, 1H, *J* = 2,4 Hz), 7.11 (dd, 1H, *J* = 8,4 Hz, *J* = 2,4 Hz). Analyses élémentaires: (MM = 352.17) Calculé C: 74,98, H: 6,86; Trouvé C: 74,11, H: 6,94.

### 1.14. Composé de formule (I-16)

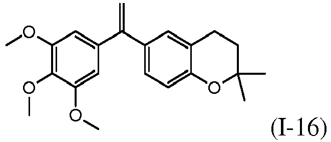

Ce composé a été préparé selon le mode opératoire décrit pour le composé de formule (I-2) à partir du dérivé lithié préparé à partir du 6-iodo-2,2-diméthyl-2H-chroman.
Rendement 32%.
¹H RMN: δ, ppm, CDCl₃, 300 MHz: 1,35 (s, 6H), 1,88 (t, 2H, *J* = 6,6 Hz), 2,76 (t, 2H, *J* = 6,6 Hz), 3,82 (s, 6H), 3,88 (s, 3H), 5,26 (d, 1H, *J* = 1,2 Hz), 5,35 (d, 1H, *J* = 1,2 Hz), 6,57 (s, 2H), 6,74 (d, 1H, *J* = 8,1 Hz), 7,08 (s, 1H), 7,09 (d, 1H, *J* = 8,1 Hz). Analyses élémentaires: (MM = 354.18) Calculé C: 74,55, H: 7,39; Trouvé C: 74,50, H: 7,36.

### 1.15. Composé de formule (I-17)

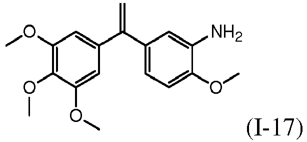

Sous atmosphère inerte, à -40°C, à une solution composée de 560 mg de (3-amino-4-méthoxy)acétophenone (3,39 mmol, 1 éq.) dans 20 mL de tétrahydrofurane (THF), sont ajoutés, goutte à goutte, 11,9 mL d'une solution molaire de bromure de 3,4,5-triméthoxyphénylmagnésium (3,5 éq.). Le milieu réactionnel est agité à cette température pendant 3 heures puis une nuit à température ambiante pour être ensuite hydrolysé par une solution saturée en chlorure d'ammonium. Le milieu réactionnel est extrait 3 fois par de l'acétate d'éthyle (3 x 30 mL). Les phases organiques réunies sont lavées par une solution saturée en chlorure de sodium, séchées sur sulfate de sodium, filtrées et concentrées sous vide. Le résidu est chromatographié sur gel de silice (Cyclohexane / Acétate d'éthyle : 6/4), pour conduire à l'intermédiaire alcool secondaire. Celui-ci est ensuite mis en solution avec quelques grains d'acide *para-*toluènesulfonique (APTS) dans 20 mL de dichlorométhane, sous agitation, à température ambiante durant 30 minutes. Après addition de 20 mL d'eau, le milieu réactionnel est extrait 3 fois au dichlorométhane (3 x 20mL). Les phases organiques réunies sont séchées sur sulfate de magnésium, filtrées puis concentrées sous vide. Le résidu est chromatographié sur gel de silice
Rendement 12%.
¹H RMN: δ, ppm, CD₃COCD₃, 200 MHz: 3,80 (s, 3H), 3,64 (s, 6H), 3,70 (s, 3H), 4,38 (s, 2H), 5,26 (d, 1H, *J* = 1,6 Hz), 5,29 (d, 1H, *J* = 1,6 Hz), 6,61 (s, 2H), 6,61 (dd, 1H, *J* = 8,4 Hz, *J* = 2,2 Hz), 6,71 (d, 1H, *J* = 2,2 Hz), 6,79 (d, 1H, *J* = 8,4 Hz). Analyses élémentaires: (MM = 315,15) Calculé C: 68,55, H: 6,71, N, 4,44; Trouvé C: 68,50, H: 6,67, N, 4.38.

### 1.16. Composé de formule (I-18)

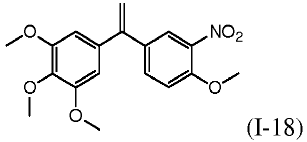

Sous atmosphère inerte, 1,07 g de bromure de méthyl triphénylphosphonium (3 mmol, 1 éq.) sont dilués dans 10 mL de THF. Puis, 2,83 mL d'une solution molaire de lithium hexamethyldisilazide (LiHMDS) dans le THF (3 mmol) sont additionnés lentement goutte à goutte à 0 °C. Le milieu réactionnel est agité à 0 °C durant 1 heure. La solution vire au jaune vif. Puis une solution de 520 mg de diarylcétone (1,5 mmol) dans 10 mL de THF est additionnée goutte à goutte à 0°C. Le mélange est laissé sous agitation durant 30 minutes sous atmosphère inerte à 0 °C puis à température ambiante. On ajoute au milieu 1 mL d'eau puis le milieu est concentré sous vide. Le résidu est dissout dans 20 mL de dichlorométhane puis est lavé 3 fois à l'eau. La phase organique est séchée sur MgSO₄ puis condensée sous vide. Le résidu est chromatographié sur gel de silice
Rendement 70%
¹H RMN: δ, ppm, CDCl₃, 300 MHz: 3,77 (s, 6H), 3,83 (s, 3H), 3,89 (s, 3H), 6,44 (s, 2H), 7,14 (dd, 1H, *J* = 8,4 Hz, *J* = 2,7 Hz), 7,34 (d, 1H, *J* = 8,4 Hz), 7,42 (d, 1H, *J* = 2,7 Hz). Spectrométrie de masse (ESI) [M+Na]⁺ = 368.

### 1.17. Composé de formule (I-19)

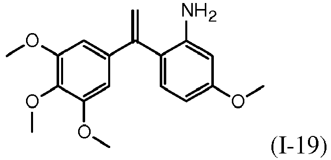

86 mg de composé (I-18) (0,25 mmol, 1 eq) sont dissouts dans 5 mL d'acide acétique glacial. Après addition de 98 mg de zinc (1,5 mmol, 6 eq), le milieu réactionnel est laissé sous agitation à température ambiante durant 1 heure. Après filtration sur célite puis condensation sous vide, le résidu est repris dans 15 mL d'acétate d'éthyle puis lavé 3 fois à l'eau. La phase organique est séchée sur MgSO₄ puis condensée sous vide. Le résidu est chromatographié sur gel de silice Rendement 46%.
¹H RMN: δ, ppm, CDCl₃, 300 MHz: 3,79 (s, 3H), 3,80 (s, 6H), 3,85 (s, 3H), 5,28 (d, 1H, *J* = 1,5 Hz), 5,65 (d, 1H, *J* = 1,5 Hz), 6,24 (d, 1H, *J* = 2,7 Hz), 6,59 (s, 2H), 7,43 (dd, 1H, *J* = 8,4 Hz, J = 2,7 Hz), 7,03 (d, 1H, *J* = 8,4 Hz). Spectrométrie de masse (ESI) [M+Na]⁺ = 338.

### 1.18. Composé de formule (I-20)

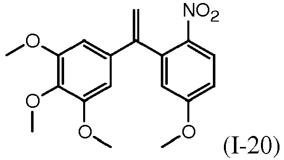

Ce composé a été préparé selon le mode opératoire décrit pour le composé de formule (I-18) à partir de la diarylcétone correspondante.
Rendement 54%
¹H RMN: δ, ppm, CDCl₃, 300 MHz: 3,79 (s, 3H), 3,77 (s, 6H), 3,83 (s, 3H), 3,92 (s, 3H), 6,45 (s, 2H), 6,91 (d, 1H, *J* = 3,0 Hz), 6,96 (dd, 1H, *J* = 9,0 Hz, *J* = 3,0 Hz), 8,05 (d, 1H, *J* = 9,0 Hz). Spectrométrie de masse (ESI) [M+Na]⁺ = 368.

### 1.19. Composé de formule (I-21)

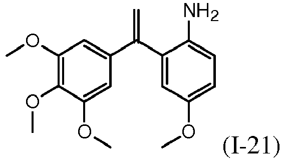

Ce composé a été préparé selon le mode opératoire décrit pour le composé de formule (I-19) à partir de la diarylcétone correspondante.
Rendement 70%
¹H RMN: δ, ppm, CDCl₃, 300 MHz: 3,77 (s, 3H), 3,80 (s, 6H), 3,85 (s, 3H), 3,92 (s, 3H), 5,32 (d, 1H, *J* = 1,5 Hz), 5,71 (d, 1H, *J* = 1,5 Hz), 6,59 (s, 2H), 6,66 (d, 1H, J = 8,4 Hz), 6,72-6,79 (m, 2H). Spectrométrie de masse (ESI) [M+Na]⁺ = 338.

### 1.20. Composé de formule (I-22)

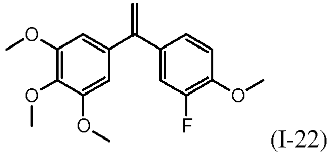

Ce composé a été préparé selon le mode opératoire décrit pour le composé de formule (I-5) à partir de 3,4,5-triméthoxyacétophénone et de 2-fluoro-4-iodoanisole. Rendement 48%
¹H RMN: δ, ppm, CDCl₃, 300 MHz: 3,82 (s, 6H), 3,88 (s, 3H), 3,92 (s, 3H), 3,92 (s, 3H), 5,35 (d, 1H, *J* = 1,5 Hz), 5,38 (d, 1H, *J* = 1,5 Hz), 6,58 (s, 2H), 6,95 (m, 1H), 7,05-7,19 (m, 2H). Spectrométrie de masse (ESI) [M+Na]⁺ = 341.

### 1.21. Composé de formule (I-23)

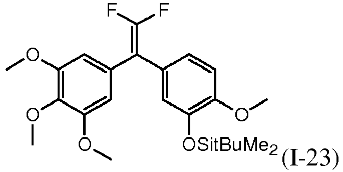

Ce composé a été préparé selon le mode opératoire décrit pour le composé de formule (I-18) à partir de la diarylcétone correspondante
Rendement 60%
¹H RMN: δ, ppm, CDCl₃, 300 MHz: 0,00 (s, 6H), 0,83 (s, 9H), 3,70 (s, 6H), 3,72 (s, 3H), 3,76 (s, 3H), 6,40 (s, 2H), 6,68 (s, 1H), 6,72 (s, 2H). Analyses élémentaires: (MM = 466.20) Calculé C: 61,78, H: 6,91; Trouvé C: 66,70, H: 6,84.

### 1.22. Composé de formule (I-24)

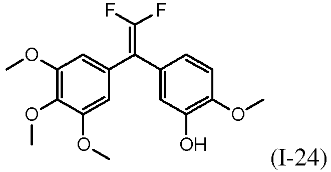

Ce composé a été préparé selon le mode opératoire décrit pour le composé de formule (I-1) à partir de (I-23).
Rendement 89%
¹H RMN: δ, ppm, CDCl₃, 300 MHz: 3,85 (s, 6H), 3,92 (s, 3H), 3,99 (s, 3H), 5,68 (s, 1H), 6,50 (s, 2H), 6,80-6,98 (m, 3H). Spectrométrie de masse (ESI) [M+Na]⁺ = 375,2.

### 1.23. Composé de formule (I-25)

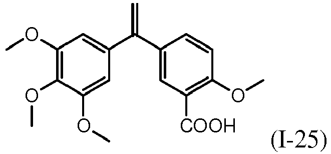

A -78°C, on introduit lentement dans un ballon contenant 400 mg de (I-13) dans 10 mL d'éther diéthylique, 0,85 mL de BuLi (1,6 M). L'ensemble est mis à réagir 1h 30 à -70°C puis 2 g de CO₂ sous forme de carboglace sont additionnés au milieu réactionnel à -78°C. Après 30 minutes d'agitation à -78°C on laisse la solution revenir à température ambiante. Le brut réactionnel est hydrolysé par une solution saturée de NH₄Cl et est extrait à l'éther diéthylique (10 mL). La phase organique est traitée par une solution de soude (1M; 10 mL) et après décantation, la phase aqueuse est reprise par une solution d'HCl 1M (15 mL). Après extraction à l'éther diéthylique (3 x 10 mL), les phases organiques réunies sont lavées par une solution saturée en chlorure de sodium, séchées sur sulfate de sodium, filtrées et concentrées sous vide et le résidu est chromatographié sur gel de silice.
Rendement 44%
¹H RMN: δ, ppm, CDCl₃, 300 MHz: 3,68 (s, 3H), 3,71 (s, 6H), 3,76 (s, 3H), 5,25 (m, 1H), 5,64 (m, 1H), 6,42 (s, 2H), 6,88 (d, 1H, *J* = 8,6 Hz), 7,94 (d, 1H, *J* = 2,2 Hz), 8,05 (dd, 1H, *J* = 8,6 Hz, J = 2,2 Hz). Analyses élémentaires: (MM = 344,13) Calculé C: 66,27, H: 5,85; Trouvé C: 66,19, H: 5,80.

### 1.24. Composé de formule (I-26)

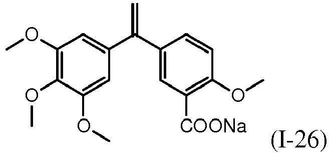

344 mg de (I-25) et 40 mg de NaOH sont dissouts dans 5 mL de MeOH. Après totale dissolution, le milieu est concentré au rotavapor puis sous le vide d'une pompe à palette.
Rendement 100%
¹H RMN: δ, ppm, D₂O, 300 MHz: 3,72 (s, 12H), 5,32 (s, 1H), 5,79 (s, 1H), 6,60 (s, 2H), 7,08 (d, 1H, *J* = 8,8 Hz), 7,80 (m, 1H), 7,90 (m, 1H). Analyses élémentaires: (MM = 366,11) Calculé C: 62,29, H: 5,23; Trouvé C: 62,07, H: 5,12.

### 1.25. Composé de formule (I-27)

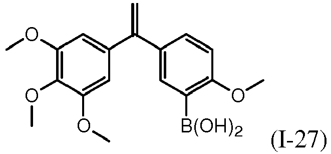

A -78°C, on introduit lentement dans un ballon contenant 342 mg de (I-13) dans 5 mL de THF, 0.56 mL de BuLi (1,6 M). L'ensemble est mis à réagir 30 minutes à -70°C et le brut réactionnel est mélangé à température ambiante jusqu'à dissolution complète puis l'ensemble est à nouveau refroidit à -78°C. 0,21 g de triisopropylborate sont additionnés lentement au milieu réactionnel à -78°C et la solution est lentement ramenée à température ambiante. Après 10 minutes d'agitation, 5 mL d'H₂O sont ajoutés au brut réactionnel puis 20 mL d'éther et 15 mL d'une solution saturée de NH₄Cl. Après extraction, les phases organiques réunies sont séchées sur sulfate de sodium, filtrées et concentrées sous vide.
Rendement 89%
¹H RMN: δ, ppm, CDCl₃, 300 MHz: 3,80 (s, 3H), 3,84 (s, 6H), 3,91 (s, 3H), 5,40 (d, 1H, *J* = 1,5 Hz), 5,80 (d, 1H, *J* = 1,5 Hz), 6,58 (s, 2H), 7,02 (d, 1H, *J* = 7,8 Hz), 8,10 (d, 1H, *J* = 2,2 Hz), 8,28 (dd, 1H, *J* = 7,8 Hz, *J* = 2,2 Hz).

### 1.26. Composé de formule (I-28)

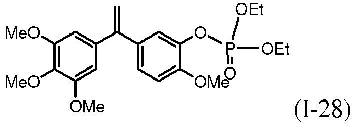

A une solution du composé (I-1) (1,15 mmol) dans 8 mL de THF sec est ajoutée 850 µL d'un mélange de pyridine/diisopropylethylamine (5:1) et 5,17 mmol de chlorure de diéthoxyphosphate goutte à goutte. Après une nuit d'agitation à température ambiante, le mélange réactionnel est hydrolysé et extrait avec de l'acétate d'éthyle (3 x 2 mL). Les phases organiques sont rassemblées, séchées sur sulfate de sodium, filtrées et le solvant est évaporé et le résidu est chromatographié sur gel de silice. Rendement 79%.
¹H NMR CDCl₃, 300 MHz δppm: 1,23 (td, 9H, *J₁* = 7 Hz , *J₂* = 1 Hz), 3,72 (s, 6H), 3,76 (s, 3H), 3,78 (s, 3H), 4,13 (qd, 8H, *J₁* = 8,4 Hz , *J₂* = 1,5 Hz), 5,25 (d, 1H, *J* = 1,2 Hz), 5,29 (d, 1H, *J* = 1,2 Hz), 6,46 (s, 2H), 6,82 (d, 1H, *J* = 8,7 Hz), 7,05 (m, 1H); 7,17 (t, 1H, *J* = 1,8 Hz). Spectroscopie de masse (APCI) [M+H]⁺ = 453.

### 1.27. Composé de formule (I-29)

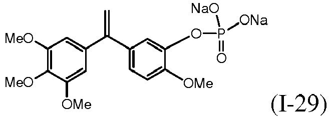

A une solution du composé (I-28) (0,126 mmol) dans 1 mL de CH₂Cl₂ sec est ajouté goutte à goutte à 0°C 0,252 mL d'iodure de triméthylsilane. Après une heure d'agitation à température ambiante, le mélange réactionnel est hydrolysé et extrait avec de l'acétate d'éthyle (2x 3 mL). Les phases organiques sont rassemblées, lavées avec une solution saturée de thiosulfate de sodium, séchées sur sulfate de sodium, filtrées et le solvant est évaporé. Le brut (23,2 mg) est repris dans une solution de 0,252 mmol de méthanoate de sodium et le brut est laissé encore une heure à température ambiante. Le solvant est ensuite évaporé et le brut est repris dans l'éther, filtré sur verre fritté et lavé à l'éther. Le composé attendu (très hygroscopique) est obtenu sous forme de poudre jaune.
Rendement 41 %.
Analyses élémentaires: (MM = 440,06) Calculé C: 49,10, H: 4,35; Trouvé C: 48,67, H: 4,00.

### 1.28. Composé de formule (I-30)

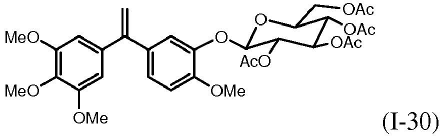

A une solution du composé (I-1) (0,316 mmol) dans 5 mL d'acétonitrile sec est ajouté 1,15 mmol de potasse. Le mélange est agité 20 min à et ensuite une solution d'α,-bromo-glucopyranose tétraacétylé (0,632 mmol) dans 5 mL d'acétonitrile est ajoutée goutte à goutte. Après une nuit d'agitation le mélange réactionnel est hydrolysé avec une solution de HCl 1N (5 mL), et extrait avec à l'acétate d'éthyle (3 x 5 mL). Les phases organiques sont rassemblées, séchées sur sulfate de sodium, filtrées et le solvant est évaporé et le résidu est chromatographié sur gel de silice.
Rendement 25%, 50% du produit de départ n'ayant pas réagi.
¹H NMR CDCl₃, 300 MHz δppm: 2,01 (s, 3H), 2,02 (s, 6H), 2,05 (s, 3H), 3,80 (s, 6H), 3,83 (s, 3H), 3,86 (s, 3H), 4,04-4,16 (m, 2H), 4,24 (dd, 1H, *J* = 12 Hz , *J* = 5,1 Hz), 4,99 (m, 1H), 5,13 (m, 1H), 5,24-5,30 (m, 2H), 5,31 (s, 1H), 5,35 (s, 1H), 6,52 (s, 2H), 6,83 (d, 1H, *J* = 8,4 Hz), 7,00 (dd, 1H, *J* = 8,4 Hz , *J* = 2,1 Hz), 7,18 (d, 1H, *J* = 2,4 Hz). Spectroscopie de masse (ESI) [M+Na]⁺ = 669,7.

### 1.29. Composé de formule (I-31)

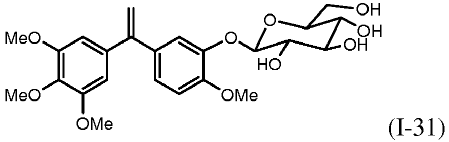

A une solution du composé (I-30) (0,078 mmol) dans 2 mL méthanol sec sont ajoutés 8 mL d'une solution ammoniacale à 28%. Après 2 h d'agitation à 60°C., le mélange réactionnel est hydrolysé avec une solution de HCl 1N, et extrait avec de l'acétate d'éthyle (3 x 5 mL). Les phases organiques sont rassemblées, séchées sur sulfate de sodium, filtrées et le solvant est évaporé et le résidu est chromatographié sur gel de silice.
Rendement 90%.
¹H NMR CDCl₃, 300 MHz δppm: 3,10-3,50 (m, 5H), 3,52 (d, 1H, *J* = 11,7 Hz), 3,68 (s, 6H), 3,73 (s, 6H), 3,78 (s, 3H), 4,48 (s, 1H), 4,86 (m, 1H), 4,94 (s, 1H), 5,03 (s, 1H), 5,18 (s, 1H), 5,34 (m, 1H), 5,45 (s, 1H), 6,55 (s, 2H), 6,86 (dd, 1H, *J* = 7,8 Hz , *J* = 1,5 Hz), 6,96 (d, 1H, *J* = 8,4 Hz), 7,12 (d, 1H, *J* = 1,5 Hz). Spectroscopie de masse (ESI) [M+Na]⁺ = 501.

### 1.30. Composé de formule (I-32)

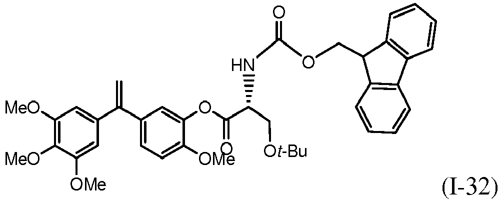

A une solution du composé (I-1) (0,79 mmol) dans 15 mL de CH₂Cl₂ sont ajoutés 0,94 mmol de chlorhydrate de 1-éthyl-3-(3-diméthylaminopropyl)carbodiimide (EDCI), 0,87 mmol de *N*,*N*-4-diméthylaminopyridine (DMAP) et 0,87 mmol de N-Fmoc sérine (O*t*-Bu) (sérine dont la fonction amine est protégée par un groupement 9-fluorénylméthoxycarbonyle (Fmoc) et dont la fonction acide est protégée par un groupement *tert*-butyle). Après une nuit d'agitation, le mélange réactionnel est hydrolysé avec une solution aqueuse saturée de NaHCO₃, et extrait à l'acétate d'éthyle (3 x 10 mL). Les phases organiques sont rassemblées, séchées sur sulfate de sodium, filtrées et le solvant est évaporé et le résidu est chromatographié sur gel de silice.
Rendement 39.%.
¹H NMR CDCl₃, 300 MHz δppm: 1,09 (s, 9H), 3,64 (dd, 1H, *J* = 9,0 Hz , *J* = 2,7 Hz), 3,73 (s, 6H), 3,76 (s, 3H), 3,79 (s, 3H), 3,95 (dd, 1H, *J* = 9,0 Hz , *J* = 3,0 Hz), 4,19 (t, 1H, *J* = 6,9 Hz), 4,27-4,39 (m, 2H), 4,72 (m, 1H), 5,26 (s, 1H), 5,31 (s, 1H), 6,67 (d, 1H, *J* = 9,0 Hz), 6,47 (s, 2H), 6,87 (d, 1H, *J* = 8,7 Hz), 6,98 (d, 1H, *J* = 2,1 Hz), 7,18 (dd, 1H, *J* = 8,4 Hz , *J* = 2,4 Hz), 7,23 (d, 2H, *J* = 7,5 Hz), 7,31 (t, 2H, *J =* 7,2 Hz), 7,53 (m, 2H), 7,68 (d, 2H, *J* = 7,2 Hz). Spectroscopie de masse (ESI) [M+Na]⁺ = 704.

### 1.31. Composé de formule (I-33)

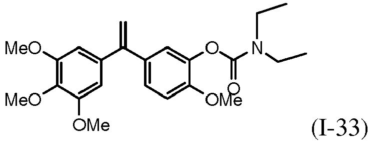

A une solution du composé (I-1) (0,316 mmol) dans 2 mL de CH₂Cl₂ sec sont ajoutés 54 µL de pyridine et 0,632 mmol de chlorure d'acide *N*,*N*-diéthylcarbamique. Après une nuit d'agitation à température ambiante, le mélange réactionnel est hydrolysé et extrait avec de l'acétate d'éthyle (3x 3 mL). Les phases organiques sont rassemblées, séchées sur sulfate de sodium, filtrées et le solvant est évaporé et le résidu est chromatographié sur gel de silice.
Rendement 50%.
¹H NMR CDCl₃, 300 MHz δppm: 1,11-1,20 (m, 6H), 3,28-3,39 (m, 4H), 3,75 (s, 6H), 3,77 (s, 3H), 3,80 (s, 3H), 5,25 (d, 1H, *J* = 0,9 Hz), 5,32 (d, 1H, *J* = 1,2 Hz), 6,50 (s, 2H) ; 6,82 (d, 1H, *J* = 8,4 Hz), 7,05-7,10 (m, 2H). Spectroscopie de masse (ESI) [M+Na]⁺ = 438.

### 1.32. Composé de formule (I-34)

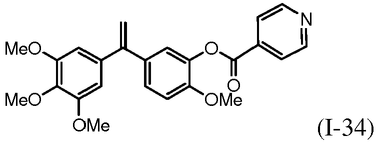

A une solution du composé (I-1) (0,316 mmol) dans 5 mL de CH₂Cl₂ sont ajoutés 0,47 mmol de d'EDCI, 0,47 mmol DMAP et 0,47 mmol d'acide isonicotinique. Après une nuit d'agitation à température ambiante, le mélange réactionnel est hydrolysé avec une solution aqueuse saturée de NaHCO₃ (3 mL), et extrait avec de l'acétate d'éthyle (3 x 3 mL). Les phases organiques sont rassemblées, séchées sur sulfate de sodium, filtrées et le solvant est évaporé et le résidu est chromatographié sur gel de silice.
Rendement 85%.
¹H NMR CDCl₃, 300 MHz δppm: 3,74 (s, 6H), 3,76 (s, 3H), 3,79 (s, 3H), 5,28 (d, 1H, *J* = 0,9 Hz), 5,34 (d, 1H, *J* = 1,2 Hz), 6,49 (s, 2H), 6,91 (d, 1H, *J* = 8,4 Hz), 7,10 (d, 1H, *J* = 2,1 Hz), 7,22 (dd, 1H, *J* = 8,4 Hz , *J* = 2,1 Hz), 7,92 (dd, 1H, *J* = 4,8 Hz , *J* = 1,8 Hz), 8,76 (d, 1H, *J* = 4,8 Hz). Spectroscopie de masse (ESI) [M+Na]⁺ = 444.

### 1.33. Composé de formule (I-35)

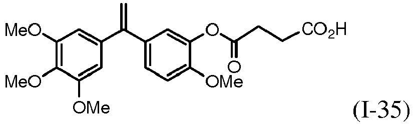

A une solution du composé (I-1) (0,316 mmol) dans 5 mL de pyridine sont ajoutés 0,47 mmol d'anhydride succinique et 0,06 mmol de DMAP. Après une nuit d'agitation, le mélange réactionnel est hydrolysé avec 5 mL d'une solution aqueuse saturée de NaHCO₃, et extrait avec de l'acétate d'éthyle (3 x 3 mL). Les phases organiques sont rassemblées, séchées sur sulfate de sodium, filtrées et le solvant est évaporé et le résidu est chromatographié sur gel de silice.
Rendement 55%.
¹H NMR CDCl₃, 300 MHz δppm: 2,73 (td, 2H, *J* = 6,3 Hz, *J* = 1,5 Hz), 2,84 (td, 2H, *J* = 6,9 Hz , *J* = 1,5 Hz), 3,74 (s, 6H), 3,76 (s, 3H), 3,80 (s, 3H), 5,27 (d, 1H, *J* = 0,9 Hz), 5,32 (d, 1H, *J* = 0,9 Hz), 6,48 (s, 2H), 6,85 (d, 1H, *J* = 8,7 Hz), 6,98 (d, 1H, *J* = 2,1 Hz), 7,15 (dd, 1H, *J* = 8,7 Hz , *J* = 2,1 Hz). Spectroscopie de masse (ESI) [M+Na]⁺ = 439.

### 1.34. Composé de formule (I-36)

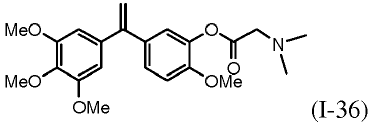

A une solution du composé (I-1) (0,316 mmol) dans 5 mL de CH₂Cl₂ sont ajoutés 0,47 mmol de l'EDCI, 0,47 mmol DMAP et 0,47 mmol de N,N-diméthylglycine. Après une nuit d'agitation à température ambiante, le mélange réactionnel est hydrolysé avec 6 mL d'une solution aqueuse saturée de NaHCO₃, et extrait avec de l'acétate d'éthyle (3x 3 mL). Les phases organiques sont rassemblées, séchées sur sulfate de sodium, filtrées et le solvant est évaporé et le résidu est chromatographié sur gel de silice.
Rendement 65%.
¹H NMR CDCl₃, 300 MHz δppm: 2,37 (s, 6H) ; 3,37 (s, 2H); 3,74 (s, 6H), 3,77 (s, 3H), 3,80 (s, 3H), 5,26 (s, 1H), 5,31 (s, 1H), 6,47 (s, 2H), 6,86 (d, 1H, *J* = 8,7 Hz), 6,97 (d, 1H, *J* = 2,1 Hz), 7,16 (dd, 1H, *J* = 8,4 Hz , *J* = 2,1 Hz). Spectroscopie de masse (ESI) [M+Na]⁺ = 424.

### 1.35. Composé de formule (I-37)

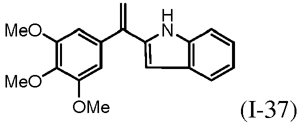

A une solution d'indole (165 mg, 1,41 mmol) dans 5 ml de THF anhydre, sont ajoutés successivement 1,83 mmol de triméthoxyacétophénone et 0,14 mmol de TiCl₄. Le mélange est agité sous azote à température ambiante pendant 2 heures. On additionne 100 mL d'eau dans le milieu réactionnel et il se forme une suspension blanche qui est filtrée sur fritté pour livrer 150 mg de poudre blanche. Le filtrat est extrait avec 3 x 30 mL de dichlorométhane. La phase aqueuse est alors alcalinisée avec une solution saturée de carbonate de sodium jusqu'à pH = 10 et est extraite à nouveau avec 3 x 30 mL de dichlorométhane. La phase organique est lavée par une solution saturée de carbonate de sodium, puis est séchée sur sulfate de sodium, filtrée et concentrée sous pression réduite pour fournir 415 mg de produit brut qui est dissout dans 5 mL de dichlorométhane et 0,68 mmol d'APTS sont addtionnés. Le mélange est agité sous azote à température ambiante pendant 30 minutes. 100 mL d'une solution saturée de carbonate de sodium sont ajoutés, et la solution est extraite avec 3 x 30 mL de dichlorométhane. La phase organique est séchée sur sulfate de sodium, filtrée et concentrée sous pression réduite pour fournir 110 mg de produit brut qui est purifié sur colonne de silice.
Rendement = 70%.
RMN ¹H 300MHz; CDCl₃, δ (ppm): 1,45 (m, 1H), 1,49 (m, 1H), 1,81 (m, 1H), 1,96 (s, 1H), 2,22 (m, 1H), 2,42 (td, 1H, *J* = 11,0 Hz, *J* = 4,0 Hz), 2,67 (m, 1H), 2,82 (m, 1H), 3,01 (dd, 1H, *J* = 16 Hz, *J* = 9,0 Hz), 3,23 (m, 2H), 4,36 (q, 1H, *J* = 9,0 Hz), 7,02 (t, 1H, *J* = 8,0 Hz), 7,21 (m, 2H), 7,58 (d, 1H, *J* = 8,0 Hz). Spectroscopie de masse (ESI) [M+Na]⁺: 332.

### 1.36. Composé de formule (I-38)

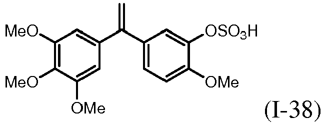

A une solution du composé (I-1) (0,316 mmol) dans 1 mL de pyridine anhydre est ajoutée 0,47 mmol du complexe SO₃/Pyridine. Après 24 h d'agitation à 20°C, le mélange réactionnel est hydrolysé avec 0,5 mL d'eau distillée et le solvant évaporé. Et le résidu est purifié par chromatographie sur gel de silice.
Rendement 80%.
¹H NMR CDCl₃, 300 MHz δppm: 3,59 (s, 3H), 3,71 (s, 6H), 3,82 (s, 3H), 5,22 (s, 1H), 5,30 (s, 1H), 6,47 (s, 2H), 6,66 (d, 1H, *J* = 8,7 Hz), 6,95 (dd, 1H, *J* = 8,7 Hz , *J* = 1,8 Hz), 7,54 (d, 1H, *J* = 1,8 Hz). Spectroscopie de masse (ESI négative) [M-H]⁺ = 395.

### 1.37. Composé de formule (I-39)

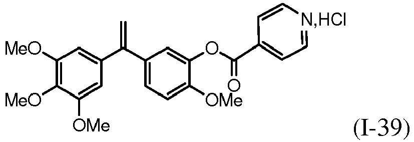

A une solution de 0,2 mmol du composé (I-34) dans 1 mL de méthanol anhydre est ajouté 1 mL d'une solution saturée de HCl/MeOH. Après 12 h d'agitation à température ambiante, le solvant est évaporé et le brut est repris dans l'éther, filtré sur verre fritté et lavé à l'éther. Le composé (I-39) est obtenu sous forme de poudre jaune.
Rendement 89%
Analyses élémentaires: (MM = 453,13) Calculé C: 63,00, H: 5,25, N: 3,06; Trouvé C: 62,87, H: 5,12, N: 2,91.

### 1.38. Composé de formule (I-40)

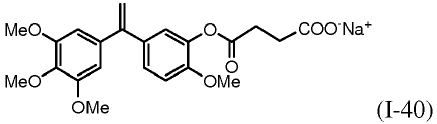

A une solution de 0,1 mmol du composé (I-35) dans 1 mL de méthanol, est ajoutée 0,1 mmol de soude. Le mélange est agité pendant 30 minutes. Le solvant est ensuite évaporé et le brut est repris dans l'éther, filtré sur verre fritté et lavé à l'éther. Le composé attendu est obtenu sous forme de poudre blanche.
Rendement 83%
Analyses élémentaires: (MM = 438,13) Calculé C: 60,27, H: 5,29; Trouvé C: 60,20, H: 5,23.

### 1.39. Composé de formule (I-41)

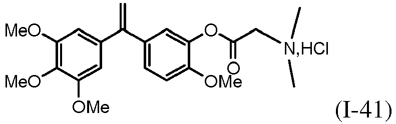

A une solution de 0,2 mmol du composé (I-36) dans 1 mL de méthanol anhydre est ajouté 1 mL d'une solution saturée de HCl/MeOH. Après 12 h d'agitation à température ambiante, le solvant est évaporé et le brut est repris dans l'éther, filtré sur verre fritté et lavé à l'éther. Le composé (I-41) est obtenu sous forme de poudre blanche.
Rendement 68%
Analyses élémentaires: (MM = 437,16) Calculé C: 60,34, H: 6,44, N: 3,20; Trouvé C: 60,21, H: 6,34, N: 3,11.

### 1.40. Composé de formule (I-42)

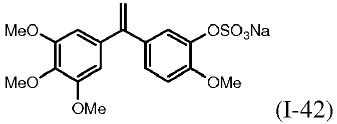

A une solution de 0,05 mmol du composé (I-38) dans 1 mL de méthanol anhydre, est ajoutée à 0° C, 0,1 mmol de méthanoate de sodium. Le mélange est agité sous azote à température ambiante pendant 30 minutes. Le solvant est ensuite évaporé et le brut est repris dans l'éther, filtré sur verre fritté et lavé à l'éther. Le composé attendu est obtenu sous forme de poudre blanche.
Rendement 69%
Analyses élémentaires: (MM = 418,07) Calculé C: 51,67, H: 4,58; Trouvé C: 51,60, H: 4,52.

### 1.41. Composé de formule (I-43)

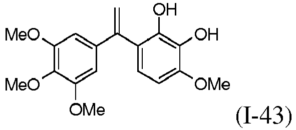

A -78°C, on additionne 1 mmol de *t*BuLi (2 eq) à une solution contenant 1,5 mmol de 5-iodo-1,2,3-triméthoxybenzène dissout dans 2 mL de toluène distillé. Après 45 minutes d'agitation à cette température on ajoute 1 mmol de 1-(2,3-dihydroxy-4-méthoxyphenyl)éthanone diluée dans 5 mL de toluène distillé. Ce mélange est agité 2 heures en laissant progressivement remonter la température puis est lentement hydrolysé par une solution de NH₄Cl saturée jusqu'à pH = 7-8. Après extraction à l'acétate d'éthyle (3 x 20 mL), les phases organiques réunies sont séchées sur Na₂SO₄ et concentrées à l'évaporateur rotatif. Le brut réactionnel est repris dans 2 mL de CH₂Cl₂ auxquel on additionne une pointe de spatule d'acide *para*-toluènesulfonique (APTS) puis est agité 3 heures à température ambiante. La solution est lavée par une solution saturée de NaCl et extraite au CH₂Cl₂. Après séchage sur Na₂SO₄ et concentration à l'évaporateur rotatif, on recueille une huile qui est purifiée par chromatographie.
Rendement 32%.
¹H RMN: δ ppm, CD₃Cl₃, 300 MHz: 3,80 (s, 3H), 3,85 (s, 3H), 3,92 (s, 3H), 5,33 (s, 1H), 5,37 (d, 1H, *J* = 1,2 Hz), 5,42 (s, 1H), 5,66 (d, 1H, *J* = 1,2 Hz), 6,50 (d, 1H, *J* = 8,7 Hz), 6,57 (s, 2H), 6,71 (d, 1H, *J* = 8,7 Hz). Spectroscopie de masse (ESI) (M+Na) = 355

### 1.42. Composé de formule (I-44)

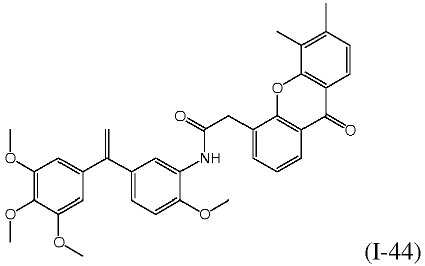

A une solution de DMXAA (220 mg, 0,78 mmoles), de pyridine (65 mg, 0,811 mmol) et de (Boc)₂O (255 mg, 1,17 mmoles) dans le diméthylformamide (DMF) (1,5 mL), est ajouté le composé (I-17) (205 mg, 0,65 mmoles). Après 24 h d'agitation à température ambiante, le gel obtenu est dilué dans 25 mL d'acétate d'éthyle. La phase organique est lavée par une solution saturée de NaHCO₃ puis par une solution saturée de KHSO₄ 1 M. La phase organique est ensuite séchée sur MgSO₄ et concentrée et le résidu obtenu est purifié sur colonne de gel de silice. Rendement 10%.
¹H RMN: δ ppm, CD₃Cl₃, 300 MHz: 2,50 (s, 3H), 2,51 (s, 3H), 3,55 (s, 3H), 3,82 (s, 6H), 3,90 (s, 3H), 4,17 (s, 2H), 5,34 (d, 1H, *J* = 13,2 Hz), 5,42 (d, 1H, *J* = 13,2 Hz), 6,57 (s, 2H), 6,70 (d, 1H, *J* = 8,4 Hz), 6,95 (m, 1H), 7,25 (d, 1H, *J* = 8,2 Hz), 7,45 (t, 1H, *J* = 8,2 Hz), 7,45 (d, 1H, *J* = 8,0 Hz), 8,06 (s, 1H), 8,17 (d, 1H, *J* = 8,1 Hz), 8,38 (d, 1H, *J* = 7,9 Hz), 8,48 (m, 1H).
Analyses élémentaires: (MM = 579,23) Calculé C: 72,52, H: 5,74, N: 2,42; Trouvé C: 72,17, H: 5,55, N: 2,25.

### 1.43. Composé de formule (I-45)

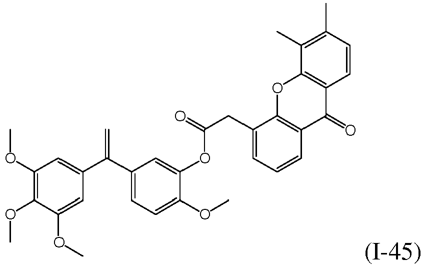

Sous atmosphère inerte, a une solution de DMXAA (214 mg, 0,76 mmol, 1,2 éq.) dans 3 mL de DMF distillé et 0,06 mL de pyridine distillée, est additionné (Boc)₂O (207 mg, 0,95 mmol, 1,5 éq.). Après quelques minutes sous agitation, le dérivé (I-1) (200 mg, 0,63 mmol, 1 eq) est ajouté au milieu réactionnel. Après 24 heures d'agitation sous atmosphère inerte, le milieu est repris par 30 mL d'acétate d'éthyle. La phase organique est lavée par une solution de NaHCO₃ saturée (3x 20 mL), puis par une solution de NH₄Cl saturée (3x 20 mL), et par une solution saturée de NaCl (3x 20 mL), séchée sur sulfate de sodium, filtrée sur verre fritté, puis concentrée sous vide. Le résidu est ensuite purifié par chromatographie sur colonne de gel de silice
Rendement 34 %
¹H RMN: δ ppm, CD₃Cl₃, 300 MHz: 3,62 (s, 3H); 3,78 (s, 6H); 3,86 (s, 3H); 4,23 (s, 2H); 5,32 (d, 2H, *J* = 3,8 Hz); 6,52 (s, 2H); 6,86 (d, 1H, *J* = 8,5 Hz); 7,01 (d, 1H, *J* = 2,2 Hz); 7,19 (m, 2H); 7,35 (t, 1H, *J* = 7,6 Hz); 7,75 (d, 1H, *J* = 7,6 Hz); 8,08 (d, 1H, *J* = 8,0Hz); 8,29 (d, 1H, *J* = 8,0 Hz). Spectroscopie de masse (ESI) (M+Na) = 603,6.

### 1.44. Composé de formule (I-46)

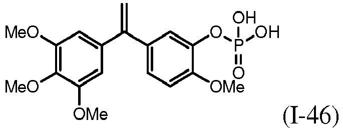

A une solution du composé diéthoxyphosphate (I-28) (0,126 mmol) dans 1 mL de CH₂Cl₂ sec est ajouté goutte à goutte à 0°C 0,252 mL d'iodure de triméthylsilane. Après une heure d'agitation à température ambiante, le mélange réactionnel est hydrolysé et extrait à l'acétate d'éthyle (3 x 3 mL). Les phases organiques sont rassemblées, lavées avec une solution aqueuse saturée de thiosulfate de sodium, séchées sur sulfate de sodium, filtrées et le solvant est concentré pour donner un résidu qui est purifié sur gel de silice.
Rendement 69%
Analyse élémentaire: (MM = 396,33) Calculé C: 54,55, H: 5,34; Trouvé C: 54,12, H: 5,17. Spectroscopie de masse (APCI négative) (M-H)⁺ = 395

### Exemple 2 : Étude biologique in vitro des composés de l'invention

Les effets sur la prolifération de différentes cellules cancéreuses ainsi que sur la prolifération de cellules endothéliales ont été étudiés.

L'activité biologique des composés de l'invention a été étudiée *in vitro* sur 7 lignées cellulaires cancéreuses humaines d'origines tissulaires différentes (*HCT116: carcinome colorectal; K562: leucémie myéloïde chronique; B16-F10: mélanome; U87: glioblastome; A549: cancer du poumon et MDA-MB 231 et MDA-MB 435: cancer du sein).* Les cellules sélectionnées pour cette étude ont été incubées à 37°C en présence de l'un des composés ajouté dans le milieu de culture à différentes concentrations. L'ensemble des expériences réalisées a permis de déterminer le degré de toxicité du composé testé, son effet sur le déroulement du cycle cellulaire ainsi que sa capacité à induire une mort cellulaire par apoptose.

### 2.1. Etude de la cytotoxicité

Les lignées cellulaires cancéreuses proviennent de l'American Type Culture Collection (Rockville, MD, USA) et ont été cultivées selon les recommandations du fournisseur.

Les cellules A549, U87, MDA-MB231, MDA-MB435 et B16F10 ont été cultivées dans du milieu de culture "Dulbecco minimal essential médium" (DMEM) contenant 4,5 g/L de glucose et supplémenté avec 10% de sérum de veau foetal et 1% de glutamine. Les cellules K562 et HCT116 ont été cultivées dans du milieu RPMI 1640 contenant 10% de sérum de veau foetal et 1% de glutamine. Toutes les lignées cellulaires ont été maintenues en culture à 37°C dans une atmosphère humide contenant 5% de CO₂. La viabilité cellulaire a été évaluée en utilisant le réactif "CellTiter-Blue TM" (Promega, WI, USA) en respectant les instructions du fabriquant. Les cellules ont été ensemencées dans des plaques de culture de 96 puits à raison de 5000 cellules par puits dans 50 µl de milieu de culture. Après 24 heures de culture, les composés de formule générale (I) dissous dans du DMSO ont été ajoutés individuellement dans chacun des puits à raison de 50 µl par puits. Tous les composés ont été testés en triplicat pour chaque concentration définie et chaque expérience a été répétée 3 fois. Après 72 heures d'incubation, 20 µL de resazurin ont été ajoutés dans chaque puits. Après 2 heures d'incubation, la fluorescence émise a été mesurée à 590 nm après excitation à 560 nm à l'aide d'un lecteur de fluorescence de type Victor (Perkin-Elmer, USA).

La concentration de chacun des composés qui induit la mort de 50% des cellules (CI₅₀) a été déterminée après 72 heures d'incubation. Certains composés conformes à l'invention présentent une CI₅₀ de l'ordre du nanomolaire, donc équivalente à celle de CA-4 prise comme référence. Les résultats obtenus sont présentés dans le tableau 1 suivant.

**Tableau 1**

| **Molécules de l'invention** | **CI₅₀ pour différentes lignées cellulaires (nM)** | | | | | | |
|---|---|---|---|---|---|---|---|
| | HCT116 | K562 | B16F10 | U87 | A549 | M435 | M231 |
| (I-1) | 2-4 | 5 | 2 | 8 | 8 | 4,5 | 4 |
| (I-2) | 42 | - | - | - | 28 | 25 | 40 |
| (I-6) | 8 | - | - | 18 | 25 | 11 | 9 |
| (I-7) | 8 | - | - | 15 | 28 | 25 | 50 |
| (I-8) | 25 | - | - | 35 | 42 | 40 | 50 |
| (I-17) | - | - | - | - | 8 | - | 6 |
| (I-22) | 7 | - | - | - | - | - | - |
| (I-32) | 8 | - | - | - | - | - | - |
| (I-34) | 3 | - | - | - | - | - | - |
| (I-35) | 5 | - | - | - | - | - | - |
| (I-36) | 3 | - | - | - | - | - | - |
| (I-45) | 7 | - | - | - | - | - | - |

| | | | | | | | |
|---|---|---|---|---|---|---|---|
| - signifie qu'aucune mesure n'a été effectuée. | | | | | | | |

### 2.2. Étude du cycle cellulaire

Les cellules K562 et MDA-MB231 sont ensemencées dans des plaques de culture 6 puits à raison de 300 000 cellules par puits dans leurs milieux respectifs décrits ci-dessus. Après 24 heures de culture, le composé (I-1) a été ajouté dans chacun des puits à différentes concentrations. Après 24 heures d'incubation, les cellules sont collectées individuellement dans des tubes de 15 mL puis centrifugées. Les cellules sont ensuite lavées 2 fois dans du PBS froid puis remises en suspension dans 1 ml de PBS, fixées en ajoutant 2 mL d'éthanol absolu froid et placées à 4°C pendant 1h. Après centrifugation, les cellules sont lavées 2 fois dans du PBS puis le culot cellulaire est repris dans 100 µL de Triton X100 à 1%. Après 30 minutes d'incubation à température ambiante, 50 µL de RNase A préalablement bouillie (1 mg/mL) et 500 µL d'iodure de propidium (50 µg/mL) sont ajoutés dans chaque tube et incubés dans l'obscurité à température ambiante pendant 30 minutes. La distribution du nombre de cellules dans chacune des phases du cycle cellulaire est ensuite déterminée par cytométrie en flux à l'aide d'un cytomètre de type FC500 (Beckman-Coulter, France).

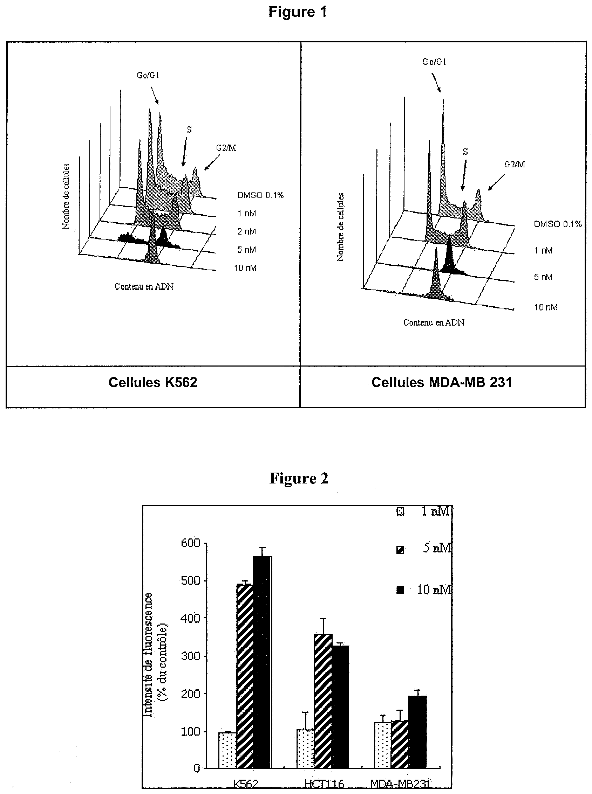

L'analyse par cytométrie en flux des cellules traitées (K562 et MDA-MB 231) avec le composé (I-1) a montré que ce dernier bloque la division cellulaire en phase G2/M. Cet effet est significatif après 24 heures d'exposition des cellules au composé (I-1) utilisé à la concentration de 5 nM (Figure 1).

### 2.3. Étude de l'apoptose

Afin de préciser si le composé (I-1) entraîne une mort cellulaire par apoptose, l'activité enzymatique intracellulaire des caspases 3 et 7 a été évaluée dans les cultures des cellules *K562*, *HCT116* et *MDA-MB 231* exposées pendant 24 heures à l'action du composé (I-1).

L'apoptose est mesurée en utilisant le kit "Apo-one homogeneous caspase-3/7 assay" (Promega Co, WI, USA) en suivant les recommandations du fabriquant. En bref, les cellules sont ensemencées dans des plaques de culture de 96 puits à raison de 50 000 cellules par puits dans 100 µL de milieu de culture. Après 24h d'incubation, le milieu est remplacé par 100 µL de milieu de culture contenant différentes concentrations du composé (I-1) ou 0,1% de DMSO (contrôle négatif). Après 24 heures de traitement, on ajoute dans chaque puits 100 µL de réactif contenant le substrat des caspases et le tampon de la réaction. Après 1 heure d'incubation, la fluorescence émise par les cellules est mesurée à 527 nm à l'aide d'un lecteur de microplaque de type Victor (Perkin-Elmer, USA).

Les résultats présentés à la Figure 2 montrent que l'incubation des différentes cellules avec le composé (I-1) conduit à une forte induction de l'apoptose.

### Exemple 3 : Étude de l'inhibition de la polymérisation de la tubuline

Des tests concernant l'inhibition de la polymérisation de la tubuline ont été effectués sur les composés qui présentaient les meilleures activités cytotoxiques. Ces tests ont été effectués sur une tubuline purifiée par la méthode Shelanski (Shelanski, M. C.; Gaskin, F.; Cantor, C. R. Proc. Natl. Acad. Sci. USA, 1973, 70, 765-768) à partir de cerveaux de porcs, où elle constitue 20 à 25% des protéines solubles. La méthode de purification est basée sur des cycles d'assemblage-désassemblage température dépendants. La polymérisation de la tubuline a été suivie par turbidimétrie suivant la méthode de Gaskin (Gaskin, F.; Cantor, C. R.; Shelanski, M. L. J. Bio. Mol., 1974, 89, 737) à une longueur d'onde de 350 nm. Les différents échantillons ont été dissous dans le DMSO et incubés 10 minutes à 37°C puis 5 minutes à 0°C.

Le composé CA-4 et le DMSO ont été pris comme référence.

Les tests ont montré, pour ces composés, une activité inhibitrice de la polymérisation de la tubuline similaire à celle du composé CA-4 de référence (de l'ordre du micromolaire à quelques dizaines de micromolaires seulement). Les résultats obtenus sont présentés dans le tableau 3 suivant.

**Tableau 3**

| **Molécules de l'invention** | **Inhibition de la tubuline (CI₅₀ en µM)** |
|---|---|
| (I-1) | 2,2 |
| (I-2) | 2,0-3,3 |
| (I-17) | 1,8 |
| (I-22) | 4,1 |

### Exemple 4 : Étude de l'activité anti-vasculaire

### 4.1. Étude in vitro de la cytotoxicité sur les cellules endothéliales humaines.

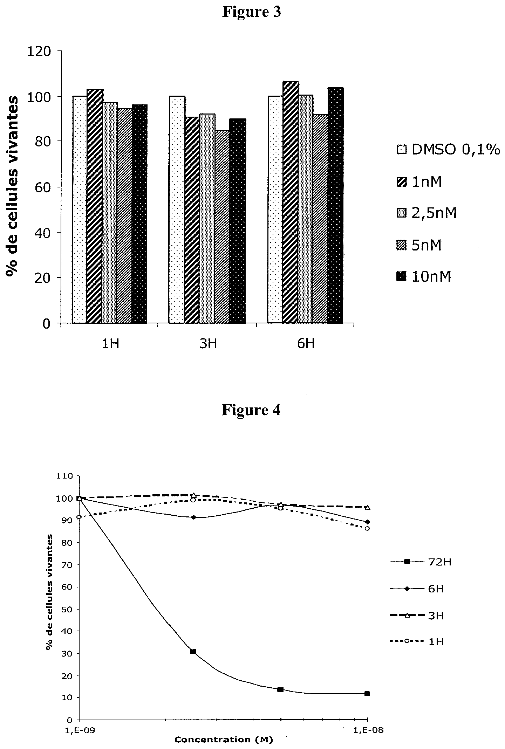

La cytotoxicité du composé (I-1) vis-à-vis des cellules endothéliales humaines (EAhy926) a été évaluée après 1 heure, 3 heures et 6 heures de traitement. Le nombre de cellules vivantes a été compté soit immédiatement à la fin du traitement (Figure 3), soit 72 heures après l'arrêt du traitement (Figure 4). On observe que lorsque les cellules endothéliales sont traitées pendant 72 heures avec le composé (I-1), la CI₅₀ est de 2 nM. En revanche, après 1 heure, 3 heures ou 6 heures de traitement, le composé (I-1) ne présente pas d'activité cytotoxique même à la dose de 10 nM.

### 4.2. Étude in vitro sur la formation de tubes vasculaires sur Matrigel®

Pour savoir si le composé (I-1) perturbe l'organisation spatiale des cellules endothéliales en structures similaires à des capillaires vasculaires, des cellules endothéliales humaines (EAhy926) ont été traitées immédiatement après la mise en culture sur Matrigel™ ou après 24 heures de culture, afin de leur permettre de former des tubes vasculaires.

Les cellules EAhy926 (cellules endothéliales macro-vasculaires HUVEC immortalisées) ont été cultivées dans du milieu de culture "Dulbecco minimal essential médium" (DMEM) contenant 4,5 g/L de glucose et supplémenté avec 10% de sérum de veau foetal, 1% de glutamine et complément HAT (100 µM de hypoxanthine, 0,4 µM d'aminoptérine et 16 µM de thymidine, Invitrogen; Cergy-Pontoise, France). Les cellules ont été maintenues en culture à 37°C dans une atmosphère humide contenant 5% de CO₂.

Les cellules ont été ensemencées dans des plaques de culture de 96 puits à raison de 3000 cellules par puits dans 50 µL de milieu de culture. Après 24 heures d'incubation, le composé (I-1) a été ajouté à différentes concentrations pendant 1 heure, 3 heures, 6 heures ou 72 heures. A la fin du traitement le nombre de cellules a été évalué en utilisant le réactif "CellTiter-Blue TM" (Promega, WI, USA) comme décrit précédemment. En parallèle, après 1 heure, 3 heures ou 6 heures de traitement avec le composé (I-1), le milieu de culture a été retiré et remplacé par du milieu frais pendant 72 heures et le nombre de cellules vivantes a ensuite été mesuré en utilisant le réactif "CellTiter-Blue TM".

Pour évaluer l'activité anti-vasculaire du composé (I-1), les cellules EAhy926 ont été mise en culture dans des plaques de culture de 96 puits préalablement recouvertes avec un extrait de matrice extracellulaire (Matrigel™, BD Biosciences, Le Pont-de-Claix, France) dans lequel elles forment spontanément des tubes capillaires.

Tout d'abord, nous avons mesuré la capacité du composé (I-1) à inhiber la formation du réseau capillaire. Le Matrigel™ est déposé dans des plaques de culture de 96 puits à raison de 70µL/puits et laissé à incuber à 37°C pendant 45 minutes pour permettre sa polymérisation. 15 000 cellules en suspension dans 150 µL de milieu de culture sont ensemencées par puits dans chacun des puits contenant le Matrigel™ en absence ou en présence de différentes concentrations du composé (I-1), à raison de 3 puits par concentration. Après 1 heure, 3 heures et 6 heures d'incubation à 37°C, les cellules sont observées et photographiées à l'aide d'un microscope optique de type TE2000 (Nikon, France), équipé d'une caméra.

En parallèle, 15 000 cellules EAhy926 en suspension dans 150 µL de milieu de culture ont été ensemencées dans chacun des puits contenant le Matrigel™. Après 18 heures d'incubation, quand le réseau capillaire est bien formé, le composé (I-1) a été ajouté à différentes concentrations. L'effet du produit a été observé après 1 heure, 3 heures et 6 heures d'incubation à l'aide d'un microscope optique.

On peut observer qu'après un traitement de 3 heures à une dose de 10 nM (non toxique), le composé (I-1) induit une diminution très importante du nombre de tubes vasculaires. Ces résultats indiquent que le composé (I-1) possède également une activité anti-vasculaire potentiellement utile en thérapeutique.

### Exemple 5 : Étude de l'activité anti-vasculaire de la molécule (1-1) in vivo

L'effet antivasculaire de la molécule (I-1) a été testé sur des souris porteuses de tumeurs humaines en comparaison au contrôle positif DMXAA, selon le protocole décrit par Beauregard et al. (NMR Biomed., 2002, 15 :99-105).

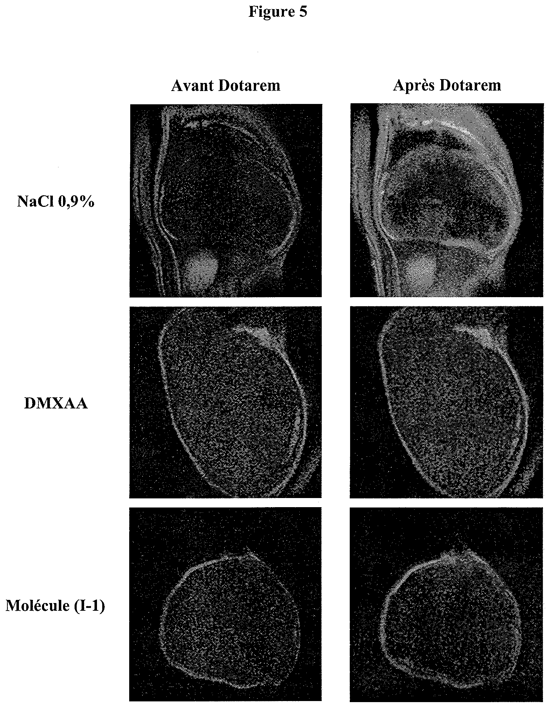

Ainsi, des souris femelles CD-1 *Nude* ont été xénogreffées par injection sous-cutanée de cellules tumorales de colon humaines LS174T. Les souris porteuses de tumeurs ont alors été traitées par du NaCl 0,9% (véhicule, 10 ml/kg), du DMXAA (27,5 mg/kg) par voie intrapéritonéale (IP) ou la molécule (I-1) (45 mg/kg) par voie intraveineuse (IV). Trois heures après administration, les souris ont été traitées par l'agent de contraste Dotarem® par voie intraveineuse (IV). La pénétration de l'agent de contraste dans les tumeurs a été suivie en temps réel par Imagerie par Résonance Magnétique (IRM) et s'est traduit par un réhaussement de l'intensité du signal dans les tumeurs. L'analyse densitométrique des images acquises a permis de déterminer le réhaussement maximum (Rmax) ainsi que la pente P0 correspondant à la vitesse d'entrée du Dotarem® dans les tumeurs. Ces résultats sont présentés dans le tableau 2 suivant. Des images caractéristiques des effets observées sont présentées également sur la figure 5.

**Tableau 2**

| **Traitement (dose, voie d'administration)** | **Moyenne Rmax (% du contrôle NaCl)** | **Moyenne P0 (unité arbitraire)** | **Nombre de souris analysées par groupe** |
|---|---|---|---|
| NaCl 0,9% (10 ml/kg, IP) | 100,00 ± 49,01 | 1,97 ± 0,96 | 5/8 |
| DMXAA (27,5 mg/kg, IP) | 26,58 ± 21,19 | 0,50 ± 0,40 | 4/8 |
| (I-1) (45 mg/kg, IV) | 67,56 ± 33,10 | 1,53 ± 0,82 | 6/8 |

Le Rmax moyen était de 100,00 ± 49,01% dans le groupe traité par le NaCl 0,9% (n=5). La pente P0 était de 1,97 ± 0,96 (unité arbitraire).

Ces deux paramètres étaient fortement diminués chez les animaux ayant reçu le DMXAA (Rmax de 26,58 ± 21,19% et P0 de 0,50 ± 0,40 ; n=4), ceci traduisant une diminution de l'entrée du Dotarem® dans les tumeurs et donc un effet du DMXAA sur la vascularisation des tumeurs.

La molécule (I-1) administrée à 45 mg/kg a produit un effet comparable en provoquant la baisse du Rmax moyen à 67,56 ± 33,10% et de la pente P0 à 1,53 ± 0,82 (n=6). Ainsi, la molécule (I-1) a un effet anti-vasculaire sur les tumeurs de colon humaines LS174T xénogreffées chez la souris *Nude.*

## Revendications

1. Composé de formule (I) suivante : 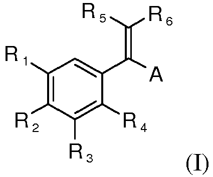 dans laquelle :
- R₁, R₂ et R₃ représentent chacun un groupe méthoxy,
- R₄ représente un atome d'hydrogène,
- R₅ et R₆ sont identiques et représentent chacun un atome d'hydrogène ou de fluor, et représentent avantageusement chacun un atome d'hydrogène et
- A est un cycle choisi dans le groupe comprenant les groupes aryles et hétéroaryles, lesdits groupes pouvant être :
▪ soit accolés à un hétérocycle comportant de 5 à 7 chaînons, comportant éventuellement une ou plusieurs insaturations et éventuellement substitué par un ou plusieurs groupements alkyles en C₁ à C₄,
▪ soit substitués par un ou plusieurs groupes choisis parmi les halogènes, les groupes -B(OH)₂, alkyles en C₁ à C₄, alcényles en C₂ à C₄, alcynyles en C₂ à C₄, aryle, hétéroaryle, -COOH, -NO₂, méthylènedioxy, -NR₇R₈, -NHCOR₇, -CONR₇R₈, -NHCOOR₉, -OSi(alkyle en C₁-C₄)₃, -NHSO₂R₉, alkoxy en C₁ à C₄ éventuellement substitué par un ou plusieurs atomes de fluor, -OCONR₇R₈, -OSO₂CF₃, -OSO₂R₉, -SO₂R₉, -OSO₃H, -OPO(OR₁₀)₂, -ONR₇R₈, -OR₁₁, -SO₂NR₁₂R₁₃, -SO₂NHCOR₁₄, -OCOR₁₅, -OCOOR₁₆, -SR₁₇, et -OCO(CH₂)ₙC₆H₄N[(CH₂)ₘCl]₂ avec n = 1 à 4 et m = 1 à 3,
dans lesquels :
◆ R₇ et R₈ représentent, indépendamment l'un de l'autre, un atome d'hydrogène ou un groupe alkyle en C₁ à C₄, aryle ou hétéroaryle, et avantageusement représentent un atome d'hydrogène ou un groupe alkyle en C₁ à C₄,
◆ R₉ représente un groupe alkyle en C₁ à C₄, aryle ou hétéroaryle, et avantageusement représente un groupe alkyle en C₁ à C₄,
◆ R₁₀ représente un atome d'hydrogène ou un groupe alkyle en C₁ à C₄ ou un groupe benzyle,
◆ R₁₁ représente un atome d'hydrogène, un groupe O-protecteur, un sucre choisi parmi le glucose, le mannose, l'arabinose ou le galactose, un aminosucre ou un acide aminé, les groupements OH et NH₂ libres des sucres, aminosucres et acides aminés pouvant être éventuellement substitués par un groupement O-protecteur et N-protecteur, respectivement,
◆ R₁₂ et R₁₃ représentent, indépendamment l'un de l'autre, un atome d'hydrogène, un groupe alkyle en C₁ à C₄, un groupe aryle ou hétéroaryle,
◆ -COR₁₄ représente le reste d'une molécule d'acide aminé liée au groupement -SO₂NH- ou R₁₄ représente un groupe alkyle en C₁ à C₄,
◆ R₁₅ représente un atome d'hydrogène, un groupe alkyle en C₁ à C₄, aryle, ou hétéroaryle, ou un groupe -(CH₂)ₘCO₂H ou -(CH₂)ₘNR₇R₈ avec m = 1 à 3,
◆ R₁₆ représente un groupe alkyle en C₁ à C₄, aryle, ou hétéroaryle, ou un groupe -(CH₂)ₘCO₂H ou -(CH₂)ₘNR₇R₈ avec m = 1 à 3, et
◆ R₁₇ représente un atome d'hydrogène ou un groupe alkyle en C₁ à C₄ ou aryle, ainsi que ses sels pharmaceutiquement acceptables,
à l'exclusion des composés de formules suivantes :

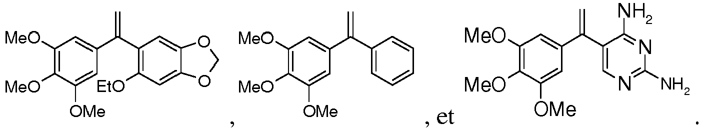

2. Composé selon l'une quelconque des revendications 1, **caractérisé en ce que** A est choisi dans le groupe comprenant les groupes phényle, naphtyle, quinolinyle et indolyle, et de préférence phényle, lesdits groupes pouvant être :
▪ soit accolés à un hétérocycle comportant de 5 à 7 chaînons, comportant éventuellement une ou plusieurs insaturations et éventuellement substitué par un ou plusieurs groupements alkyles en C₁ à C₄,
▪ soit substitués par un ou plusieurs groupes choisis parmi les halogènes, les groupes -B(OH)₂, alkyles en C₁ à C₄, alcényles en C₂ à C₄, alcynyles en C₂ à C₄, aryle, hétéroaryle, -COOH, -NO₂, méthylènedioxy, -NR₇R₈, -NHCOR₇, -CONR₇R₈, -NHCOOR₉, -OSi(alkyle en C₁-C₄)₃, -NHSO₂R₉, alkoxy en C₁ à C₄ éventuellement substitué par un ou plusieurs atomes de fluor, -OCONR₇R₈, -OSO₂CF₃, -OSO₂R₉, -SO₂R₉, -OSO₃H, -OPO(OR₁₀)₂, -ONR₇R₈, -OR₁₁, -SO₂NR₁₂R₁₃, -SO₂NHCOR₁₄, -OCOR₁₅, -OCOOR₁₆, -SR₁₇, -OCO(CH₂)ₙC₆H₄N[(CH₂)ₘCl]₂ avec n = 1 à 4 et m = 1 à 3,
R₇, R₈, R₉, R₁₀, R₁₁, R₁₂, R₁₃, R₁₄, R₁₅, R₁₆ et R₁₇ étant tels que définis à la revendication 1.

3. Composé selon l'une quelconque des revendications 1 et 2, **caractérisé en ce que** A est un cycle choisi dans le groupe comprenant les groupes aryles et hétéroaryles, ledit cycle étant avantageusement choisi parmi un groupe phényle, naphtyle, quinolinyle et indolyle, et de préférence étant un phényle, et ledit cycle pouvant être substitué par un ou plusieurs groupes choisis parmi -Me, -OH, -OMe,-OCF₃, -NH₂, -NO₂, -COOH, -B(OH)₂, -OSi*t*BuMe₂, -OCOMe, -OCO*t*Bu, méthylénedioxy, -OCONEt₂, -OCO(CH₂)₂COOH, -OCOCH₂NMe₂, -OSO₃H,-OSO₂CF₃, -OP(O)(OH)₂, -OP(O)(OEt)₂, -OPO(OCH₂Ph)₂, -Br, -F, -OCO(CH₂)₃C₆H₄N[(CH₂)₂Cl]₂, -OCO(C₅H₄N),

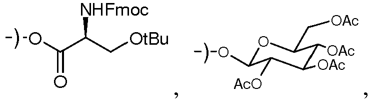

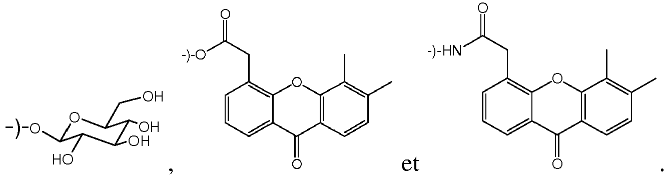

4. Composé selon l'une quelconque des revendications 1 à 4, **caractérisé en ce qu'**il répond à la formule (II) suivante : 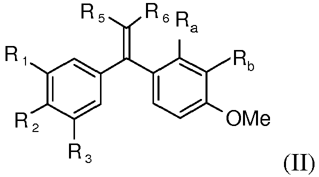 dans laquelle :
- R₁, R₂, R₃, R₅ et R₆ sont tels que définis à la revendication 1,
- Rₐ représente un atome d'hydrogène ou d'halogène, ou un groupe -B(OH)₂, alkyles en C₁ à C₄, alcényles en C₂ à C₄, alcynyles en C₂ à C₄, aryle, hétéroaryle, -COOH, -NO₂, méthylènedioxy, -NR₇R₈, -NHCOR₇, -CONR₇R₈, -NHCOOR₉, -OSi(alkyle en C₁-C₄)₃, -NHSO₂R₉, alkoxy en C₁ à C₄ éventuellement substitué par un ou plusieurs atomes de fluor, -OCONR₇R₈, -OSO₂CF₃, -OSO₂R₉, -SO₂R₉, -OSO₃H, -OPO(OR₁₀)₂, -ONR₇R₈, -OR₁₁, -SO₂NR₁₂R₁₃, -SO₂NHCOR₁₄, -OCOR₁₅, -OCOOR₁₆, -SR₁₇ ou -OCO(CH₂)ₙC₆H₄N[(CH₂)ₘCl]₂ avec n = 1 à 4 et m = 1 à 3, Rₐ représentant avantageusement un atome d'hydrogène, et
- R_{b} représente un atome d'halogène, et de préférence un atome de fluor, un groupe -OR₁₁, -OCOR₁₅, -OCOOR₁₅, -OCONR₇R₈, -OSO₂R₉, -OSO₂CF₃, -OSO₃H, -OPO(OR₁₀)₂, -NH₂, -NHCOR₇, -NHCOOR₉, ou -NHSO₂R₉,
R₇, R₈, R₉, R₁₀, R₁₁, R₁₂, R₁₃, R₁₄, R₁₅, R₁₆ et R₁₇ étant tels que définis à la revendication 1.

5. Composé selon l'une quelconque des revendications 1 à 4 **caractérisé en ce qu'**il est choisi parmi :

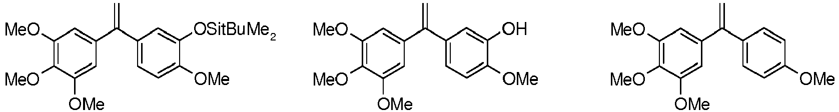

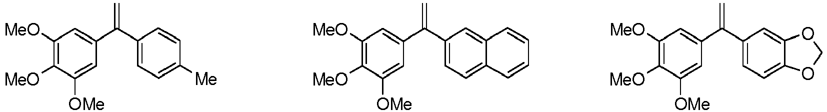

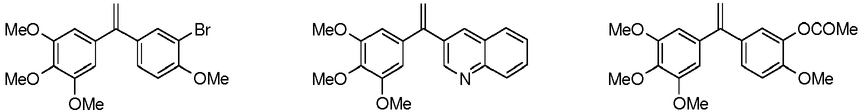

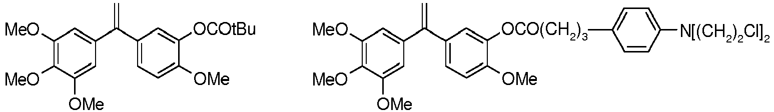

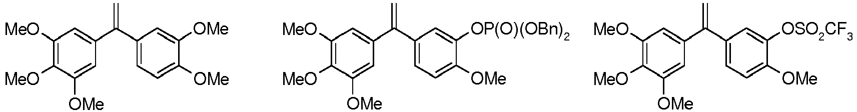

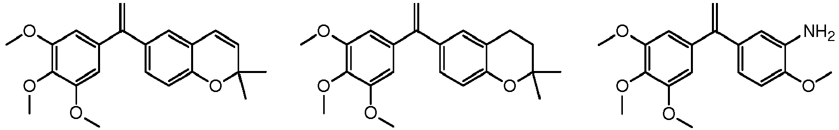

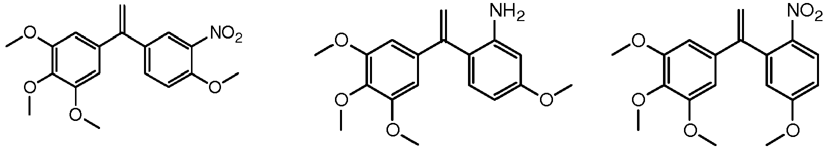

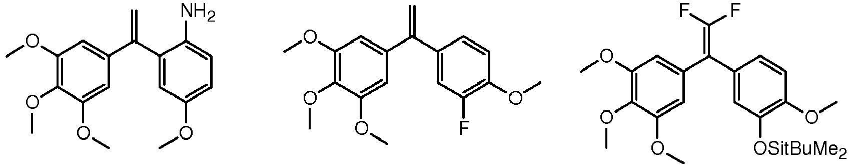

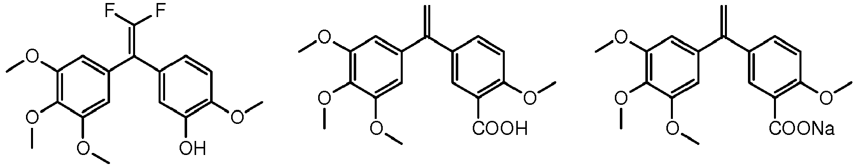

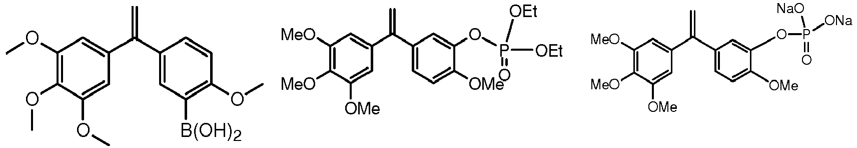

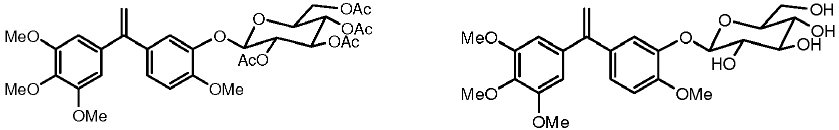

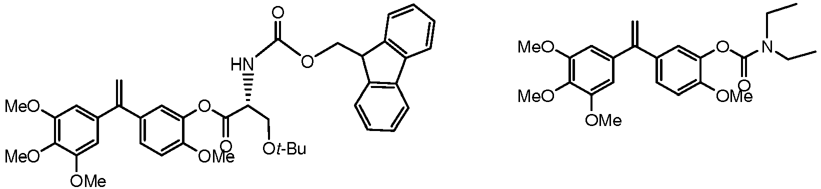

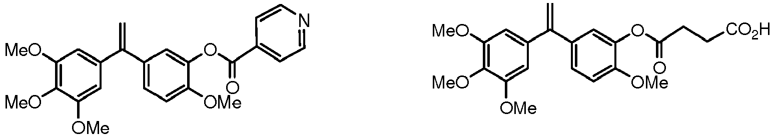

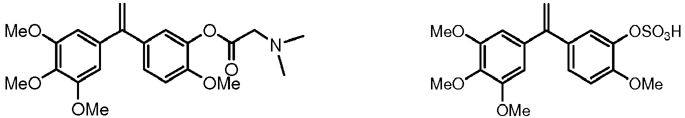

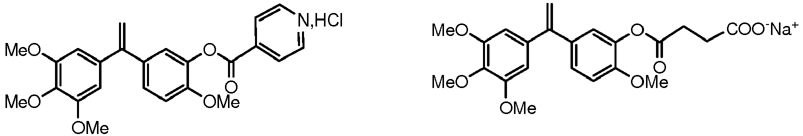

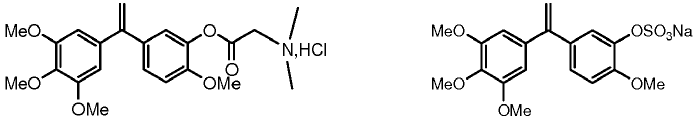

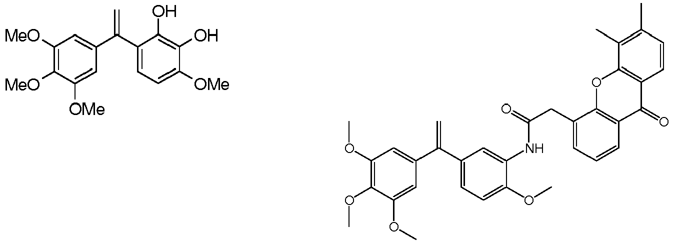

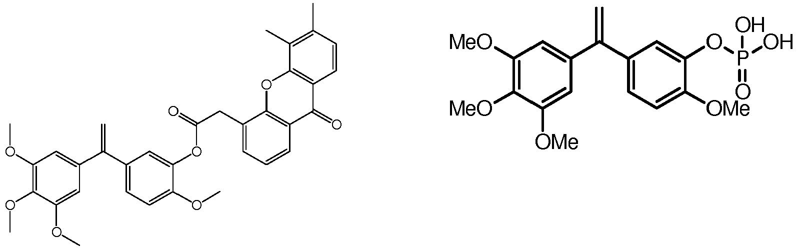

6. Procédé de préparation d'un composé de formule (I) tel que défini à la revendication 1, **caractérisé en ce qu'**il comprend les étapes successives suivantes :
- faire réagir un composé de formule (II) suivante : 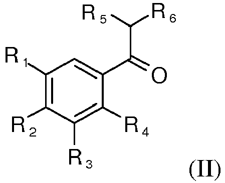 dans laquelle R₁, R₂, R₃, R₄, R₅ et R₆ sont tels que définis à la revendication 1,
avec un composé organométallique de formule A-M dans laquelle A est tel que défini à la revendication 1 et M représente un métal alcalin ou un métal alcalino-terreux substitué par un halogène, pour former un composé de formule (III) suivante : 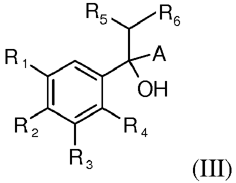
- faire réagir un composé de formule (III) avec un acide pour former un composé de formule (I).

7. Composé selon l'une quelconque des revendications 1 à 5 ou composé choisi parmi 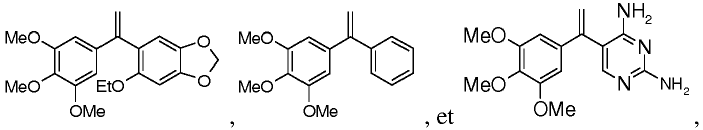 ainsi que leurs sels pharmaceutiquement acceptables et leurs prodrogues, en tant que médicament.

8. Composé selon la revendication 7, en tant que médicament inhibiteur de la polymérisation de la tubuline.

9. Composé selon l'une quelconque des revendications 7 et 8, en tant que médicament destiné à traiter ou prévenir les maladies prolifératives, telles que le cancer, le psoriasis ou la fibrose.

10. Composition pharmaceutique, **caractérisée en ce qu'**elle comprend au moins un composé selon l'une quelconque des revendications 1 à 5 ou un composé choisi parmi 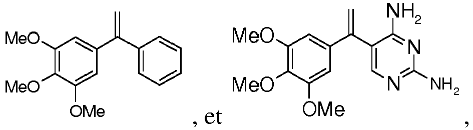 ainsi que leurs sels pharmaceutiquement acceptables, en association avec un ou plusieurs excipients pharmaceutiquement acceptables.

11. Composition pharmaceutique selon la revendication 10, **caractérisée en ce qu'**elle comprend au moins un autre principe actif.

12. Composition pharmaceutique selon la revendication 11, **caractérisée en ce que** le principe actif est choisi parmi la 6-mercaptopurine, la fludarabine, la cladribine, la pentostatine, la cytarabine, le 5-fluorouracile, la gemcitabine, le méthotrexate, le raltitrexed, l'irinotécan, le topotécan, l'étoposide, la daunorubicine, la doxorubicine, l'épirubicine, l'idarubicine, la pirarubicine, la mitoxantrone, la chlorméthine, la cyclophosphamide, l'ifosfamide, le melphalan, le chlorambucil, le busulfan, la carmustine, la fotémustine, la streptozocine, le carboplatine, le cisplatine, l'oxaliplatine, la procarbazine, la dacarbazine, la bléomycine, la vinblastine, la vincristine, la vindésine, la vinorelbine, le paclitaxel, le docétaxel, la L-asparaginase, la flutamide, la nilutamide, la bicalutamide, l'acétate de cyprotérone, la triptoréline, la leuproréline, la goséréline, la buséréline, le formestane, l'aminoglutéthimide, l'anastrazole, le létrozole, le tamoxifène, l'octréotide, le lanréotide, l'acide (Z)-3-[2,4-diméthyl-5-(2-oxo-1,2-dihydro-indol-3-ylidèneméthyl)-1H-pyrrol-3-yl]-propionique, l'acide 4-((9-chloro-7-(2,6-difluorophényl)-5H-pyrimidol(5,4-d)(2)benzazépin-2-yl)amino)benzoïque, l'acide 5,6-diméthylxanthénone-4-acétique et l'acide 3-(4-(1,2-diphénylbut-1-ényl)phényl)acrylique.

13. Composition pharmaceutique comprenant :
(i) au moins un composé selon l'une quelconque des revendications 1 à 5 ou un composé choisi parmi 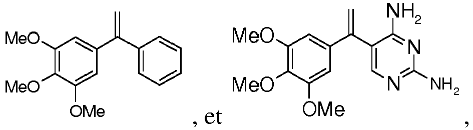
(ii) au moins un autre principe actif,
en tant que produits de combinaison pour une utilisation simultanée ou étalée dans le temps.

14. Composition selon la revendication 13, **caractérisée en ce que** le(s) principe(s) actif(s) est(sont) choisi(s) parmi la 6-mercaptopurine, la fludarabine, la cladribine, la pentostatine, la cytarabine, le 5-fluorouracile, la gemcitabine, le méthotrexate, le raltitrexed, l'irinotécan, le topotécan, l'étoposide, la daunorubicine, la doxorubicine, l'épirubicine, l'idarubicine, la pirarubicine, la mitoxantrone, la chlorméthine, la cyclophosphamide, l'ifosfamide, le melphalan, le chlorambucil, le busulfan, la carmustine, la fotémustine, la streptozocine, le carboplatine, le cisplatine, l'oxaliplatine, la procarbazine, la dacarbazine, la bléomycine, la vinblastine, la vincristine, la vindésine, la vinorelbine, le paclitaxel, le docétaxel, la L-asparaginase, la flutamide, la nilutamide, la bicalutamide, l'acétate de cyprotérone, la triptoréline, la leuproréline, la goséréline, la buséréline, le formestane, l'aminoglutéthimide, l'anastrazole, le létrozole, le tamoxifène, l'octréotide, le lanréotide, l'acide (Z)-3-[2,4-diméthyl-5-(2-oxo-1,2-dihydro-indol-3-ylidèneméthyl)-1H-pyrrol-3-yl]-propionique, l'acide 4-((9-chloro-7-(2,6-difluorophényl)-5H-pyrimidol(5,4-d)(2)benzazépin-2-yl)amino)benzoïque, l'acide 5,6-diméthylxanthénone-4-acétique et l'acide 3-(4-(1,2-diphénylbut-1-ényl)phényl)acrylique.

15. Composition selon l'une quelconque des revendications 13 et 14, en tant que médicament pour le traitement des maladies prolifératives, telles que le cancer, le psoriasis ou la fibrose.

16. Composition pharmaceutique pour son utilisation dans le traitement et la prévention des maladies prolifératives, comprenant un composé de formule : 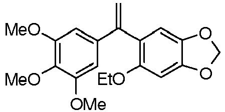 en association avec un ou plusieurs excipients pharmaceutiquement acceptables.

17. Composition pour son utilisation selon la revendication 16, **caractérisée en ce qu'**elle comprend au moins un autre principe actif, notamment choisi parmi la 6-mercaptopurine, la fludarabine, la cladribine, la pentostatine, la cytarabine, le 5-fluorouracile, la gemcitabine, le méthotrexate, le raltitrexed, l'irinotécan, le topotécan, l'étoposide, la daunorubicine, la doxorubicine, l'épirubicine, l'idarubicine, la pirarubicine, la mitoxantrone, la chlorméthine, la cyclophosphamide, l'ifosfamide, le melphalan, le chlorambucil, le busulfan, la carmustine, la fotémustine, la streptozocine, le carboplatine, le cisplatine, l'oxaliplatine, la procarbazine, la dacarbazine, la bléomycine, la vinblastine, la vincristine, la vindésine, la vinorelbine, le paclitaxel, le docétaxel, la L-asparaginase, la flutamide, la nilutamide, la bicalutamide, l'acétate de cyprotérone, la triptoréline, la leuproréline, la goséréline, la buséréline, le formestane, l'aminoglutéthimide, l'anastrazole, le létrozole, le tamoxifène, l'octréotide, le lanréotide, l'acide (Z)-3-[2,4-diméthyl-5-(2-oxo-1,2-dihydro-indol-3-ylidèneméthyl)-1H-pyrrol-3-yl]-propionique, l'acide 4-((9-chloro-7-(2,6-difluorophényl)-5H-pyrimidol(5,4-d)(2)benzazépin-2-yl)amino)benzoïque, l'acide 5,6-diméthylxanthénone-4-acétique et l'acide 3-(4-(1,2-diphénylbut-1-ényl)phényl)acrylique.

18. Composition pharmaceutique pour son utilisation dans le traitement et la prévention des maladies prolifératives, comprenant :
(i) un composé de formule : 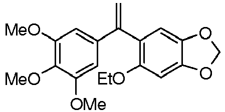
(ii) au moins un autre principe actif, notamment choisi parmi la 6-mercaptopurine, la fludarabine, la cladribine, la pentostatine, la cytarabine, le 5-fluorouracile, la gemcitabine, le méthotrexate, le raltitrexed, l'irinotécan, le topotécan, l'étoposide, la daunorubicine, la doxorubicine, l'épirubicine, l'idarubicine, la pirarubicine, la mitoxantrone, la chlorméthine, la cyclophosphamide, l'ifosfamide, le melphalan, le chlorambucil, le busulfan, la carmustine, la fotémustine, la streptozocine, le carboplatine, le cisplatine, l'oxaliplatine, la procarbazine, la dacarbazine, la bléomycine, la vinblastine, la vincristine, la vindésine, la vinorelbine, le paclitaxel, le docétaxel, la L-asparaginase, la flutamide, la nilutamide, la bicalutamide, l'acétate de cyprotérone, la triptoréline, la leuproréline, la goséréline, la buséréline, le formestane, l'aminoglutéthimide, l'anastrazole, le létrozole, le tamoxifène, l'octréotide, le lanréotide, l'acide (Z)-3-[2,4-diméthyl-5-(2-oxo-1,2-dihydro-indol-3-ylidèneméthyl)-1H-pyrrol-3-yl]-propionique, l'acide 4-((9-chloro-7-(2,6-difluorophényl)-5H-pyrimidol(5,4-d)(2)benzazépin-2-yl)amino)benzoïque, l'acide 5,6-diméthylxanthénone-4-acétique et l'acide 3-(4-(1,2-diphénylbut-1-ényl)phényl)acrylique,
en tant que produits de combinaison pour une utilisation simultanée ou étalée dans le temps.

## Patentansprüche

1. Verbindung der nachfolgenden Formel (I): 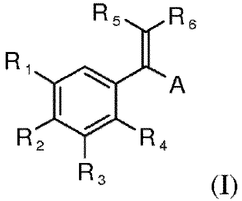 wobei:
- R₁, R₂ und R₃ jeweils eine Methoxygruppe darstellen,
- R₄ ein Wasserstoffatom darstellt,
- R₅ und R₆ identisch sind und jeweils ein Wasserstoff- oder Fluoratom darstellen und vorteilhafterweise jeweils ein Wasserstoffatom darstellen, und
- A ein Zyklus ist, der aus der Gruppe gewählt ist, die Aryl- und Heteroarylgruppen umfasst, wobei die Gruppen sein können:
▪ an einen 5 - 7-gliedrigen Heterozyklus angeschlossen, der ggf. mindestens eine ungesättigte Bindung umfasst und ggf. mit mindestens einer C₁-C₄-Alkylgruppe substituiert ist,
▪ substituiert mit mindestens einer aus der nachfolgenden Gruppe gewählten Gruppe: Halogene, -B(OH)₂-Gruppen, C₁-C₄ -Alkyl, C₂-C₄ -Alkenyl, C₂-C₄ -Alkinyl, Aryl, Heteroaryl, -COOH, -NO₂, Methylendioxy, -NR₇R₈, -NHCOR₇, -CONR₇R₈, -NHCOOR₉,-OSi(C₁-C₄-Alkyl)₃, -NHSO₂R₉, C₁-C₄ -Alkoxy, ggf. substituiert mit mindestens einem Fluoratom, -OCONR₇R₈, -OSO₂CF₃, -OSO₂R₉, -SO₂R₉, -OSO₃H, -OPO(OR₁₀)₂, -ONR₇R₈, -OR₁₁, -SO₂NR₁₂R₁₃, -SO₂NHCOR₁₄, -OCOR₁₅, -OCOOR₁₆, -SR₁₇ und-OCO(CH₂)ₙC₆H₄N[(CH₂)ₘCl]₂, wobei n = 1 - 4 und m = 1 - 3,
wobei:
◆ R₇ und R₈ jeweils unabhängig ein Wasserstoffatom oder eine C₁-C₄-Alkyl, Aryl- oder Heteroarylgruppe darstellen und vorteilhafterweise ein Wasserstoffatom oder eine C₁-C₄ -Alkylgruppe darstellen,
◆ R₉ eine C₁-C₄ -Alkyl-, -Aryl- oder -Heteroarylgruppe darstellt und vorteilhafterweise eine C₁-C₄ -Alkylgruppe darstellt,
◆ R₁₀ ein Wasserstoffatom oder eine C₁-C₄ -Alkylgruppe oder eine Benzylgruppe darstellt,
◆ R₁₁ ein Wasserstoffatom, eine O-Schutzgruppe, einen aus der nachfolgenden Gruppe gewählten Zucker: Glucose, Mannose, Arabinose oder Galactose, einen Aminozucker oder eine Aminosäure darstellt, wobei die freien OH- und NH₂-Gruppen der Zucker, Aminozucker und Aminosäuren jeweils durch eine O- bzw. N-Schutzgruppe ersetzt werden können,
◆ R₁₂ und R₁₃ unabhängig ein Wasserstoffatom, eine C₁-C₄ -Alkylgruppe, eine Aryl- oder eine Heteroarylgruppe darstellen,
◆ -COR₁₄ den Rest eines mit der -SO₂NH-Gruppe verbundenen Aminosäuremoleküls darstellt oder R₁₄ eine C₁-C₄ -Alkylgruppe darstellt,
◆ R₁₅ ein Wasserstoffatom, eine C₁-C₄ -Alkylgruppe, Aryl, Heteroaryl oder eine-(CH₂)ₘCO₂H- oder -(CH₂)ₘNR₇R₈-Gruppe darstellt, wobei m = 1 - 3,
◆ R₁₆ eine C₁-C₄ -Alkylgruppe, Aryl, Heteroaryl oder eine -(CH₂)ₘCO₂H- oder-(CH₂)ₘNR₇R₈-Gruppe darstellt, wobei m = 1 - 3, und
◆ R₁₇ ein Wasserstoffatom, eine C₁-C₄ -Alkylgruppe oder Aryl darstellt, sowie pharmazeutisch annehmbare Salze davon,
unter Ausschluss der Verbindungen der nachfolgenden Formeln:

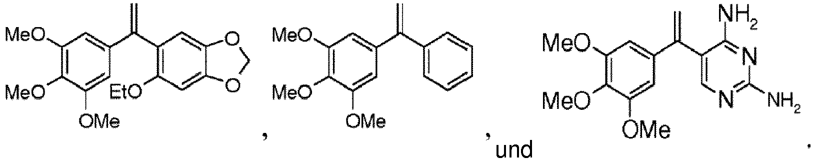

2. Verbindung nach einem der Ansprüche 1, **dadurch gekennzeichnet, dass** A aus der nachfolgenden Gruppe gewählt ist: Phenyl, Naphthyl, Chinolinyl und Indolyl, vorzugsweise Phenyl, wobei diese Gruppen sein können:
▪ an einen 5 - 7-gliedrigen Heterozyklus angeschlossen, der ggf. mindestens eine ungesättigte Bindung umfasst und ggf. mit mindestens einer C₁-C₄-Alkylgruppe substituiert ist,
▪ substituiert mit mindestens einer aus der nachfolgenden Gruppe gewählten Gruppe: Halogene, -B(OH)₂-Gruppen, C₁-C₄ -Alkyl, C₂-C₄ -Alkenyl, C₂-C₄ -Alkinyl, Aryl, Heteroaryl, -COOH, -NO₂, Methylendioxy, -NR₇R₈, -NHCOR₇, -CONR₇R₈, -NHCOOR₉,-OSi(C₁-C₄-Alkyl)₃, -NHSO₂R₉, C₁-C₄ -Alkoxy, ggf. substituiert mit mindestens einem Fluoratom, -OCONR₇R₈, -OSO₂CF₃, -OSO₂R₉, -SO₂R₉, -OSO₃H, -OPO(OR₁₀)₂, -ONR₇R₈, -OR₁₁, -SO₂NR₁₂R₁₃, -SO₂NHCOR₁₄, -OCOR₁₅, -OCOOR₁₆, -SR₁₇ und-OCO(CH₂)ₙC₆H₄N[(CH₂)ₘCl]₂, wobei n = 1 - 4 und m = 1 - 3,
wobei R₇, R₈, R₉, R₁₀, R₁₁, R₁₂, R₁₃, R₁₄, R₁₅, R₁₆ und R₁₇ den Definitionen nach Anspruch 1 entsprechen.

3. Verbindung nach einem der Ansprüche 1 und 2, **dadurch gekennzeichnet, dass** A ein Zyklus ist, der aus der Gruppe gewählt ist, die Aryl- und Heteroarylgruppen umfasst, wobei der Zyklus vorteilhafterweise aus einer Phenyl-, Naphthyl-, Chinolinyl- und Indolylgruppe gewählt ist, vorzugsweise Phenyl ist, und mit mindestens einer aus der nachfolgenden Gruppe gewählten Gruppe substituiert sein kann: -Me, -OH, -OMe,-OCF₃, -NH₂, -NO₂, -COOH, -B(OH)₂, -OSi*t*BuMe₂, -OCOMe, -OCO*t*Bu, Methylendioxy,-OCONEt₂, -OCO(CH₂)₂COOH, -OCOCH₂NMe₂, -OSO₃H, - OSO₂CF₃, -OP(O)(OH)₂,-OP(O)(OEt)₂, -OPO(OCH₂Ph)₂, -Br, -F, OCO(CH₂)₃C₆H₄N[(CH₂)₂Cl]₂, OCO(C₅H₄N),

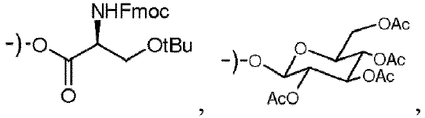

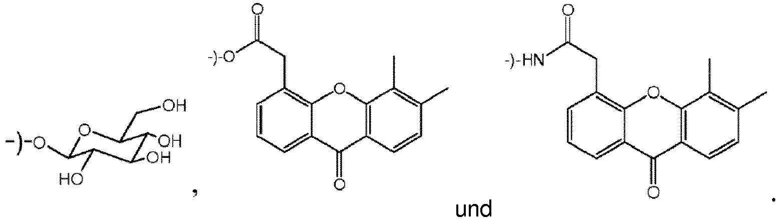

4. Verbindung nach einem der Ansprüche 1 - 4, **dadurch gekennzeichnet, dass** sie der nachfolgenden Formel (II) entspricht: 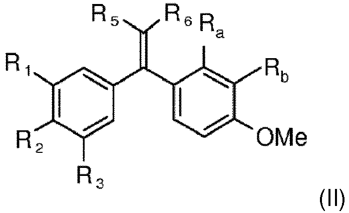 wobei:
- R₁, R₂, R₃, R₅ und R₆ den Definitionen nach Anspruch 1 entsprechen,
- Rₐ ein Wasserstoff- oder Halogenatom, oder eine -B(OH)₂-Gruppe, C₁ - C₄-Alkyl, C₂ - C₄ -Alkenyl, C₂ - C₄ -Alkinyl, Aryl, Heteroaryl, -COOH, -NO₂, Methylendioxy, -NR₇R₈,-NHCOR₇, -CONR₇R₈, -NHCOOR₉, -OSi(C₁-C₄ -Alkyl)₃, -NHSO₂R₉, C₁-C₄ -Alkoxy, ggf. substituiert mit mindestens einem Fluoratom, -OCONR₇R₈, -OSO₂CF₃, -OSO₂R₉, -SO₂R₉, -OSO₃H, -OPO(OR₁₀)₂, -ONR₇R₈, -OR₁₁, -SO₂NR₁₂R₁₃, -SO₂NHCOR₁₄, -OCOR₁₅,-OCOOR₁₆, -SR₁₇ oder -OCO(CH₂)ₙC₆H₄N[(CH₂)ₘCl]₂, wobei n = 1 - 4 und m = 1 - 3, wobei Rₐ vorteilhafterweise ein Wasserstoffatom darstellt, und
- R_{b} ein Halogenatom, vorzugsweise ein Fluoratom, eine -OR₁₁-, -OCOR₁₅-,-OCOOR₁₅-, -OCONR₇R₈-, -OSO₂R₉-, -OSO₂CF₃-, -OSO₃H-, -OPO(OR₁₀)₂-, -NH₂-,-NHCOR₇-, -NHCOOR₉- oder -NHSO₂R₉-Gruppe darstellt,
wobei R₇, R₈, R₉, R₁₀, R₁₁, R₁₂, R₁₃, R₁₄, R₁₅, R₁₆ und R₁₇ den Definitionen nach Anspruch 1 entsprechen.

5. Verbindung nach einem der Ansprüche 1 - 4, **dadurch gekennzeichnet, dass** sie aus der nachfolgenden Gruppe gewählt ist:

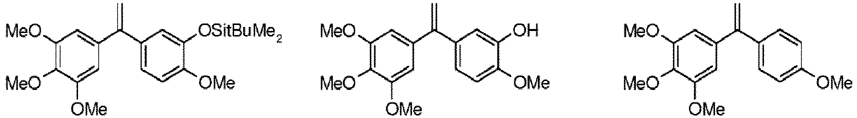

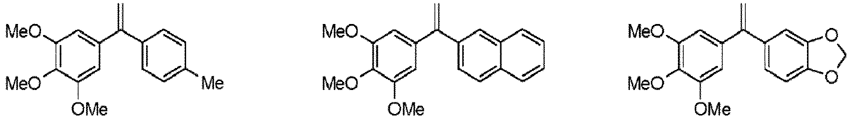

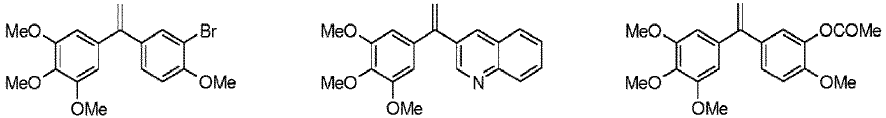

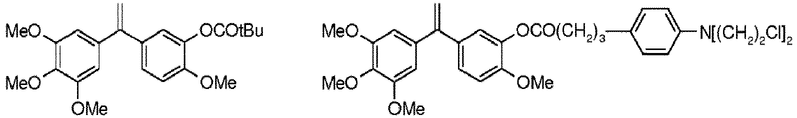

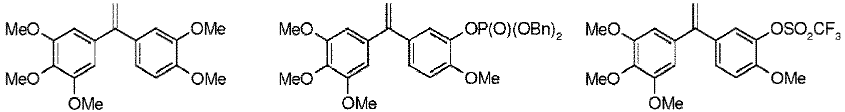

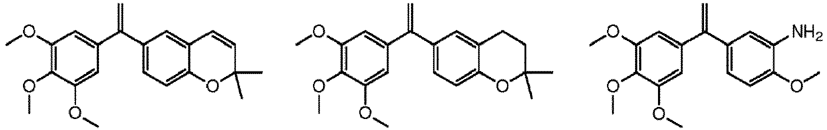

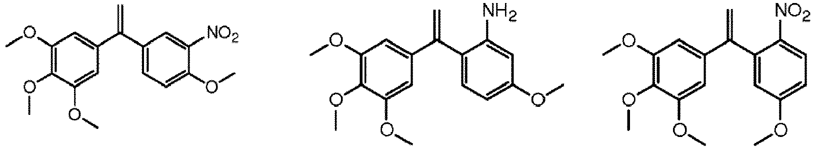

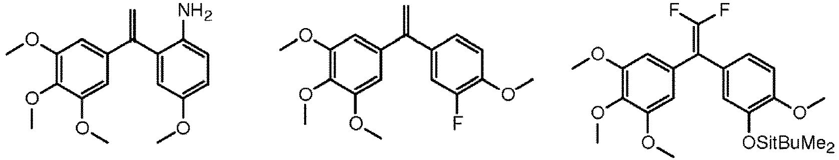

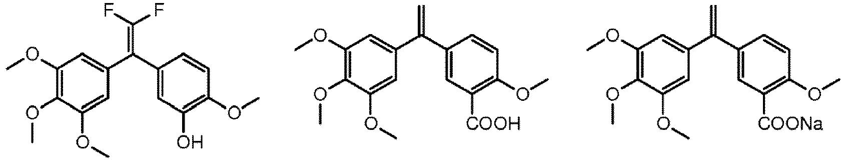

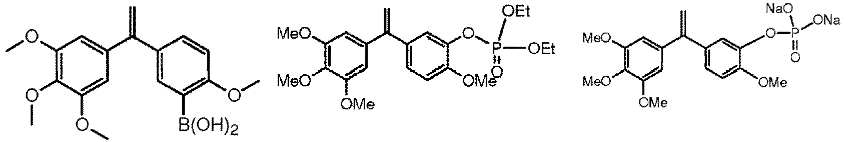

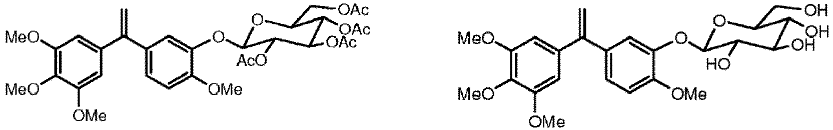

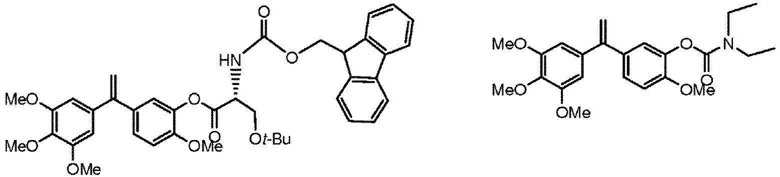

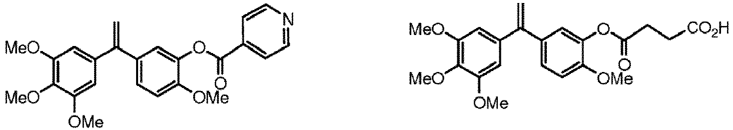

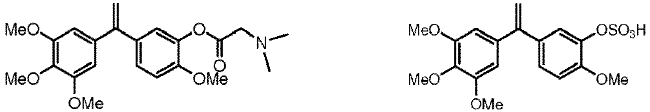

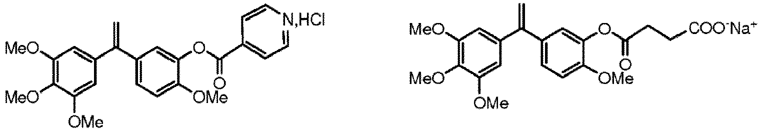

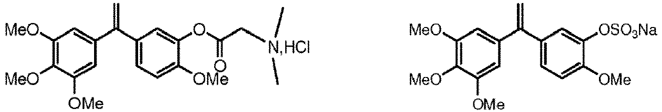

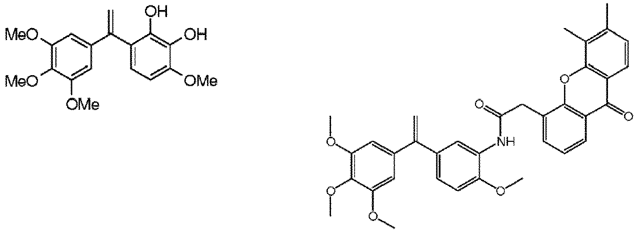

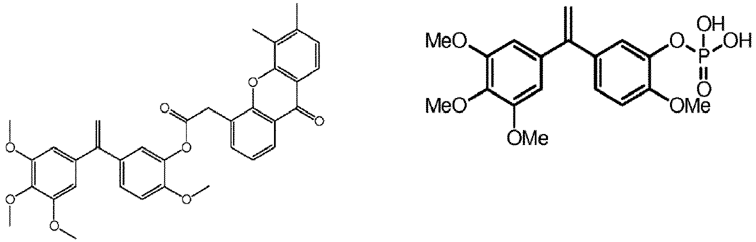

6. Verfahren zur Herstellung einer Verbindung der Formel (I) nach Anspruch 1, **dadurch gekennzeichnet, dass** es folgende Schritte in der nachfolgend angegebenen Reihenfolge umfasst:
- Umsetzen einer Verbindung der nachfolgenden Formel (II): 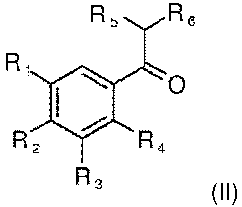 wobei R₁, R₂, R₃, R₄, R₅ und R₆ den Definitionen nach Anspruch 1 entsprechen,
mit einer Organometallverbindung der Formel A-M, wobei A der Definition nach Anspruch 1 entspricht und M ein Alkali- oder Erdalkalimetall darstellt, das mit einem Halogen substituiert ist, um eine Verbindung der nachfolgenden Formel (III) zu bilden: 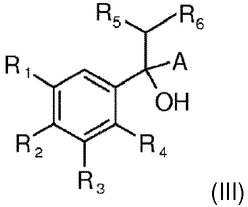
- Umsetzen einer Verbindung der Formel (III) mit einer Säure, um eine Verbindung der Formel (I) zu bilden.

7. Verbindung nach einem der Ansprüche 1 - 5, oder die aus der nachfolgenden Gruppe gewählt ist: 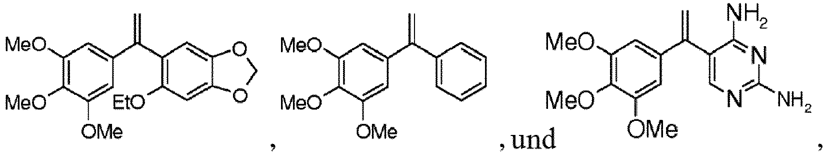 sowie deren pharmazeutisch annehmbare Salze und deren Prodrugs, als Arzneimittel.

8. Verbindung nach Anspruch 7 als Tubulinpolymerisierungshemmer.

9. Verbindung nach einem der Ansprüche 7 und 8 als Arzneimittel zur Behandlung oder Verhütung von proliferativen Erkrankungen wie z.B. Krebs, Psoriasis oder Fibrose.

10. Pharmazeutische Zusammensetzung, **dadurch gekennzeichnet, dass** sie mindestens eine Verbindung nach einem der Ansprüche 1 - 5 oder eine aus der nachfolgenden Gruppe gewählte Verbindung umfasst: 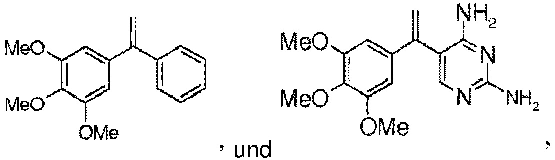 sowie deren pharmazeutisch annehmbare Salze, in Assoziation mit mindestens einem pharmazeutisch annehmbaren Hilfsstoff.

11. Pharmazeutische Zusammensetzung nach Anspruch 10, **dadurch gekennzeichnet, dass** sie mindestens einen weiteren Wirkstoff umfasst.

12. Pharmazeutische Zusammensetzung nach Anspruch 11, **dadurch gekennzeichnet, dass** der Wirkstoff aus der nachfolgenden Gruppe gewählt ist: 6-Mercaptopurin, Fludarabin, Cladribin, Pentostatin, Cytarabin, 5-Fluorouracil, Gemcitabin, Methotrexat, Raltitrexed, Irinotecan, Topotecan, Etoposid, Daunorubicin, Doxorubicin, Epirubicin, Idarubicin, Pirarubicin, Mitoxantron, Chlormethin, Cyclophosphamid, Ifosfamid, Melphalan, Chlorambucil, Busulfan, Carmustin, Fotemustin, Streptozocin, Carboplatin, Cisplatin, Oxaliplatin, Procarbazin, Dacarbazin, Bleomycin, Vinblastin, Vincristin, Vindesin, Vinorelbin, Paclitaxel, Docetaxel, L-Asparaginase, Flutamid, Nilutamid, Bicalutamid, Cyproteronacetat, Triptorelin, Leuprorelin, Goserelin, Buserelin, Formestan, Aminoglutethimid, Anastrazol, Letrozol, Tamoxifen, Octreotid, Lanreotid, (Z)-3-[2,4-Dimethyl-5-(2-oxo-1,2-dihydro-indol-3-ylidenmethyl)-1H-pyrrol-3 -yl] -Propionsäure, 4-((9-Chlor-7-(2,6-difluorphenyl)-5H-pyrimidol(5,4-d)(2)benzazepin-2-yl)amino)benzoesäure, 5,6- Dimethylxanthenon-4-essigsäure und 3-(4-(1,2-Diphenylbut-1-enyl)phenyl)acrylsäure.

13. Pharmazeutische Zusammensetzung, umfassend:
(i) mindestens eine Verbindung nach einem der Ansprüche 1 - 5 oder eine aus der nachfolgenden Gruppe gewählte Verbindung 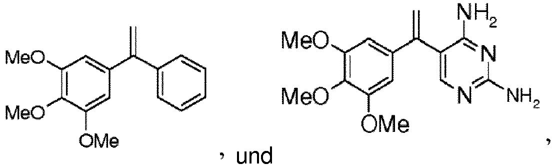
(ii) mindestens einen weiteren Wirkstoff, als Kombinationsprodukte zum gleichzeitigen oder sequentiellen Gebrauch.

14. Pharmazeutische Zusammensetzung nach Anspruch 13, **dadurch gekennzeichnet, dass** der mindestens eine Wirkstoff aus der nachfolgenden Gruppe gewählt ist: 6-Mercaptopurin, Fludarabin, Cladribin, Pentostatin, Cytarabin, 5-Fluorouracil, Gemcitabin, Methotrexat, Raltitrexed, Irinotecan, Topotecan, Etoposid, Daunorubicin, Doxorubicin, Epirubicin, Idarubicin, Pirarubicin, Mitoxantron, Chlormethin, Cyclophosphamid, Ifosfamid, Melphalan, Chlorambucil, Busulfan, Carmustin, Fotemustin, Streptozocin, Carboplatin, Cisplatin, Oxaliplatin, Procarbazin, Dacarbazin, Bleomycin, Vinblastin, Vincristin, Vindesin, Vinorelbin, Paclitaxel, Docetaxel, L-Asparaginase, Flutamid, Nilutamid, Bicalutamid, Cyproteronacetat, Triptorelin, Leuprorelin, Goserelin, Buserelin, Formestan, Aminoglutethimid, Anastrazol, Letrozol, Tamoxifen, Octreotid, Lanreotid, (Z)-3-[2,4-Dimethyl-5-(2-oxo-1,2-dihydro-indol-3-ylidenmethyl)-1H-pyrrol-3-yl]-Propionsäure, 4-((9-Chlor-7-(2,6-difluorphenyl)-5H-pyrimidol(5,4-d)(2)benzazepin-2-yl)amino)benzoesäure, 5,6-Dimethylxanthenon-4-essigsäure und 3-(4-(1,2-Diphenylbut-1-enyl)phenyl)acrylsäure.

15. Zusammensetzung nach einem der Ansprüche 13 und 14 als Arzneimittel zur Behandlung von proliferativen Erkrankungen wie z.B. Krebs, Psoriasis oder Fibrose.

16. Pharmazeutische Zusammensetzung zur Verwendung bei der Behandlung und Verhütung von proliferativen Erkrankungen, umfassend eine Verbindung der nachfolgenden Formel: 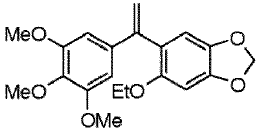 in Assoziation mit mindestens einem pharmazeutisch annehmbaren Hilfsstoff.

17. Zusammensetzung zur Verwendung nach Anspruch 16, **dadurch gekennzeichnet, dass** sie mindestens einen weiteren Wirkstoff umfasst, der insbesondere aus der nachfolgenden Gruppe gewählt ist: 6-Mercaptopurin, Fludarabin, Cladribin, Pentostatin, Cytarabin, 5-Fluorouracil, Gemcitabin, Methotrexat, Raltitrexed, Irinotecan, Topotecan, Etoposid, Daunorubicin, Doxorubicin, Epirubicin, Idarubicin, Pirarubicin, Mitoxantron, Chlormethin, Cyclophosphamid, Ifosfamid, Melphalan, Chlorambucil, Busulfan, Carmustin, Fotemustin, Streptozocin, Carboplatin, Cisplatin, Oxaliplatin, Procarbazin, Dacarbazin, Bleomycin, Vinblastin, Vincristin, Vindesin, Vinorelbin, Paclitaxel, Docetaxel, L-Asparaginase, Flutamid, Nilutamid, Bicalutamid, Cyproteronacetat, Triptorelin, Leuprorelin, Goserelin, Buserelin, Formestan, Aminoglutethimid, Anastrazol, Letrozol, Tamoxifen, Octreotid, Lanreotid, (Z)-3-[2,4-Dimethyl-5-(2-oxo-1,2-dihydro-indol-3-ylidenmethyl)-1H-pyrrol-3-yl]-Propionsäure, 4-((9-Chlor-7-(2,6-difluorphenyl)-5H-pyrimidol(5,4-d)(2)benzazepin-2-yl)amino)benzoesäure, 5,6-Dimethylxanthenon-4-essigsäure und 3-(4-(1,2-Diphenylbut-1-enyl)phenyl)acrylsäure.

18. Pharmazeutische Zusammensetzung zur Verwendung bei der Behandlung und Verhütung von proliferativen Erkrankungen, umfassend:
(i) eine Verbindung der nachfolgenden Formel: 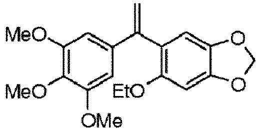
(ii) mindestens einen weiteren Wirkstoff, der insbesondere aus der nachfolgenden Gruppe gewählt ist: 6-Mercaptopurin, Fludarabin, Cladribin, Pentostatin, Cytarabin, 5-Fluorouracil, Gemcitabin, Methotrexat, Raltitrexed, Irinotecan, Topotecan, Etoposid, Daunorubicin, Doxorubicin, Epirubicin, Idarubicin, Pirarubicin, Mitoxantron, Chlormethin, Cyclophosphamid, Ifosfamid, Melphalan, Chlorambucil, Busulfan, Carmustin, Fotemustin, Streptozocin, Carboplatin, Cisplatin, Oxaliplatin, Procarbazin, Dacarbazin, Bleomycin, Vinblastin, Vincristin, Vindesin, Vinorelbin, Paclitaxel, Docetaxel, L-Asparaginase, Flutamid, Nilutamid, Bicalutamid, Cyproteronacetat, Triptorelin, Leuprorelin, Goserelin, Buserelin, Formestan, Aminoglutethimid, Anastrazol, Letrozol, Tamoxifen, Octreotid, Lanreotid, (Z)-3-[2,4-Dimethyl-5-(2-oxo-1,2-dihydro-indol-3-ylidenmethyl)-1H-pyrrol-3-yl]-Propionsäure, 4-((9-Chlor-7-(2,6-difluorphenyl)-5H-pyrimidol(5,4-d)(2)benzazepin-2-yl)amino)benzoesäure, 5,6-Dimethylxanthenon-4-essigsäure und 3-(4-(1,2-Diphenylbut-1-enyl)phenyl)acrylsäure,
als Kombinationsprodukte zum gleichzeitigen oder sequentiellen Gebrauch.

## Claims

1. Compound of following formula (I): 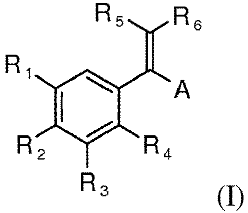 where:
- R₁, R₂ and R₃ are each a methoxy group,
- R₄ is a hydrogen atom,
- R₅ and R₆ are the same and each represent a hydrogen or fluorine atom, and advantageously each represent a hydrogen atom, and
- A is a ring selected from the group comprising aryl and heteroaryl groups, said groups possibly being:
• either fused to a 5 to 7-membered heterocycle, optionally comprising one or more unsaturations and optionally substituted by one or more C₁ to C₄ alkyl groups,
• or substituted by one or more groups selected from among halogens, and the groups -B(OH)₂, C₁ to C₄ alkyls, C₂ to C₄ alkenyls, C₂ to C₄ alkynyls, aryl, heteroaryl, -COOH, -NO₂, methylenedioxy, -NR₇R₈,-NHCOR₇, - CONR₇R₈, -NHCOOR₉, -OSi(C₁ to C₄ alkyl)₃, -NHSO₂R₉, C₁ to C₄ alkoxy optionally substituted by one or more fluorine atoms, -OCONR₇R₈, -OSO₂CF₃, -OSO₂R₉, -SO₂R₉, -OSO₃H, -OPO(OR₁₀)₂, -ONR₇R₈, OR₁₁,-SO₂NR₁₂R₁₃, -SO₂NHCOR₁₄, -OCOR₁₅, -OCOOR₁₆, -SR₁₇ and-OCO(CH₂)ₙC₆H₄N[(CH₂)ₘCl]₂ with n = 1 to 4 and m = 1 to 3,
where:
◆ R₇ and R₈ are each independently a hydrogen atom or C₁ to C₄ alkyl group, aryl or heteroaryl group, and are advantageously a hydrogen atom or C₁ to C₄ alkyl group,
◆ R₉ is a C₁ to C₄ alkyl group, aryl or heteroaryl group, and is advantageously a C₁ to C₄ alkyl group,
◆ R₁₀ is a hydrogen atom or C₁ to C₄ alkyl group or a benzyl group,
◆ R₁₁ is a hydrogen atom, O-protector group, a sugar selected from among glucose, mannose, arabinose or galactose, an amino sugar or amino acid, the free OH and NH₂ groups of sugars, amino sugars and amino acids optionally being able to be substituted by an O-protector and N-protector group respectively,
◆ R₁₂ and R₁₃ are each independently a hydrogen atom, C₁ to C₄ alkyl group, aryl or heteroaryl group,
◆ -COR₁₄ represents the remainder of an amino acid molecule linked to the -SO₂NH- group or R₁₄ is a C₁ to C₄ alkyl group,
◆ R₁₅ is a hydrogen atom, C₁ to C₄ alkyl group, aryl or heteroaryl group, or a group -(CH₂)ₘCO₂H or -(CH₂)ₘNR₇R₈ with m = 1 to 3,
◆ R₁₆ is a C₁ to C₄ alkyl group, aryl or heteroaryl group, or a group -(CH₂)ₘCO₂H or -(CH₂)ₘNR₇R₈ with m = 1 to 3, and
◆ R₁₇ is a hydrogen atom or C₁ to C₄ alkyl group, or aryl group, and the pharmaceutically acceptable salts thereof,
with the exception of the compounds of following formulas:

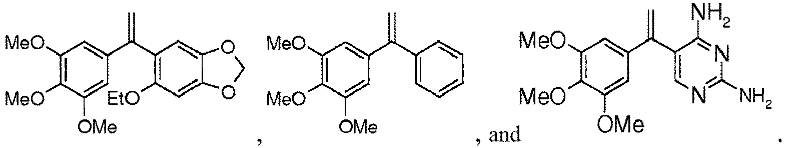

2. The compound according to claim 1, **characterized in that** A is selected from the group comprising the phenyl, naphthyl, quinolinyl and indolyl groups, and preferably phenyl, said groups possibly being:
• either fused to a 5 to 7-membered heterocycle, optionally comprising one or more unsaturations and optionally substituted by one or more C₁ to C₄ alkyl groups,
• or substituted by one or more groups selected from among halogens, the groups -B(OH)₂, C₁ to C₄ alkyls, C₂ to C₄ alkenyls, C₂ to C₄ alkynyls, aryl, heteroaryl, -COOH, -NO₂, methylenedioxy, -NR₇R₈,-NHCOR₇, -CONR₇R₈, -NHCOOR₉, -OSi(C₁ to C₄ alkyl)₃, -NHSO₂R₉, C₁ to C₄ alkoxy optionally substituted by one or more fluorine atoms, -OCONR₇R₈,-OSO₂CF₃, -OSO₂R₉, -SO₂R₉, -OSO₃H, -OPO(OR₁₀)₂, -ONR₇R₈, OR₁₁,-SO₂NR₁₂R₁₃, -SO₂NHCOR₁₄, -OCOR₁₅, -OCOOR₁₆, -SR₁₇ and-OCO(CH₂)ₙC₆H₄N[(CH₂)ₘCl]₂ with n = 1 to 4 and m = 1 to 3,
R₇, R₈, R₉, R₁₀, R₁₁, R₁₂, R₁₃, R₁₄, R₁₅, R₁₆ and R₁₇ being such as defined in claim 1.

3. The compound according to any of claims 1 and 2, **characterized in that** A is a ring selected from the group comprising the aryl and heteroaryl groups, said ring advantageously being selected from among a phenyl, naphthyl, quinolinyl and indolyl group, preferably phenyl, and said ring possibly being substituted by one or more groups selected from among -Me, -OH, -OMe, -OCF₃, -NH₂, -NO₂, -COOH,-B(OH)₂, -OSi*t*BuMe₂, -OCOMe, -OCO*t*Bu, methylenedioxy, -OCONEt₂,-OCO(CH₂)₂COOH, -OCOCH₂NMe₂, -OSO₃H, -OSO₂CF₃, -OP(O)(OH)₂,-OP(O)(OEt)₂, -OPO(OCH₂Ph)₂, -Br, -F, -OCO(CH₂)₃C₆H₄N[(CH₂)₂Cl]₂,-OCO(C₅H₄N),

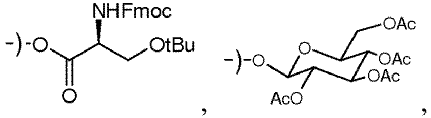

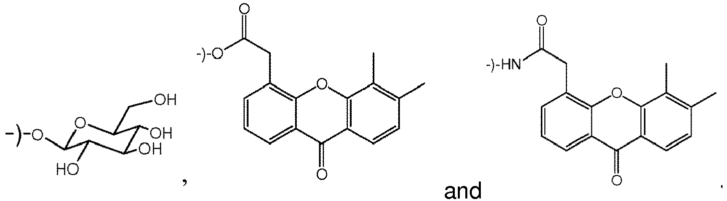

4. The compound according to any of claims 1 to 4, **characterized in that** it meets following formula (II): 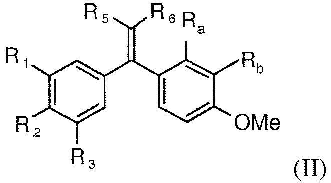 where:
- R₁, R₂, R₃, R₅ and R₆ are such as defined in claim 1,
- Rₐ is a hydrogen or halogen atom, or groups -B(OH)₂, C₁ to C₄ alkyls, C₂ to C₄ alkenyls, C₂ to C₄ alkynyls, aryl, heteroaryl, -COOH, -NO₂, methylenedioxy,-NR₇R₈, -NHCOR₇, - CONR₇R₈, -NHCOOR₉, -OSi(C₁ to C₄ alkyl)₃, -NHSO₂R₉, C₁ to C₄ alkoxy optionally substituted by one or more fluorine atoms, -OCONR₇R₈, -OSO₂CF₃, -OSO₂R₉, -SO₂R₉, -OSO₃H, -OPO(OR₁₀)₂, -ONR₇R₈, OR₁₁,-SO₂NR₁₂R₁₃, -SO₂NHCOR₁₄, -OCOR₁₅, -OCOOR₁₆, -SR₁₇ or-OCO(CH₂)ₙC₆H₄N[(CH₂)ₘCl]₂ with n = 1 to 4 and m = 1 to 3, Rₐ advantageously being a hydrogen atom, and
- R_{b} is a halogen atom, preferably a fluorine atom, a group OR₁₁, -OCOR₁₅,-OCOOR₁₅, -OCONR₇R₈, -OSO₂R₉, -OSO₂CF₃, -OSO₃H, -OPO(OR₁₀)₂, -NH₂,-NHCOR₇, -NHCOOR₉ or -NHSO₂R₉,
- R₇, R₈, R₉, R₁₀, R₁₁, R₁₂, R₁₃, R₁₄, R₁₅, R₁₆ and R₁₇ being such as defined in claim 1.

5. The compound according to any of claims 1 to 4, **characterized in that** it is selected from among:

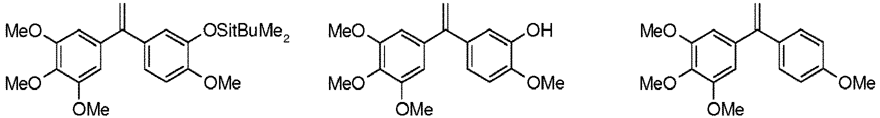

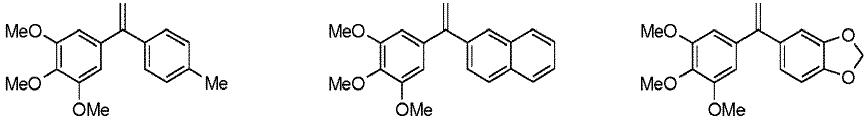

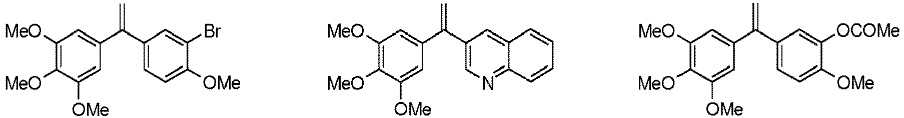

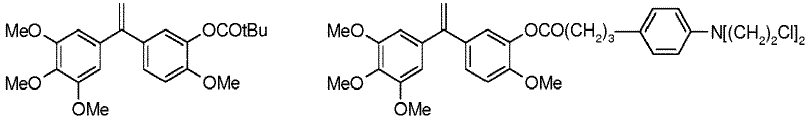

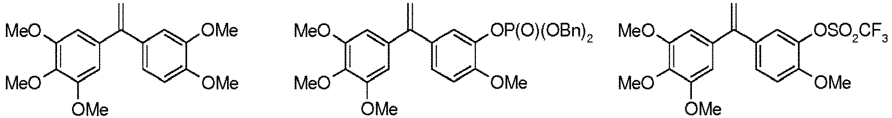

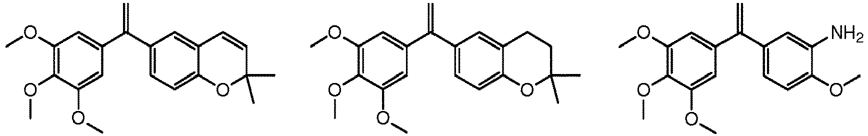

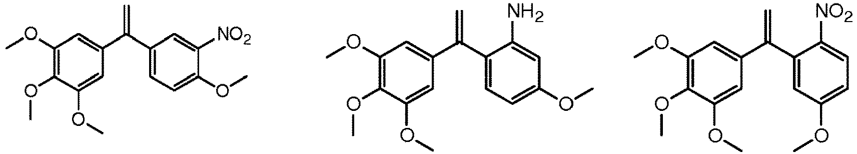

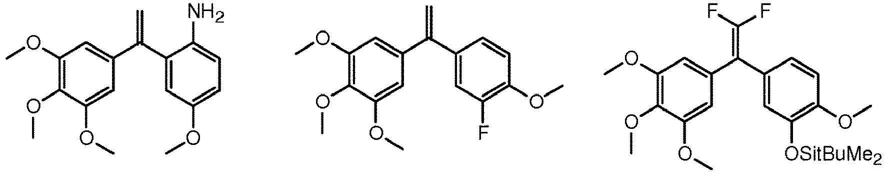

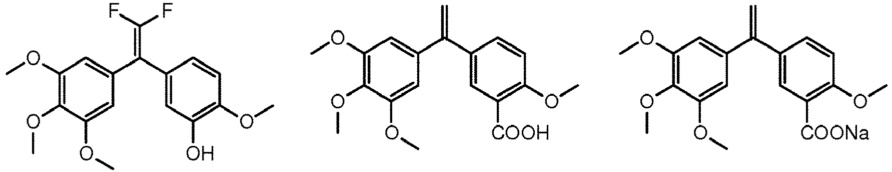

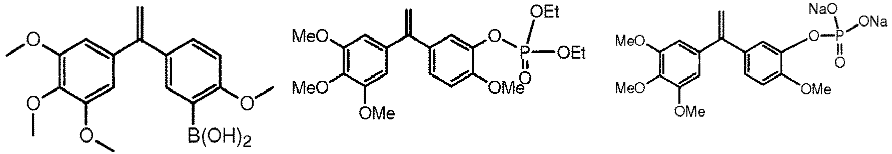

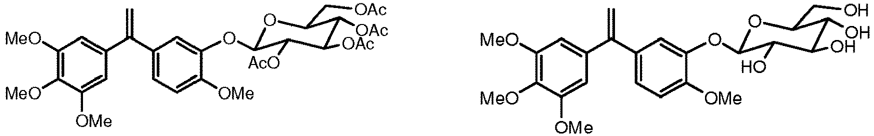

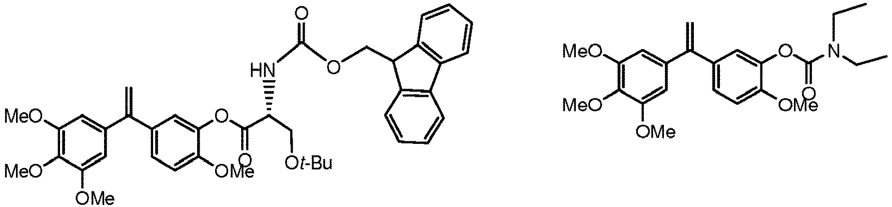

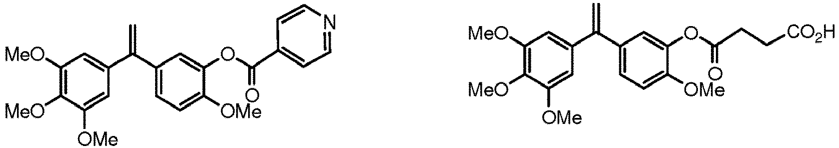

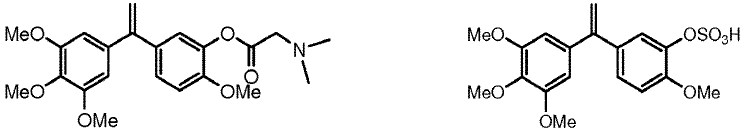

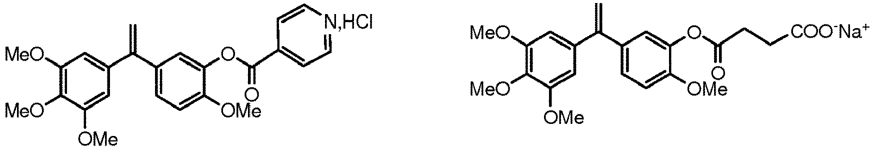

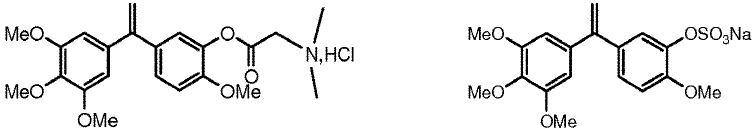

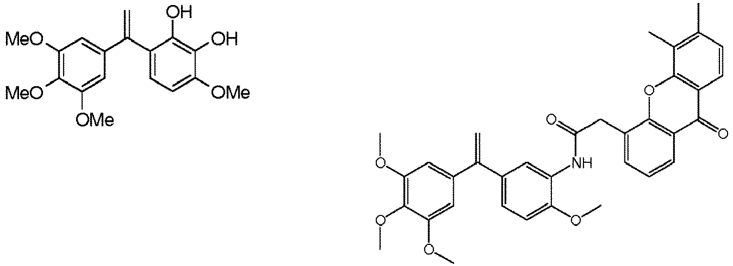

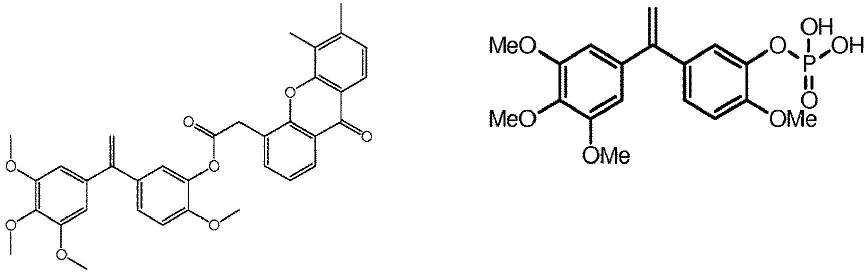

6. Method for preparing a formula (I) compound such as defined in claim 1, **characterized in that** it comprises the following successive steps:
- reacting a compound of following formula (II): 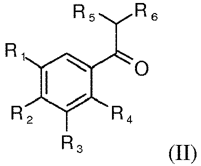 where R₁, R₂, R₃, R₄, R₅ and R₆ are such as defined in claim 1,
with an organometallic compound of formula A-M, where A is such as defined in claim 1 and M is an alkali metal or alkali-earth metal substituted by a halogen, to form a compound of following formula (III): 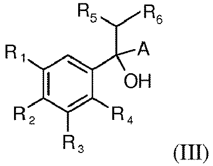
- reacting a compound of formula (III) with an acid to form a compound of formula (I).

7. The compound according to any of claims 1 to 5, or compound selected from among: 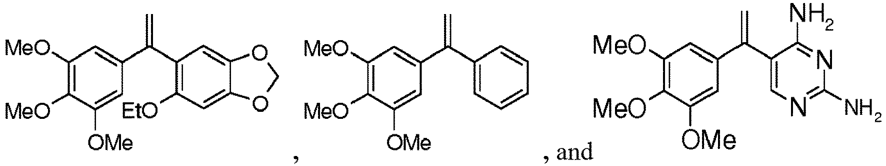 and the pharmaceutically acceptable salts and prodrugs thereof, as medicinal product.

8. The compound according to claim 7, as medicinal product inhibiting polymerisation of tubulin.

9. The compound according to any of claims 7 and 8, as medicinal product intended for the treatment or prevention of proliferative diseases such as cancer, psoriasis or fibrosis.

10. Pharmaceutical composition **characterized in that** it comprises at least one compound according to any of claims 1 to 5 or a compound selected from among: 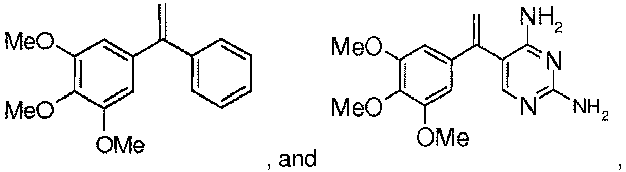 and the pharmaceutically acceptable salts thereof, in association with one or more pharmaceutically acceptable excipients.

11. The pharmaceutical composition according to claim 10, **characterized in that** it comprises at least one other active ingredient.

12. The pharmaceutical composition according to claim 11, **characterized in that** the active ingredient is selected from among 6-mercaptopurin, fludarabine, cladribine, pentostatin, cytarabine, 5-fluorouracil, gemcitabine, methotrexate, raltitrexed, irinotecan, topotecan, etoposide, daunorubicin, doxorubicin, epirubicin, idarubicin, pirarubicin, mitoxantrone, chlormethine, cyclophosphamide, ifosfamide, melphalan, chlorambucil, busulfan, carmustine, fotemustine, streptozocin, carboplatin, cisplatin, oxaliplatin, procarbazine, dacarbazine, bleomycin, vinblastine, vincristine, vindesine, vinorelbine, paclitaxel, docetaxel, L-asparaginase, flutamide, nilutamide, bicalutamide, cyproterone acetate, triptorelin, leuprorelin, goserelin, buserelin, formestane, aminoglutethimide, anastrazole, letrozole, tamoxifen, octreotide, lanreotide, (Z)-3-[2,4-dimethyl-5-(2-oxo-1,2-dihydro-indol-3-ylidenemethyl)-1H-pyrrol-3-yl] propionic acid, 4-((9-chloro-7-(2,6-difluorophenyl)-5H-pyrimidol(5,4-d)(2)benzazepin-2-yl)amino)benzoic acid, 5,6-dimethylxanthenone-4-acetic acid and 3-(4-(1,2-diphenylbut-1-enyl)phenyl)acrylic acid.

13. Pharmaceutical composition comprising:
(i) at least one compound according to any of claims 1 to 5 or a compound selected from among: 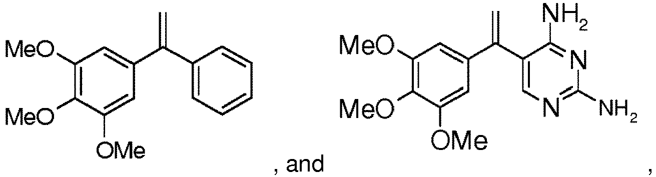
(ii) at least one other active ingredient,
as combination products for simultaneous or time-staggered use.

14. The composition according to claim 13, **characterized in that** the active ingredient(s) is(are) selected from among 6-mercaptopurin, fludarabine, cladribine, pentostatin, cytarabine, 5-fluorouracil, gemcitabine, methotrexate, raltitrexed, irinotecan, topotecan, etoposide, daunorubicin, doxorubicin, epirubicin, idarubicin, pirarubicin, mitoxantrone, chlormethine, cyclophosphamide, ifosfamide, melphalan, chlorambucil, busulfan, carmustine, fotemustine, streptozocin, carboplatin, cisplatin, oxaliplatin, procarbazine, dacarbazine, bleomycin, vinblastine, vincristine, vindesine, vinorelbine, paclitaxel, docetaxel, L-asparaginase, flutamide, nilutamide, bicalutamide, cyproterone acetate, triptorelin, leuprorelin, goserelin, buserelin, formestane, aminoglutethimide, anastrazole, letrozole, tamoxifen, octreotide, lanreotide, (Z)-3-[2,4-dimethyl-5-(2-oxo-1,2-dihydro-indol-3-ylidenemethyl)-1H-pyrrol-3-yl] propionic acid, 4-((9-chloro-7-(2,6-difluorophenyl)-5H-pyrimidol(5,4-d)(2)benzazepin-2-yl)amino)benzoic acid, 5,6-dimethylxanthenone-4-acetic acid and 3-(4-(1,2-diphenylbut-1-enyl)phenyl)acrylic acid.

15. The composition according to any of claims 13 and 14 as medicinal product for the treatment of proliferative diseases such as cancer, psoriasis or fibrosis.

16. Pharmaceutical composition for use thereof in the treatment and prevention of proliferative diseases, comprising a compound of formula: 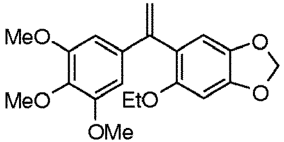 in association with one or more pharmaceutically acceptable excipients.

17. Composition for use thereof according to claim 16, **characterized in that** it comprises at least one other active ingredient selected in particular from among 6-mercaptopurin, fludarabine, cladribine, pentostatin, cytarabine, 5-fluorouracil, gemcitabine, methotrexate, raltitrexed, irinotecan, topotecan, etoposide, daunorubicin, doxorubicin, epirubicin, idarubicin, pirarubicin, mitoxantrone, chlormethine, cyclophosphamide, ifosfamide, melphalan, chlorambucil, busulfan, carmustine, fotemustine, streptozocin, carboplatin, cisplatin, oxaliplatin, procarbazine, dacarbazine, bleomycin, vinblastine, vincristine, vindesine, vinorelbine, paclitaxel, docetaxel, L-asparaginase, flutamide, nilutamide, bicalutamide, cyproterone acetate, triptorelin, leuprorelin, goserelin, buserelin, formestane, aminoglutethimide, anastrazole, letrozole, tamoxifen, octreotide, lanreotide, (Z)-3-[2,4-dimethyl-5-(2-oxo-1,2-dihydro-indol-3-ylidenemethyl)-1H-pyrrol-3-yl] propionic acid, 4-((9-chloro-7-(2,6-difluorophenyl)-5H-pyrimidol(5,4-d)(2)benzazepin-2-yl)amino)benzoic acid, 5,6-dimethylxanthenone-4-acetic acid and 3-(4-(1,2-diphenylbut-1-enyl)phenyl)acrylic acid.

18. Pharmaceutical composition for use thereof in the treatment and prevention of proliferative diseases, comprising:
(i) a compound of formula: 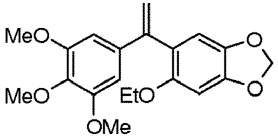
(ii) at least one other active ingredient selected in particular from among 6-mercaptopurin, fludarabine, cladribine, pentostatin, cytarabine, 5-fluorouracil, gemcitabine, methotrexate, raltitrexed, irinotecan, topotecan, etoposide, daunorubicin, doxorubicin, epirubicin, idarubicin, pirarubicin, mitoxantrone, chlormethine, cyclophosphamide, ifosfamide, melphalan, chlorambucil, busulfan, carmustine, fotemustine, streptozocin, carboplatin, cisplatin, oxaliplatin, procarbazine, dacarbazine, bleomycin, vinblastine, vincristine, vindesine, vinorelbine, paclitaxel, docetaxel, L-asparaginase, flutamide, nilutamide, bicalutamide, cyproterone acetate, triptorelin, leuprorelin, goserelin, buserelin, formestane, aminoglutethimide, anastrazole, letrozole, tamoxifen, octreotide, lanreotide, (Z)-3-[2,4-dimethyl-5-(2-oxo-1,2-dihydro-indol-3-ylidenemethyl)-1H-pyrrol-3-yl] propionic acid, 4-((9-chloro-7-(2,6-difluorophenyl)-5H-pyrimidol(5,4-d)(2)benzazepin-2-yl)amino)benzoic acid, 5,6-dimethylxanthenone-4-acetic acid and 3-(4-(1,2-diphenylbut-1-enyl)phenyl)acrylic acid,
as combination products for simultaneous or time-staggered use.
